(19) Europäisches Patentamt
European Patent Office
Office européen des brevets

(11) EP 4 479 095 B1

(12) # EUROPEAN PATENT SPECIFICATION

(45) Date of publication and mention of the grant of the patent:
**04.02.2026 Bulletin 2026/06**

(21) Application number: **23705562.9**

(22) Date of filing: **17.02.2023**

(51) International Patent Classification (IPC):
***A61K 47/69*** (2017.01) ***A61K 47/64*** (2017.01)
***A61K 47/54*** (2017.01) ***C08G 69/10*** (2006.01)
***A61K 9/16*** (2006.01) ***A61K 48/00*** (2006.01)
***C12N 15/88*** (2006.01)

(52) Cooperative Patent Classification (CPC):
**A61K 47/6935; A61K 9/0014; A61K 9/0019; A61K 9/0021; A61K 9/0031; A61K 9/0053; A61K 9/107; A61K 9/127; A61K 9/19; A61K 9/5123; A61K 47/542; A61K 47/543; A61K 47/64; C08G 69/10; C08G 69/48;** (Cont.)

(86) International application number:
**PCT/EP2023/054101**

(87) International publication number:
**WO 2023/156629 (24.08.2023 Gazette 2023/34)**

(54) **STEALTH-STABILIZED LIPO-POLYAMINO ACIDS**
TARN-STABILISIERTE LIPO-POLYAMINOSÄUREN
ACIDES LIPO-POLYAMINES FURTIFS STABILISÉS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.02.2022 EP 22382135**

(43) Date of publication of application:
**25.12.2024 Bulletin 2024/52**

(73) Proprietor: **Polypeptide Therapeutic Solutions, S.L.**
**46980 Paterna (ES)**

(72) Inventors:
- **FELIP LEON, Carles**
  **46980 PATERNA (ES)**
- **GARCIA GARCIA, Josep**
  **46980 PATERNA (ES)**
- **HERRERA MUÑOZ, Lidia**
  **46980 PATERNA (ES)**
- **NEBOT CARDA, Vicent Josep**
  **46980 PATERNA (ES)**

(74) Representative: **ZBM Patents - Zea, Barlocci & Markvardsen**
**Rambla de Catalunya, 123**
**08008 Barcelona (ES)**

(56) References cited:
**WO-A1-2020/069718 US-A1- 2021 128 747**

- **NOGUEIRA SARA S. ET AL: "Polysarcosine-Functionalized Lipid Nanoparticles for Therapeutic mRNA Delivery", vol. 3, no. 11, 25 November 2020 (2020-11-25), pages 10634 - 10645, XP055809176, ISSN: 2574-0970, Retrieved from the Internet <URL:https://pubs.acs.org/doi/pdf/10.1021/acsanm.0c01834> DOI: 10.1021/acsanm.0c01834**
- **BENJAMIN WEBER ET AL: "Polysarcosine-Based Lipids: From Lipopolypeptoid Micelles to Stealth-Like Lipids in Langmuir Blodgett Monolayers", POLYMERS, vol. 8, no. 12, 9 December 2016 (2016-12-09), pages 427, XP055574069, DOI: 10.3390/polym8120427**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MUHL CHRISTIAN ET AL: "Synthesis and characterization of bisalkylated polysarcosine-based lipopolymers", EUROPEAN POLYMER JOURNAL, PERGAMON PRESS LTD OXFORD, GB, vol. 120, 30 August 2019 (2019-08-30), XP085894748, ISSN: 0014-3057, [retrieved on 20190830], DOI: 10.1016/ J.EURPOLYMJ.2019.109223**
- **JOSBERT M. METSELAAR ET AL: "A Novel Family of L-Amino Acid-Based Biodegradable Polymer-Lipid Conjugates for the Development of Long-Circulating Liposomes with Effective Drug-Targeting Capacity", BIOCONJUGATE CHEMISTRY, vol. 14, no. 6, 1 November 2003 (2003-11-01), pages 1156 - 1164, XP055051086, ISSN: 1043-1802, DOI: 10.1021/bc0340363**
- **XIONG QINGQING ET AL: "Biomedical applications of mRNA nanomedicine", NANO RESEARCH, TSINGHUA UNIVERSITY PRESS, CN, vol. 11, no. 10, 27 July 2018 (2018-07-27), pages 5281 - 5309, XP037073241, ISSN: 1998-0124, [retrieved on 20180727], DOI: 10.1007/ S12274-018-2146-1**
- **ZHOU X ET AL: "Lipophilic polylysines mediate efficient DNA transfection in mammalian cells", BIOCHIMICA ET BIOPHYSICA ACTA, ELSEVIER, AMSTERDAM, NL, vol. 1065, no. 1, 31 May 1991 (1991-05-31), pages 8 - 14, XP027222372, ISSN: 0005-2736, [retrieved on 19910531]**
- **IRIS BIOTECH: "PotM: Functionalized Polysarcosines for the Formation of Lipid Nanoparticles: Towards Improved RNA Delivery", 3 July 2022 (2022-07-03), XP093047123, Retrieved from the Internet <URL:https://www.iris-biotech.de/de/blog/potm-functionalized-polysarcosines-for-the-formation-of-lipid-nanoparticles-towards-improved-rna-delivery/> [retrieved on 20230515]**


Remarks:

The file contains technical information submitted after the application was filed and not included in this specification

(52) Cooperative Patent Classification (CPC): (Cont.)
**C12N 15/88**

## Description

### Technical Field

The present disclosure relates to lipo-polyamino acid conjugates which comprise a polyamino acid (PAA) moiety conjugated to a lipid-like moiety. These lipo-polyamino acid conjugates may form self-assembled nanoparticles, such as for example lipid nanoparticles (LNPs), and can be used as non-viral vectors for delivery of active ingredients, including nucleic acids, to cells.

### Background Art

Nucleic acids have emerged in recent years to yield promising drug candidates for a wide range of diseases, including, among others, cancer, infectious diseases, and cardiovascular, inflammatory, and neurodegenerative diseases. Nucleic acids exert their action via gene inhibition, addition, replacement or editing. However, their clinical efficacy is very much limited because of their inability to cross physiological barriers. The negative charge and hydrophilicity of nucleic acids hinder their passive diffusion across plasma membranes. Moreover, the association of nucleic acids with serum proteins as well as their susceptibility to enzymatic degradation by endogenous nucleases also interferes with their efficient translation to functional response.

Non-viral vectors such as LNPs are known to permeate plasma membranes and thus to be able to deliver nucleic acids to cells. LNPs are vesicles composed of mixtures of lipids that are physically associated with each other by intermolecular forces forming a micellar structure. LNPs typically have a small diameter of less than 200 nm. LNPs are used as drug delivery system thanks to their ability to encapsulate biologically active agents and deliver them to specific locations within the body at a desired time. The lipid components of LNPs may include ionizable or cationic lipids, helper lipids such as phospholipids and structural lipids such as sterols or cholesterol.

Ionizable/cationic lipids, which have either a permanent or ionizable positively charged head group followed by a hydrophobic tail, can form complexes with negatively charged nucleic acids by means of electrostatic bonds.

One of the potential problems of LNPs is that once they are injected, they may be recognised by the immune system as foreign particles and subsequently cleared from the bloodstream by phagocytes. In order to overcome this problem, different strategies may be used including decreasing the particle size of the vesicles or modifying their surface. For example, the LNP surface may be modified by conjugating the hydrophilic polymer polyethylene glycol (PEG) to lipids such as phospholipids or long-chain fatty acids to form PEG-lipids.

PEG is a hydrophilic polymer widely used in drug delivery and nanotechnology because of its stealth properties and biocompatibility. Thanks to these properties, particulate delivery systems containing PEG are able to evade the immune system, and, as a result, have a prolonged circulation time within the body.

Despite the above, it has been recently reported that the presence of covalently bonded PEG also significantly reduces the transfection efficiency because the neutral surface of the nanoparticles can decrease the cellular uptake efficiency. Also, it is well known that the PEG also generates anti-PEG antibodies that may cause immunogenicity and allergic reactions.

While other hydrophilic polymers such as poly(vinylpyrrolidones), poly(acryloyl-morpholines) or polyoxazolines have been reported as alternatives to PEG-lipids, no LNP containing such polymers have been marketed yet.

Polysarcosine (pSar) is a polypeptoid based on the endogenous amino acid sarcosine (N-methylated glycine), which has previously shown potent stealth properties. Lipid nanoparticles (LNPs) for therapeutic application of messenger RNA assembled using pSarcosinylated lipids have been reported as a promising tool for particle engineering (ACS Appl. Nano Mater., 2020, 3, 10634-10645) but this is still under development and no clinical data including this polymer into LNPs is presented yet.

In the same context, WO 2020/069718 A1 also discloses self-assembled nanoparticles comprising a lipid-pSar conjugate and mRNA, which demonstrate high in vivo and in vitro transfection efficiency as well as reduced toxicity compared to PEG-containing nanoparticles.

Therefore, from what it is known in the field, there is still the need to develop alternative LNPs which overcome the disadvantages of the prior art.

### Summary of Invention

The inventors have developed new lipo-polyamino acid conjugates which comprise a polyamino acid (PAA) moiety conjugated to at least one lipid-like moiety. These conjugates may form self-assembled nanoparticles, such as for example lipid nanoparticles (LNPs), and can be effectively used as non-viral vectors for delivery of active ingredients, including nucleic acids, to cells, thanks to their stealth properties and consequent improved plasma half-life time.

Furthermore, the lipo-polyamino acid conjugates show low cytotoxicity, high biodegradability and may confer to

the final nanosystem enhanced efficiency, high stability due to limited or even completely suppressed aggregation issues in the bloodstream and potential different cell and tissue tropism.

Therefore, a first aspect of the invention relates to a lipo-polyamino acid conjugate of formula (I), a salt thereof, or any stereoisomer or mixtures of stereoisomers, either of the compound of formula (I), or of any of its salts,

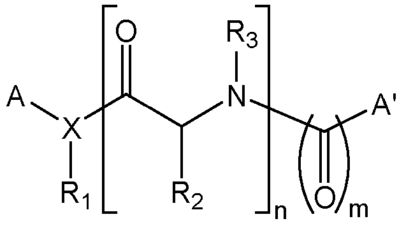

(I)

wherein

n is an integer from 5 to 150; m is 0 or 1;
X is selected from the group consisting of N, S, and O;
$R_1$ is selected from the group consisting of H, -($C_1$-$C_{18}$)alkyl, and -($C_2$-$C_{18}$)alkenyl, with the condition that $R_1$ is absent when X is O or S;
$R_2$ is a radical selected from the group consisting of (II), (III), (IV), and (V)

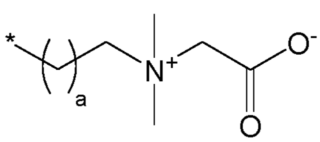

(II)

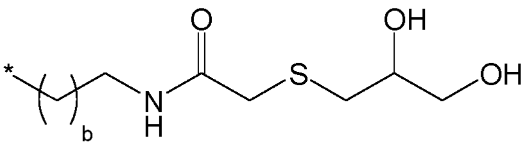

(III)

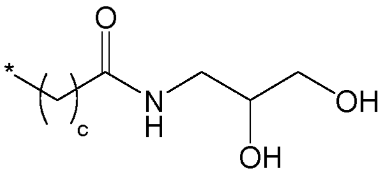

(IV)

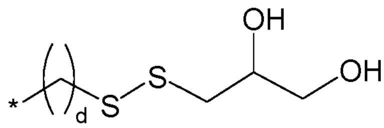

(V)

wherein:
a, b, c, and d are independently an integer from 1 to 4;
$R_3$ is H;
A is a lipid-like moiety $R_5$,
A' is selected from the group consisting of H, -and ($C_1$-$C_6$)alkyl,
wherein

(i) the lipid-like moiety $R_5$ has the formula (XIX):

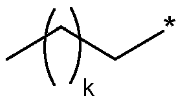

(XIX)

wherein k is 10, 11, 12, 13, 14, 15, or 16; or alternatively

(ii) the lipid-like moiety $R_5$ is ($C_6$-$C_{18}$)alkenyl or alternatively has the formula (XX):

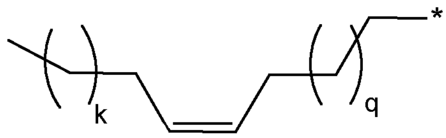

(XX)

wherein k is 10, 11, 12, 13, 14, 15, or 16, and q is an integer from 0 to 18; or alternatively

(iii) the lipid-like moiety $R_5$ has the formula (IX):

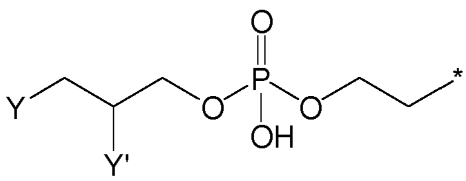

(IX)

wherein Y and Y' are independently selected from -OH, -COORx, and -OCORx; and each Rx is a radical independently selected from formula (ixa) and (ixb):

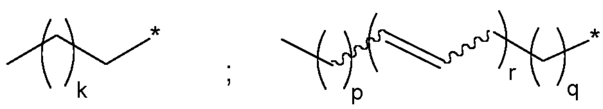

(ixa) (ixb)

wherein the wavy bonds mean that the stereochemistry of the double bonds is undefined, k is 10, 11, 12, 13, 14, 15, or 16; q is an integer from 0 to 18; p is 10, 11, 12, 13, 14, 15, 16, 17, or 18; and r is an integer from 0 to 6; or alternatively

(iv) the lipid-like moiety $R_5$ has the formula (X):

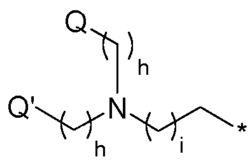

(X)

wherein Q and Q' are independently selected from -OCORy and -COORy; each h is independently an integer from 1 to 18; i is an integer from 0 to 12; and each Ry is a radical independently selected from formula (xa), (xb), and (xc):

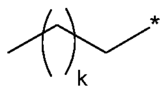 ; 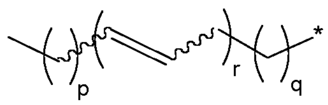 ; 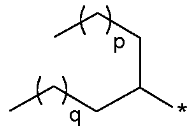

(xa) (xb) (xc)

wherein the wavy bonds mean that the stereochemistry of the double bonds is undefined, k is 10, 11, 12, 13, 14, 15, or 16; q is an integer from 0 to 18, p is 10, 11, 12, 13, 14, 15, 16, 17, or 18; and r is an integer from 0 to 6; or alternatively

(v) the lipid-like moiety $R_5$ has the formula (XII):

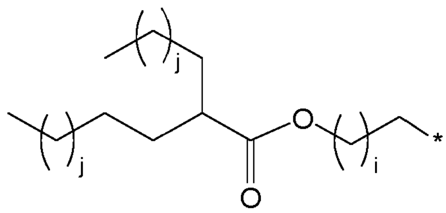

(XII)

wherein i is an integer from 0 to 18; and each j is independently 3, 4, 5, 6, 7, 8, 9, 10, 11, 12; or alternatively

(vi) the lipid-like moiety $R_5$ has the formula (XIII):

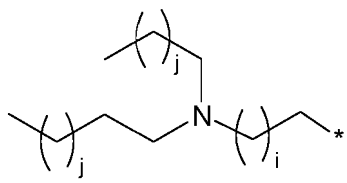

(XIII)

wherein i is an integer from 0 to 18; and each j is independently 10, 11, 12; or alternatively

(vii) the lipid-like moiety $R_5$ has the formula (XIV):

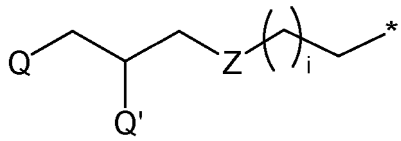

(XIV)

wherein Q and Q' are independently selected from -OCORy and -COORy; Z is selected from -OCO-, -COO-, -NRz'CO-, and -CONRz'-; each Rz' is H or Rz; each Rz is $(C_1\text{-}C_{18})$alkyl or $-(C_2\text{-}C_{18})$alkenyl; i is an integer from 0 to 18; and each Ry is a radical independently selected from formula (xiva) and (xivb):

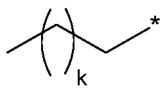 ; 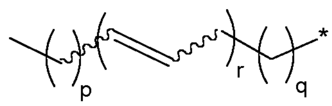

(xiva) (xivb)

wherein the wavy bonds mean that the stereochemistry of the double bonds is undefined, k is 10, 11, 12, 13, 14,

15, or 16; p is an integer from 0 to 18; p is 10, 11, 12, 13, 14, 15, 16, 17, or 18; and r is an integer from 0 to 6.

Additionally, thanks to their amphiphilic nature, the lipo-polyamino acid conjugates of the invention may form self-assembled particles in solution such as for example liposomes or lipid nanoparticles which may be used to encapsulate and deliver molecules of different nature such as nucleic acids. Thus, the lipo-polyamino acid conjugates of the invention allow carrying and/or delivering different active agents at the same time, contained in self-assembled particles formed from the conjugated. Accordingly, another aspect of the invention relates to a self-assembled particle comprising the lipo-polyamino acid conjugate of formula (I) as defined herein, and one or more nucleic acids.

The lipo-polyamino acid conjugates of the invention may be formulated in a variety of compositions, including pharmaceutical, cosmetic and diagnostic compositions, with excipients and carriers. Thus, another aspect of the invention relates to a composition comprising the lipo-polyamino acid conjugate as defined herein; or alternatively, the self-assembled particle containing them, together with one or more appropriate excipients or carriers.

The lipo-polyamino acid conjugates of the invention, self-assembled particles, and compositions thereof may be used in medicinal, cosmetic and diagnostic applications.

Thus, another aspect of the invention relates to a therapeutic product which comprises: a) a self-assembled particle as defined herein; or alternatively, b) a composition comprising the self-assembled particle a), for use in medicine.

According to another aspect, the invention relates to a diagnostic product which comprises: a') a self-assembled particle as defined herein and one or more diagnostically active agents; or alternatively, b') a composition containing the self-assembled particle a'), for use in diagnostics.

## Brief Description of Drawings

FIG. 1 shows an agarose gel (2%) for the detection of free mRNA from different LNPs formulations: M) free mRNA; 1) benchmark LNPs formulation; 2) benchmark LNPs formulation + 10% triton X-100 and 2 mg/mL heparin; 3) LNP4 formulation; 4) LNP4 formulation + 10% triton X-100 and 2 mg/mL heparin; 5) LNP9 formulation; 6) LNP9 formulation + 10% triton X-100 and 2 mg/mL heparin; 7) LNP12 formulation; 8) LNP12 formulation + 10% triton X-100 and 2 mg/mL heparin; 9) LNP11 formulation; 10) LNP11 formulation + 10% triton X-100 and 2 mg/mL heparin.

FIG. 2 shows the *in vivo* mRNA transfection capacity in mice of a LNP comprising a lipo-polyamino acid conjugate according to the invention (LNP15) and comparative LNPs using DMG-PEG as lipo-shielding compound (total counts per g of tissue in liver (A), ALNs (B), spleen (C), lungs (D)).

## Detailed description of the invention

All terms as used herein in this application, unless otherwise stated, shall be understood in their ordinary meaning as known in the art. Other more specific definitions for certain terms as used in the present application are as set forth below and are intended to apply throughout the description and claims.

The term "about" or "around" as used herein refers to a range of values $\pm$ 10% of a specified value. For example, the expression "about 10" or "around 10" includes $\pm$ 10% of 10, i.e. from 9 to 11.

As used herein, the indefinite articles "a" and "an" are synonymous with "at least one" or "one or more." Unless indicated otherwise, definite articles used herein, such as "the" also include the plural of the noun.

The term "moiety" refers to a specific segment or functional group of a molecule or compound.

As used herein, the term "subject" refers to any mammal, including both human and other mammals.

The term $-(C_1-C_x)$alkyl refers to a saturated linear or branched hydrocarbon chain which contains from 1 to x carbon atoms and only single bonds. Examples of alkyl groups may include without limitation methyl, ethyl, n-propyl, isopropyl, n-butyl, sec-butyl, tert-butyl, pentyl, neopentyl, n-hexyl, decyl, undecyl, dodecyl, tetradecyl and hexadecyl. The term $-(C_2-C_x)$alkenyl refers to an unsaturated branched or linear hydrocarbon chain which comprises from 2 to x carbon atoms and at least one or more double bonds. Examples of alkenyl groups may include without limitation ethenyl (i.e. vinyl), allyl, propenyl, butenyl, pentenyl and hexenyl, dodecenyl, tetradecenyl and hexadecenyl.

The term "$-(C_1-C_{10})$alkoxy" refers to an alkyl group as defined herein comprising 1 to 10 carbon atoms which is connected to the rest of the molecule via an oxygen atom. Examples of alkoxy groups may include without limitation methoxy, ethoxy, 1-propoxy, and 2-propoxy.

The term "$-(C_5-C_{10})$aryl" refers to an aromatic carbocyclic mono- or bicyclic ring system comprising 5 to 10 carbon ring atoms. Examples of aryl groups may include without limitation phenyl, biphenyl, and naphthyl.

The term "$-(C_6-C_{10})$aryloxy" refers to an aryl group as defined herein comprising 6 to 10 carbon ring atoms, which is connected to the rest of the molecule via an oxygen atom. Examples of aryloxy groups may include without limitation

phenoxy and biphenyloxy.

The term "-($C_5$-$C_{10}$)heteroaryl" refers to an aromatic carbocyclic mono- or bicyclic ring system comprising 5 to 10 ring atoms, wherin at least one aromatic ring containing one, two, or three ring heteroatoms selected from N, O, and S, the remaining ring atoms being carbon atoms. Examples of heteroaryl groups may include without limitation pyridyl, azetidinyl, furyl, pyrrolyl, thienyl, thiazolyl, oxazolyl, imidazolyl, tetrazolyl, benzofuryl, isoquinolinyl, benzthiazolyl, benzimidazolyl, benzthiazolyl, quinolyl, and quinazolyl.

The term "-($C_6$-$C_{10}$)aralkyl" refers to a lower alkyl group as defined herein (e.g. -($C_1$-$C_6$)alkyl) which is substituted by an aryl group (e.g. "-($C_5$-$C_{10}$)aryl) as defined herein. Examples of aralkyl groups may include without limitation benzyl, phenethyl, and methylbenzyl.

The term "-($C_6$-$C_{10}$)aralkoxy" refers to a lower alkoxy group as defined herein (e.g. -($C_1$-$C_6$)alkoxy) which is substituted by an aryl group (e.g. "-($C_5$-$C_{10}$)aryl) as defined herein. Examples of aralkoxy groups may include without limitation benzyloxy, phenethoxy, and methylbenzyloxy.

The term "-($C_5$-$C_{10}$)heteroaralkoxy" refers to a lower alkoxy group as defined herein (e.g. -($C_1$-$C_6$)alkoxy) which is substituted by an heteroaryl group (e.g. "-($C_5$-$C_{10}$)heteroaryl) as defined herein. Examples of heteroaralkoxy groups may include without limitation furyloxy, pyridyloxy, and azetidinyloxy.

The term "-($C_5$-$C_{10}$)heterocycloalkyl" refers to a 5-10 membered mono- or bicylic (fused or bridged) saturated o unsaturated ring structure, in which one or more of the ring atoms is a heteroatom selected from N, O, and S. Examples of a heterocycloalkyl groups may include without limitation include piperidyl, piperazyl, tetrahydropyranyl, tetrahydrofuryl, 1,4-dioxolanyl, oxazolidyl, isoxazolidyl, morpholinyl, thiomorpholyl.

As used herein, the term "protective group" refers to a grouping of atoms that when attached to a reactive group in a molecule masks, reduces or prevents that reactivity. Protective groups for carboxyl and amino groups are described for example in T. W. Green and P.G. M. Wuts, Protective Groups in Organic Chemistry (Wiley, 3rd ed. 1999) in Chapter 5 (pp. 369-451) and Chapter 7 (pp. 495-653), respectively.

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. It is possible that groups in the conjugates according to the invention are substituted with one, two, three, four or five identical or different substituents, particularly with one, two or three substituents.

In the embodiments of the invention where the substitution or unsubstitution of a certain group is not specified, i.e., a certain substitution for that group is not indicated, nor is it indicated that the group is unsubstituted, it has to be understood that the possible substitution of this group is the broadest one as defined herein.

<u>Lipo-polyamino acid conjugates</u>

A first aspect of the invention refers to a lipo-polyamino acid conjugate of formula (I), a salt thereof, or any stereoisomer or mixtures of stereoisomers, either the compound of formula (I), or of any of its salts.

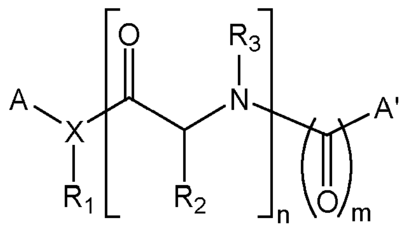

(I)

wherein A, X, $R_1$, $R_2$, $R_3$, n, m, and A' are as defined herein.

For the purposes of the invention, the term "lipo-polyamino acid conjugate" refers to a molecule which comprises a) a polyamino acid-based moiety and b) at least one lipid-like moiety. The term "lipid-like moiety" refers to a moiety which, while it cannot be considered to be a lipid as such, structurally and/or functionally resembles a lipid. The term lipid-like moiety is intended to include moieties that are able to form amphiphilic layers. The term "lipid" refers to a group of organic compounds such as fatty acids, glycerolipids, glycerophospholipids, sphingolipids, saccharolipids, sterols, and the like, which are insoluble in water.

There is no limitation on the type of salt of the lipo-polyamino acid conjugates that can be used, provided that these are pharmaceutically, cosmetically or diagnostically acceptable when they are used for pharmaceutical, cosmetic or diagnostic purposes, respectively. The term "pharmaceutically, cosmetically or diagnostically acceptable salts", embraces non-toxic salts commonly used. The preparation of pharmaceutically, cosmetically or diagnostically acceptable salts of the lipo-polyamino acid conjugates of the invention can be carried out by methods well-known in the art. Generally, such salts can be prepared by reacting the free acid or base form of a lipo-polyamino acid conjugate of the invention with a stoichiometric amount of an appropriate base or acid, respectively, in a suitable solvent such as water, an organic solvent or a mixture of them.

Examples of pharmaceutically, cosmetically or diagnostically acceptable salts include acid addition salts formed with inorganic acids e.g. hydrochloric, hydrobromic, sulfuric, nitric, hydroiodic, metaphosphoric, or phosphoric acid; and organic acids e.g. succinic, maleic, acetic, fumaric, citric, tartaric, benzoic, trifluoroacetic, malic, lactic, formic, propionic, glycolic, gluconic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), ethanesulfonic, pantothenic, stearic, sulfinilic, alginic and galacturonic acid; and arylsulfonic, for example benzenesulfonic, p-toluenesulfonic, oxalic, methanesulfonic or naphthalenesulfonic acid; and base addition salts formed with alkali metals and alkaline earth metals and organic bases such as N,N-dibenzylethylene-diamine, chloroprocaine, choline, diethanolamine, ethylenediamine, meglumine (N-methylglucamine), lysine and procaine; and internally formed salts. The lipo-polyamino acid conjugates of the invention and their salts may differ in some physical properties, but they are equivalent for the purposes of the present invention.

Some of the lipo-polyamino acid conjugates of the invention can have chiral centres that can give rise to various stereoisomers. As used herein, the term "stereoisomer" refers to all isomers of individual lipo-polyamino acid conjugates that differ only in the orientation of their atoms in space. The term stereoisomer includes mirror image isomers (enantiomers), mixtures of mirror image isomers (racemates, racemic mixtures), geometric (cis/trans or syn/anti or E/Z) isomers, and isomers of lipo-polyamino acid conjugates with more than one chiral center that are not mirror images of one another (diastereoisomers). The present invention relates to each of these stereoisomers and also mixtures thereof. Hence, the definition of the lipo-polyamino acid conjugates of formula (I) is also intended to encompass all R- and S-isomers of a chemical structure in any ratio, e.g. with enrichment (i.e. enantiomeric excess or diastereomeric excess) of one of the possible isomers and corresponding smaller ratios of other isomers. In the particular case of amino acids, they may acquire L-configuration or D-configuration.

Diastereoisomers and enantiomers can be separated by conventional techniques such as chromatography or fractional crystallization. Optical isomers may be obtained using enantiospecific synthesis. Alternatively, optical isomers can be resolved by conventional techniques to give optically pure isomers. The resolution can be carried out on any chiral synthetic intermediates or on the polymers of the invention.

In all embodiments of the invention referring to the lipo-polyamino acid conjugates, the pharmaceutically, cosmetically or diagnostically acceptable salts thereof and the stereoisomers or mixtures of stereoisomers, either of any of the lipo-polyamino acid conjugates or of any of their pharmaceutically, cosmetically or diagnostically acceptable salts are always contemplated even if they are not specifically mentioned.

The lipo-polyamino acid conjugates of the invention may be in crystalline form either as free solvation lipo-polyamino acid conjugates or as solvates (e.g. hydrates). It is intended that all these forms are within the scope of the present invention. Methods of solvation are generally known within the art. For the purposes of the invention, the solvated forms with pharmaceutically, cosmetically or diagnostically acceptable solvents such as water, ethanol and the like are equivalent to the unsolvated form.

Except as otherwise specified, the lipo-polyamino acid conjugates of the present disclosure also include lipo-polyamino acid conjugates that differ only in the presence of one or more isotope-enriched atoms. Examples of isotope-enriched atoms, without limitation, are deuterium, tritium, $^{13}C$ or $^{14}C$, or a nitrogen atom enriched in $^{15}N$ or a fluorine atom enriched in $^{18}F$.

The lipo-polyamino acid conjugates of the invention are polymeric structures containing as a repeating unit a polyamino acid (PAA)-based moiety of formula (XVIII)

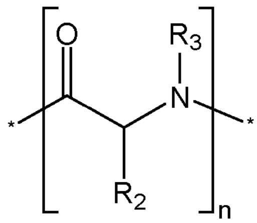

(XVIII)

wherein the "*" denote the attaching points of the PAA-based moiety to the rest of the molecule. The orientation of the PAA-based moiety does not influence the amphiphilic behavior of the polymers described herein, and consequently the activity displayed is maintained.

According to one embodiment, optionally in combination with any of the embodiments provided above or below, the lipo-polyamino acid conjugate of formula (I) has the formula (I'):

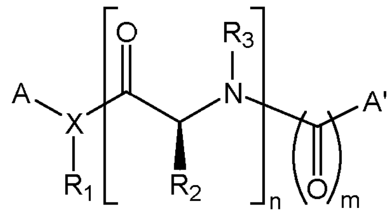

(I')

wherein A, A', X, $R_1$, $R_2$, $R_3$, n, and m are as defined herein.

As mentioned above, the lipo-polyamino acid conjugates of the invention further comprise one lipid-like moiety $R_5$, placed at the left side of the drawn molecule of formula (I).

The lipo-polyamino acid conjugates of the invention are characterized by a molecular weight, which can be average molecular weight (MW) or number average molecular weight (Mn), a degree of polymerization, and a polydispersity index. The molecular weight can be measured by methods well-known in the art such as size exclusion chromatography (SEC) (also referred to as gel permeation chromatography (GPC)) matrix assisted laser desorption ionization-time-of-flight mass spectrometry (MALDI-TOF MS). Composition and ratios are characterized by $^1$H-NMR spectroscopy among other techniques, for example. Some of these methods are indicated in more detail in the examples below.

As used herein, the term "repeating unit", refers to the monomeric amino acid-based unit and is defined by square brackets ("[ ]") depicted around it. The number on the lower right of the brackets (represented by letter "n") refers to the degree of polymerization (DP) for each monomeric unit as a statistical number. The DP is calculated by dividing the corrected molecular weight (subtracting the initiator MW) of the polymer determined by SEC-MALS technique by the molecular weight of the monomer unit. The DP value is reported as the central value of a gaussian distribution which comprises polymers of variable DP (depending on intrinsic polydispersity). The DP value is subject to a reasonable uncertainty, due to the ring-opening polymerization mechanism, which, in the context of the present invention, may be considered within the range $\pm$20%, preferably $\pm$15%, more preferably $\pm$10%, even more preferably $\pm$5%, being particularly preferred $\pm$2%.

The term "polydispersity index" (PDI) is used as a measure of broadness of molecular weight distribution. The larger the PDI, the broader the molecular weight. PDI of a polymer is calculated as the ratio of weight average (MW) by number average (Mn) molecular weight.

$$\text{Polydispersity index} = \frac{Mw}{Mn}$$

In the lipo-polyamino acid conjugate of formula (I), n is an integer from 5 to 250. In one embodiment, optionally in combination with any of the embodiments provided above or below, n is an integer from 5 to 150, more particularly from 5 to 100, even more particularly from 5 to 50, and even more particularly from 5 to 30 or from 5 to 25.

In the lipo-polyamino acid conjugate of formula (I), X is selected from N, S, and O. In one embodiment, optionally in combination with any of the embodiments provided above or below, X is N and $R_1$ is H. In another embodiment, optionally in combination with any of the embodiments provided above or below, X is N and $R_1$ is -($C_1$-$C_{18}$)alkyl, more particularly -($C_1$-$C_{12}$)alkyl. In another embodiment, optionally in combination with any of the embodiments provided above or below, X is N and $R_1$ is -($C_2$-$C_{18}$)alkenyl, more particularly -($C_2$-$C_{12}$)alkenyl.

According to another embodiment, optionally in combination with any of the embodiments provided above or below, in the lipo-polyamino acid conjugate of formula (I), X is O and $R_1$ is absent. In another embodiment, optionally in combination with any of the embodiments provided above or below, X is S and $R_1$ is absent.

In the lipo-polyamino acid conjugate of formula (I), m is 0 or 1 depending on the meaning of A'. In one embodiment, optionally in combination with any of the embodiments provided above or below, m is 0, and A' is selected from the group consisting of H and -($C_1$-$C_6$)alkyl. In another embodiment, optionally in combination with any of the embodiments provided above or below, m is 1, and A' is selected from the group consisting of H and -($C_1$-$C_6$)alkyl.

In the lipo-polyamino acid conjugate of formula (I), $R_2$ is a radical selected from the group consisting of (II), (III), (IV), and (V) wherein a, b, c, and d are independently an integer from 1 to 4.

In one embodiment, optionally in combination with any of the embodiments provided above or below, in the lipo-polyamino acid conjugate of formula (I), $R_2$ is a radical of formula (II), more particularly in the radical of formula (II), a is 1 or 2.

In accordance with another embodiment, optionally in combination with any of the embodiments provided above or below, in the lipo-polyamino acid conjugate of formula (I), $R_2$ is a radical of formula (III), more particularly in the radical of formula (III), b is 1 or 2.

In accordance with another embodiment, optionally in combination with any of the embodiments provided above or below, in the lipo-polyamino acid conjugate of formula (I), $R_2$ is a radical of formula (IV), more particularly in the radical of formula (IV), c is 1 or 2.

In another embodiment, optionally in combination with any of the embodiments provided above or below, in the lipo-polyamino acid conjugate of formula (I), $R_2$ is a radical of formula (V), more particularly in the radical of formula (V), d is 1 or 2.

In the lipo-polyamino acid conjugate of formula (I), $R_3$ is H.

In the lipo-polyamino acid conjugate of formula (I), A is a lipid-like moiety $R_5$.

In another embodiment, optionally in combination with any of the embodiments provided above or below, in the lipo-polyamino acid conjugate of formula (I), A is a lipid-like moiety $R_5$, and A' is other than a lipid-like moiety $R_5$'; more particularly, A' is selected from the group consisting of H and -($C_1$-$C_6$)alkyl.

The lipid-like moiety $R_5$ in A in the lipo-polyamino acid conjugate of formula (I) is independently selected from the group consisting of a radical of formula (IX), (X), (XII), (XIII), (XIV), (XIX) and (XX).

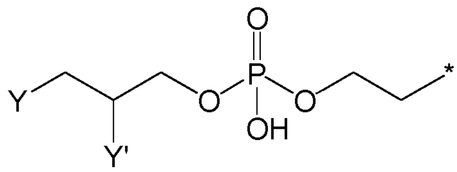

(IX)

;

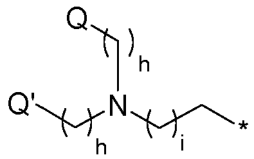

(X)

;

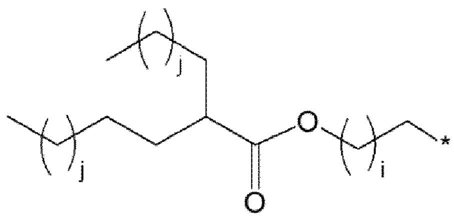

(XII)

;

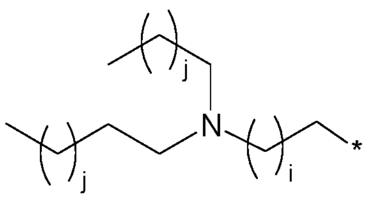

(XIII)

;

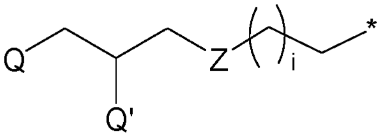

(XIV)

.

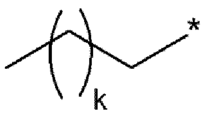 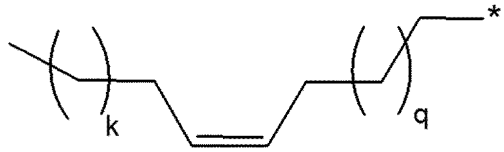

(XIX) (XX)

wherein Y, Y', Q, Q', Z, h, i, j, k, q, and t are as defined herein.

In one embodiment, optionally in combination with any of the embodiments provided above or below, the lipid-like moiety $R_5$ has the formula (XIX):

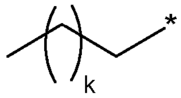

(XIX)

wherein k is 10, 11, 12, 13, 14, 15, or 16.

In another embodiment, optionally in combination with any of the embodiments provided above or below, the lipid-like moiety $R_5$ has the formula (XX):

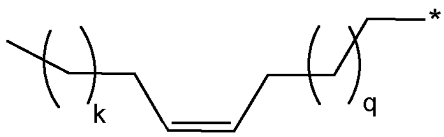

(XX)

wherein k is 10, 11, 12, 13, 14, 15, or 16, and q is an integer from 0 to 18, more particularly q is 0, 1, 2, 3, 4, 5, 6, 7, 8, 9, 10, 11, 12, 13, 14, 15, 16, 17, or 18.

In another embodiment, optionally in combination with any of the embodiments provided above or below, the lipid-like moiety $R_5$ has the formula (IX),

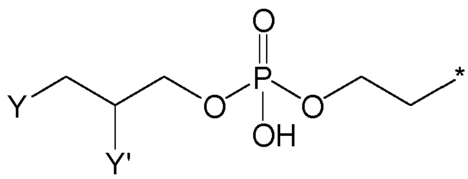

(IX)

wherein Y and Y' are independently selected from -OH, -COORx, and -OCORx; and each Rx is a radical independently selected from formula (ixa) and (ixb):

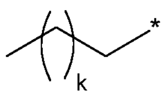 ; 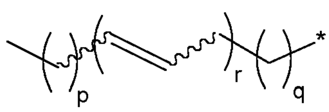

(ixa) (ixb)

wherein the wavy bonds mean that the stereochemistry of the double bonds is undefined (i.e. it includes cis and trans isomers), k is 10, 11, 12, 13, 14, 15, or 16; q is an integer from 0 to 18, more particularly q is independently 0, 1, 2, 3, 4, 5, 6, 7,

8, 9, 10, 11, 12, 13, 14, 15, 16, 17, or 18; p is 10, 11, 12, 13, 14, 15, 16, 17, or 18; and r is an integer from 0 to 6, more particularly r is 0, 1, 2, 3, 4, 5, or 6.

In one particular embodiment, optionally in combination with any of the embodiments provided above or below, in the lipid-like moiety $R_5$ of formula (IX), Y and Y' are the same. In another particular embodiment, optionally in combination with any of the embodiments provided above or below, in the lipid-like moiety $R_5$ of formula (IX), Y and Y' are different.

In another particular embodiment, optionally in combination with any of the embodiments provided above or below, the lipid-like moiety $R_5$ of formula (IX) is selected from the formulas (ix1), (ix2), (ix3), (ix4), (ix5), (ix6), (ix7) and (ix8):

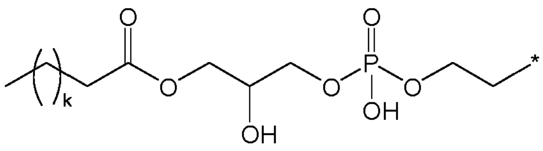

(ix1)

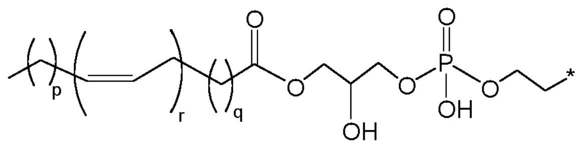

(ix2)

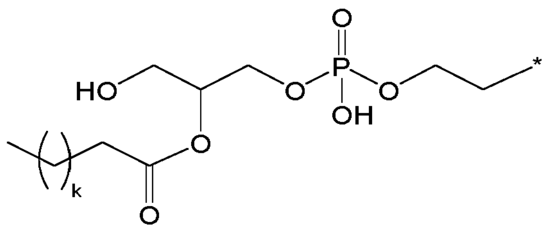

(ix3)

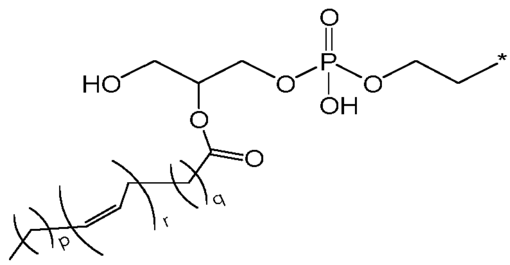

(ix4)

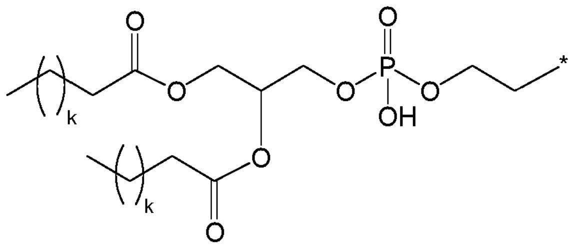

(ix5)

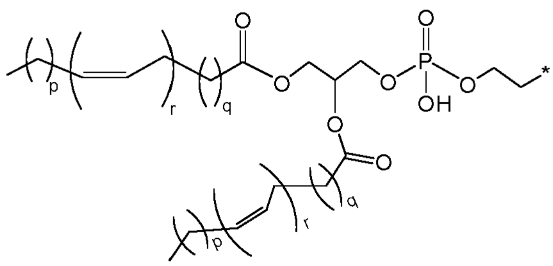

(ix6)

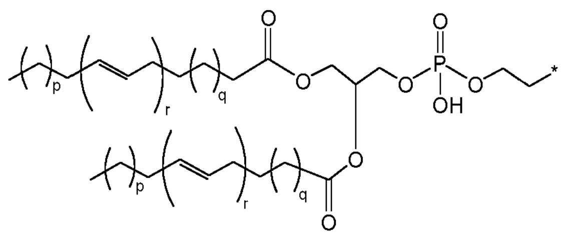

(ix7)

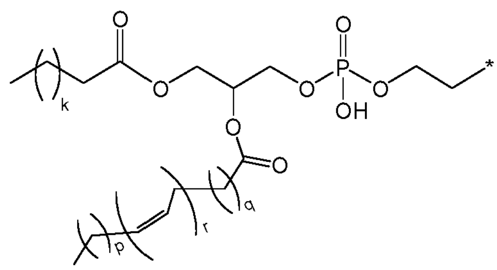

(ix8)

wherein each k is independently 10, 11, 12, 13, 14, 15, or 16; each q is independently an integer from 0 to 18, more particularly 0, 1, 2, 3, 4, 5, 6, 7, 8, 9, 10, 11, 12, 13, 14, 15, 16, 17, or 18; each p is independently 10, 11, 12, 13, 14, 15, 16, 17, or 18; and each r is independently an integer from 0 to 6, more particularly each r is independently 0, 1, 2, 3, 4, 5, or 6.

According to a particular embodiment, optionally in combination with any of the embodiments provided above or below, in the lipid-like moiety $R_5$ of formula (ix5), each k has the same meaning. According to another particular embodiment, optionally in combination with any of the embodiments provided above or below, in the lipid-like moiety $R_5$ of formulas (ix6) and (ix7), each p has the same meaning, each r has the same meaning, and each q has the same meaning.

In another embodiment, optionally in combination with any of the embodiments provided above or below, the lipid-like moiety $R_5$ has the formula (X),

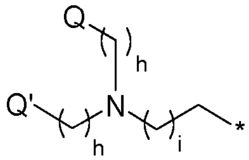

(X)

wherein Q and Q' are independently selected from -OCORy and -COORy; each h is independently an integer from 1 to 18, more particularly, each h is independently from 1 to 12, and even more particularly each h is independently 1, 2, 3, 4, 5, 6, 7, 8, 9, 10, 11, 12; and i is an integer from 0 to 12 more particularly, i is from 0 to 12, and even more particularly i is independently 0, 1, 2, 3, 4, 5, 6, 7, 8, 9, 10, 11, 12 and each Ry is a radical independently selected from formula (xa), (xb), and (xc):

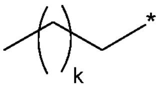

;

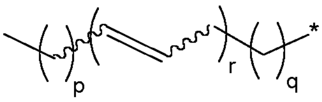

;

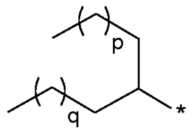

(xa) (xb) (xc)

wherein the wavy bonds mean that the stereochemistry of the double bonds is undefined (i.e. it includes cis and trans isomers), k is 10, 11, 12, 13, 14, 15, or 16; q is an integer from 0 to 18, more particularly q is 0, 1, 2, 3, 4, 5, 6, 7, 8, 9, 10, 11, 12, 13, 14, 15, 16, 17, or 18; p is 10, 11, 12, 13, 14, 15, 16, 17, or 18 and r is an integer from 0 to 6, more particularly r is 0, 1, 2, 3, 4, 5, or 6.

In one particular embodiment, optionally in combination with any of the embodiments provided above or below, in the lipid-like moiety $R_5$ of formula (X), Q and Q' are both -OCORy. In another particular embodiment, optionally in combination with any of the embodiments provided above or below, in the lipid-like moiety $R_5$ of formula (X), Q and Q' are both -COORy.

In another particular embodiment, optionally in combination with any of the embodiments provided above or below, the lipid-like moiety $R_5$ of formula (X) is selected from the formulas (x1), (x2), (x3), and (x4):

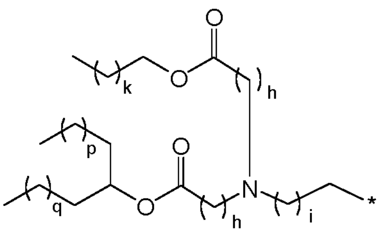

(x1)

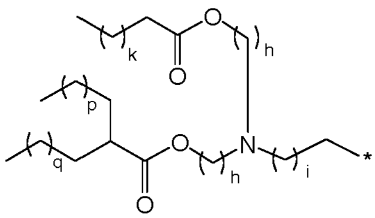

(x2)

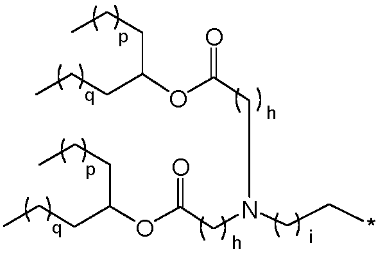

(x3)

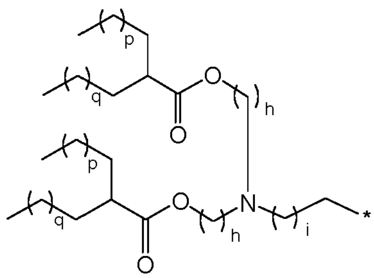

(x4)

wherein k is 10, 11, 12, 13, 14, 15, or 16; each q is independently an integer from 0 to 18, more particularly each q is independently 0, 1, 2, 3, 4, 5, 6, 7, 8, 9, 10, 11, 12, 13, 14, 15, 16, 17, or 18; each p is independently selected from 10, 11, 12, 13, 14, 15, 16, 17, or 18; each h is independently 1, 2, 3, 4, 5, 6, 7, 8, 9, 10, 11, 12; and i is an integer from 0 to 12; more particularly, i is 0, 1, 2, 3, 4, 5, 6, 7, 8, 9, 10, 11, 12.

In another embodiment, optionally in combination with any of the embodiments provided above or below, the lipid-like moiety $R_5$ has the formula (XII),

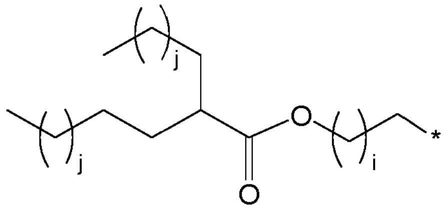

(XII)

wherein i is an integer from 0 to 18; more particularly, i is from 0 to 12, and even more particularly i is 0, 1, 2, 3, 4, 5, 6, 7, 8, 9, 10, 11, 12; and each j is independently 3, 4, 5, 6, 7, 8, 9, 10, 11, 12.

In another embodiment, optionally in combination with any of the embodiments provided above or below, the lipid-like moiety $R_5$ has the formula (XIII),

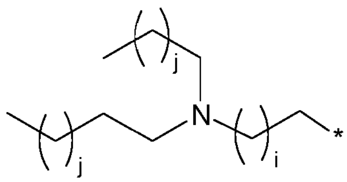

(XIII)

wherein i is an integer from 0 to 18; more particularly, i is from 0 to 12, and even more particularly i is 0, 1, 2, 3, 4, 5, 6, 7, 8, 9, 10, 11, 12; and each j is independently 10, 11, 12.

In another embodiment, optionally in combination with any of the embodiments provided above or below, the lipid-like moiety $R_5$ has the formula (XIV),

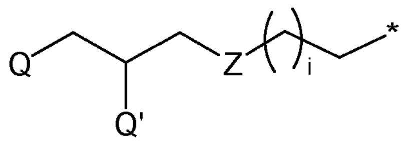

(XIV)

wherein Q and Q' are independently selected from -OCORy and -COORy; Z is selected from -OCO-, -COO-, - NRz'CO-, and -CONRz'-; each Rz' is H or Rz; each Rz is -($C_1$-$C_{18}$)alkyl or -($C_2$-$C_{18}$)alkenyl; i is an integer from 0 to 18, more particularly, i is from 0 to 12, and even more particularly i is 0, 1, 2, 3, 4, 5, 6, 7, 8, 9, 10, 11, 12 and each Ry is a radical independently selected from formula (xiva) and (xivb):

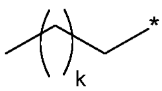

;

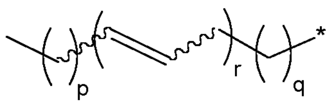

(xiva) (xivb)

wherein the wavy bonds mean that the stereochemistry of the double bonds is undefined (i.e. it includes cis and trans isomers), k is 10, 11, 12, 13, 14, 15, or 16; q is an integer from 0 to 18, more particularly q is independently 0, 1, 2, 3, 4, 5, 6, 7, 8, 9, 10, 11, 12, 13, 14, 15, 16, 17, or 18; p is 10, 11, 12, 13, 14, 15, 16, 17, or 18; and r is an integer from 0 to 6, more particularly r is 0, 1, 2, 3, 4, 5, or 6.

In one particular embodiment, optionally in combination with any of the embodiments provided above or below, in the lipid-like moiety $R_5$ of formula (XIV), Q and Q' are both -OCORy. In another particular embodiment, optionally in combination with any of the embodiments provided above or below, in the lipid-like moiety $R_5$ of formula (XIV), Q and Q' are both -COORy. In another particular embodiment, optionally in combination with any of the embodiments provided above or below, in the lipid-like moiety $R_5$ of formula (XIV), Z is -OCO-. In another particular embodiment, optionally in combination with any of the embodiments provided above or below, in the lipid-like moiety $R_5$ of formula (XIV), Z is -CONRz'.

In another particular embodiment, optionally in combination with any of the embodiments provided above or below, the lipid-like moiety $R_5$ of formula (XIV) is selected from the formulas (xiv1), and (xiv2):

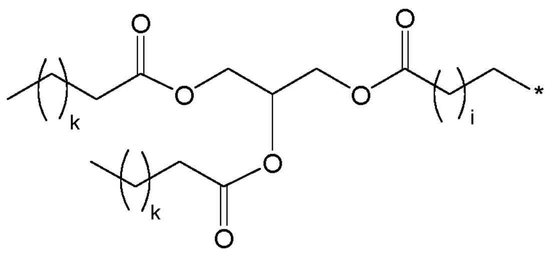

(xiv1)

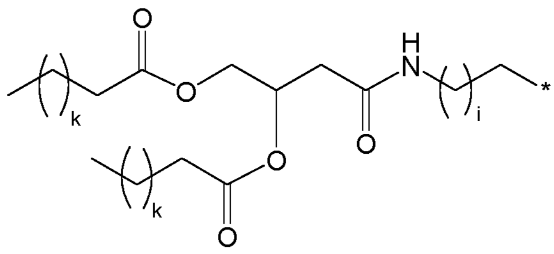

(xiv2)

wherein each k is 10, 11, 12, 13, 14, 15, or 16; and i is an integer from 0 to 12; more particularly, i is 0, 1, 2, 3, 4, 5, 6, 7, 8, 9, 10, 11, 12.

According to one embodiment, optionally in combination with any of the embodiments provided above or below, in the lipo-polyamino acid conjugate of formula (I) A is a lipid-like moiety $R_5$ selected from the group consisting of:

a) a radical of the formula (XIX) as previously defined;
b) -($C_6$-$C_{18}$)alkenyl or alternativelya radical of the formula (XX) as previously defined;
c) a radical of formula (IX) as defined herein, more particularly, a radical selected from the formulas (ix1), (ix2), (ix3), (ix4), (ix5), (ix6), (ix7) and (ix8) as previously defined;
d) a radical of formula (X) as defined herein, more particularly, a radical selected from the formulas (x1), (x2), (x3), and (x4) as previously defined;
e) a radical of formula (XII) as defined herein;
f) a radical of formula (XIII) as defined herein;
g) a radical of formula (XIV) as defined herein, more particularly, a radical selected from the formulas (xiv1) and (xiv2).

In accordance with one embodiment, optionally in combination with any of the embodiments provided above or below, in the lipo-polyamino acid conjugate of formula (I), A is a lipid-like moiety $R_5$ as defined herein, m is 0, and A' is H.

In another more particular embodiment, in the lipo-polyamino acid conjugate of formula (I) $R_2$ is a radical of formula (IV), A is a lipid-like moiety $R_5$ as defined herein, m is 0, i.e., the lipo-polyamino acid conjugate of formula (I) has the formula (Ig),

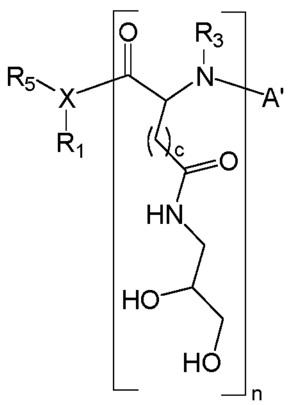

(Ig)

wherein X, $R_1$, $R_3$, $R_5$, n, and c are as defined herein, and A' is selected from the group consisting of H and -($C_1$-$C_6$)alkyl.

In another more particular embodiment, in the lipo-polyamino acid conjugate of formula (I) $R_2$ is a radical of formula (IV), A is a lipid-like moiety $R_5$ as defined herein, m is 1, i.e., the lipo-polyamino acid conjugate of formula (I) has the formula (Ig'),

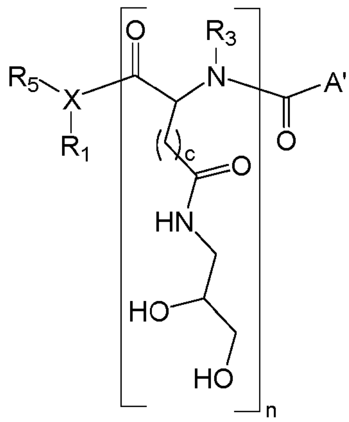

(Ig')

wherein X, $R_1$, $R_3$, $R_5$, n, and c are as defined herein, A' is selected from the group consisting of H, and -($C_1$-$C_6$)alkyl.

Preparation processes

Processes for the preparation of the lipo-polyamino acid conjugates of formula (I) are also part of the invention. The conjugates may be prepared by synthetic processes which are well-known in the art.

As mentioned above, the lipo-polyamino acid conjugates of formula (I) must contain one lipid-like moiety $R_5$ in A.

A lipo-polyamino acid conjugate of formula (I) wherein A is a lipid-like moiety $R_5$ may be prepared by ring opening polymerization (ROP) of a N-carboxy anhydride (NCA). In particular an oxazolidine-2,5-dione derivative of an amino acid containing $R_2$ as side chain (compound of formula (A)) may react with an amine, alcohol or thiol of formula (B) which already contains the lipid-like moiety $R_5$ and is used as initiator. As a result, a polyamino acid polymer of formula (I) is obtained:

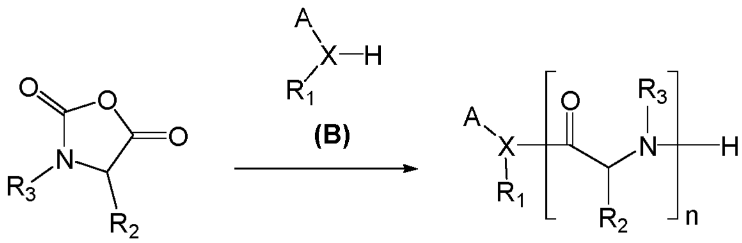

**(A)** **(B)** **(If)**

wherein m is 0, A' is H; $R_1$, $R_2$, $R_3$, and n are as defined herein; and A is $R_5$. The reaction is carried out in a suitable solvent such as dimethylformamide, at a temperature comprised from 4 °C to room temperature, preferably at about 10 °C. The ratio monomer (NCA)/initiator (alcohol/amine) allow to control the degree of polymerization (DP).

Alternatively, the above reaction may also be carried out using an NCA containing a precursor of $R_2$ instead of a $R_2$ moiety, and the $R_2$ precursor is later on converted into a $R_2$ moiety once the polymer is formed. For example, a NCA compound derived from a natural amino acid, such as glutamic acid or lysine, optionally protected with a suitable protective group, may be polymerized in the presence of a compound (B) to give a lipo-polyamino acid conjugate of formula (If) wherein $R_2$ is the side chain of the natural amino acid which has been used. This precursor may be deprotected, if needed, and further reacted with other reagents to obtain the desired $R_2$ moieties as illustrated in the examples below.

The obtained polyamino acids may be optionally purified by any known methods such as fractionation, precipitation, ultrafiltration, dialysis, size-exclusion chromatography, affinity chromatography or tangential flow filtration

If desired the polymer conjugates of formula of formula (If) may be converted into further polymer conjugates of formula (I) by reacting the terminal amine with further groups

Self-assembled particles

As mentioned above, the lipo-polyamino acid conjugates of the invention have amphiphilic nature since they comprise a polyamino acid-based moiety (hydrophilic part) and at least one lipid-like moiety (hydrophobic part). Thanks to their amphiphilic nature, the lipo-polyamino acid conjugates of the invention may form self-assembled particles in solution.

Therefore, the present invention also relates to a self-assembled particle comprising the lipo-polyamino acid conjugate of formula (I) as defined herein, and one or more nucleic acids.

As used herein, the term "self-assembled particles" refers to the arrangement of the lipo-polyamino acid conjugate molecules in a solvent, in particular, small, self-assembled particle may be enclosed structures of any shape, typically spherical and/or tubular. The term "self-assembled particles" intends to encompass any of a number of structures that are known in the art to be formed from amphiphilic polymers. Non-limiting examples of self-assembled particles include micelles (also being referred to interchangeably herein as micellar worms or simply "worms"), inverted micelles, planar bilayers, crystal nanoparticles, liposomes, microbubbles or lipid nanoparticles. The self-assembled nanoparticles and microparticles can also form gels. The self-assembled particles of the invention are non-viral particles, which means that they are not able to virally infect cells.

The self-assembled particles may show a variety of sizes, in particular, they can be nanoparticles or microparticles. A "nanoparticle", as defined herein, is any particle of nanometric size, in particular having smallest end-to-end diameter of between 1 and 900, more particularly, between 1 and 700 nm, between 1 and 500 nm, between 1 and 300 nm, between 1 and 200 nm, and between 1 and 100 nm in size. A "microparticle", as defined herein, is typically any particle of micrometric size, having a smallest end-to-end between 1 and 100 μm in size. Typically, in a composition comprising a plurality of particles, the relevant diameter is the number average diameter.

As used herein, the term "size" refers to a characteristic physical dimension. For example, in the case of a particle that is substantially spherical, the size of the particle corresponds to the diameter of the particle. In the case of a particle that is substantially rod-shaped with a substantially circular cross-section, such as wire or a tube, the size of the particle is determined by the diameter of the two relevant cross-section dimensions of the particle. In the case of a particle that is substantially box-shaped, such as a cube, a box, or a cage, the size of the nanoparticle corresponds to the maximum edge length. When referring to a set of particles as being of a particular size, it is contemplated that the set of particles can have a distribution of sizes around the specified size. Thus, as used herein, a size of a set of particles can refer to a mode of a distribution of sizes, such as a peak size of the distribution of sizes.

As used herein, the term "diameter" refers to the average diameter and is also designated as Z-average or Z-ave. The average diameter corresponds to the mean hydrodynamic diameter (Dh) and can be measured by dynamic light scattering (DLS) as shown in the examples below. In one embodiment, optionally in combination with one or more features of the various embodiments described above or below, the self-assembled particles of the invention have a hydrodynamic diameter (Dh) in water from 5 to 1200 nm, more particularly, from 10 to 1100 nm, from 10 to 1000 nm, from 10 to 700, from 20 to 500 nm, from 20 to 400 nm, from 20 to 300 nm, from 30 to 200 nm, or from 50 to 150 nm.

All embodiments indicated for the lipo-polyamino acid conjugate of formula (I) also apply to the self-assembled particles.

According to one embodiment, optionally in combination with one or more features of the various embodiments described above or below, in the self-assembled particle comprising the lipo-polyamino acid conjugate of formula (I), the lipid-like moieties (hydrophobic part) face inward and the polar head portion (the PAA) face outward.

According to another embodiment, optionally in combination with one or more features of the various embodiments described above or below, the self-assembled particle is a core-shell structure comprising an inner core and an external shell. More particularly, the lipo-polyamino acid conjugate of formula (I) forms the external shell of the self-assembled particle, and one or more nucleic acids are encapsulated or loaded in the inner core.

According to one embodiment, optionally in combination with one or more features of the various embodiments described above or below, the self-assembled particle is a nanoparticle, more particularly selected from the group consisting of a micelle, an inverted micelle, a planar bilayer, a crystal nanoparticle, a liposome, microbubbles, and a lipid nanoparticle, and even more particularly, the self-assembled particle is a lipid nanoparticle or a liposome.

For the purposes of the invention, the term "liposome" refers to artificially prepared self-assembled particles composed of concentric lipidic bi-layers enclosing one or more aqueous compartments. Non-limiting examples of liposomes include monolamellar liposome, multi-lamellar liposomes, multi-vesicular-liposomes and polymer-coated liposomes.

The term "lipid nanoparticle" refers to particles typically spherical on the order of nanometers (e.g., 1-1000 nm) that includes lipids and that is stable and dispersible in aqueous media.

For the purposes of the invention, the term "crystal nanoparticle" refers to artificially prepared crystals composed of solid lipids enclosing one or more hydrophobic compartments. The term "crystal nanoparticle" refers to particles of different shapes on the order of nanometers (e.g., 1-1000 nm) that includes lipids and that is stable and dispersible in aqueous media.

In another embodiment, optionally in combination with one or more features of the various embodiments described above or below, the invention relates to a lipid nanoparticle (LNP) or a liposome comprising the lipo-polyamino acid conjugate of formula (I) as defined herein, and one or more nucleic acids.

The liposomes and lipid nanoparticles disclosed herein may comprise any suitable lipids, including ionizable lipids, cationic lipids, zwitterionic lipids, neutral lipids, or anionic lipids.

Suitable ionizable lipids include, without limitation, (2S)-2,5- bis(3-aminopropylamino)- N-[2-(dioctadecyl-amino)acetyl]pentanamide (DOGS), N1-[2-((1S)-1-[(3-aminopropyl)amino]-4-[di(3-aminopropyl)amino]-butylcarboxamido) ethyl]-3,4-di[oleyloxy]-benzamide (MVL5), 3β-[N-(N',N'- dimethylaminoethane)-carbamoyl]cholesterol (DC-Cholesterol), (1,2-distearyloxy-N,N-dimethyl-3-aminopropane (DSDMA), 1,2-dilinoleyloxy-N,N-dimethyl-3-aminopropane (DLin-DMA), 1,2-dilinolenyloxy-N,N-dimethyl-3-aminopropane (DLenDMA), 1,2-di-y-linolenyloxy-N,N-dimethylaminopropane (y-DLenDMA), 2-dilinoleyl-4-dimethylaminomethyl-[1,3]-dioxolane (DLin-K-DMA), 2,2- dilinoleyl-4- dimethylaminoethyl-[1,3]- dioxolane (DLin- KC2-DMA, also known as DLin-C2K-DMA, XTC2, and C2K), 2,2-Dilinoleyl-4-(3-dimethylaminopropyl)-[1,3]-dioxolane (DLin-KC3-DMA), 2,2-Dilinoleyl-4-(4-dimethylaminopropyl)-[1,3]-dioxolane (DLin-KC4-DMA), 1,2-dilinoleny loxy-4-(2-dimethylaminoethyl)-1,3-dioxolane (DLen-C2K-DMA), 1,2-di- y-linolenyloxy-4-(2-dimethylaminoethyl)-1,3-dioxolane (y-DLen-C2K-DMA), (6Z,9Z,28Z,31Z)-Heptatriaconta-6,9,28,31-tetraen-19-yl 3-(dimethylamino)propanoate (DLin-M-C2-DMA, also known as MC2), (6Z,9Z,28Z,31Z)- heptatriaconta-6,9,28,31- tetraen-19- yl 4-(dimethylamino)-butanoate (DLin-M-C3-DMA, also known as MC3) and 3-((6Z,9Z,28Z,31Z)-heptatriaconta-6,9,28,31-tetraen-19-yloxy)-N,N-dimethylpropan-1-amine (DLin-MP-DMA, also known as 1-B11).

Suitable cationic lipids include, without limitation, N,N-dioleyl-N,N-dimethylammonium chloride (DODAC), N,N-distearyl-N,N-dimethylammonium bromide (DDA13), N-(1-(2,3-dioleoyloxy)propyl)-N,N,N-trimethylammonium chloride (DOTAP), N-(1-(2,3-dioleyloxy)-propyl)-N,N,N-trimethylammonium chloride (DOTMA), N-[1-(2,3,-ditetradecyloxy)propyll-N,N-dimethyl-N-hydroxyethylammonium bromide (DMRIE), N-[1-(2,3,dioleyloxy)-propyl]-N,N-dimethyl-N-hydroxy ethylammonium bromide (DORIE), 3β-[N-(N',N'-dimethylamino-ethane)-carbamoyl]cholesterol (DC-Chol), dimethyldioctadecylammonium (DDAB), 2,3-dioleyloxy- N-[2-(spermine-carboxamido)ethyl]- N,N- dimethyl-1- propanaminium trifluoroacetate (DOSPA), ethylphosphatidylcholine, (ePC), N,N-dimethyl-2,3-dioleyloxy)propylamine (DODMA), and ([(4-hydroxybutyl)azanediyl]di(hexane-6,1-diyl) bis(2-hexyldecanoate)) (ALC-0315).

Examples of anionic lipids include, but are not limited to, phosphatidylglycerol, diacylphosphatidylserine, diacylphosphatidic acid, N-Succinyl phosphatidylethanolamine, N-glutaryl phosphatidylethanolamine cholesterol hemisuccinate (CHEMS), lysylphos-phatidylglycerol, N-dodecanoyl phosphatidyl ethanoloamine, cardiolipin, and combinations thereof.

Suitable neutral lipids may be uncharged or zwitterionic lipids and include, without limitation steroids, phospholipids, and combinations thereof.

Examples of steroids include, without limitation, cholesterol, progesterone, cortisone, aldosterone, estradiol, testosterone, and combinations thereof.

Examples of phospholipids include, but are not limited to, phosphatidylcholine (PC), phosphatidic acid (PA), phosphatidylethanolamine (PE), phosphatidylglycerol (PG), phosphatidylserine (PS), and phosphatidylinositol (PI), dimyristoyl phosphatidyl choline (DMPC), distearoyl phosphatidyl choline (DSPC), dioleoyl phosphatidyl choline (DOPC), dipalmitoyl phosphatidyl choline (DPPC), dimyristoyl phosphatidyl glycerol (DMPG), distearoyl phosphatidyl glycerol (DSPG), dioleoyl phosphatidyl glycerol (DOPG), dipalmitoyl phosphatidyl glycerol (DPPG), dimyristoyl phosphatidyl serine (DMPS), distearoyl phosphatidyl serine (DSPS), dioleoyl phosphatidyl serine (DOPS), dipalmitoyl phosphatidyl serine (DPPS), dioleoyl phosphatidyl ethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyp-cyclohexane-1-carboxylate (DOPE-mal), dipalmitoyl phosphatidyl ethanolamine (DPPE), dimyristoylphosphoethanolamine (DMPE), distearoylphosphatidyl-ethanolamine (DSPE); lysophosphatidyl choline, lysophosphatidylethanolamine, 1,2-distearoyl-sn-glycero-3-phosphocholine (DAPC), dilauryloylphosphatidylcholine (DLPC), 1-myristoyl-2-palmitoyl phosphatidylcholine (MPPC), 1-palmitoyl-2-myristoyl phosphatidylcholine (PMPC), 1-palmitoyl-2-stearoyl phosphatidylcholine (PSPC), 1,2-diarachidoyl-sn-glycero-3-phosphocholine (DBPC), 1-stearoyl-2-palmitoyl phosphatidylcholine (SPPC), 1,2-dieicosenoyl-sn-glycero3-phosphocholine (DEPC), and combinations thereof.

In the liposomes and lipid nanoparticles disclosed herein the lipo-polyamino acid conjugate advantageously provides stealth properties. As used herein, the term "stealth" refers to the fact that the liposomes or lipid nanoparticles are not detected and sequestered and/or degraded by the immune system of the host to which they are administered, and consequently the length of time for which the liposomes or lipid nanoparticles can exist *in vivo* is increased.

In one embodiment, optionally in combination with one or more features of the various embodiments described above or below, the liposomes and lipid nanoparticles disclosed herein do not comprise (i.e., lack) a polyethyleneglycol (PEG)-lipid conjugate, that is a lipid containing polyethyleneglycol.

In another embodiment, optionally in combination with one or more features of the various embodiments described above or below, the self-assembled particles of the invention, in particular liposomes and lipid nanoparticles, lack polyethyleneglycol (PEG).

The liposomes and lipid nanoparticles disclosed herein may also comprise one or more cationic polymers such as cationic amine-containing polymers. Typically, these polymers may carry active ingredients, particularly nucleic acids. Examples of cationic amine-containing polymers include, without limitation, poly-L-lysine, polyamidoamine, polyethyleneimine, chitosan, poly(beta-amino esters), and the like.

In another embodiment, optionally in combination with one or more features of the various embodiments described above or below, the invention relates to a lipid nanoparticle (LNP) or a liposome comprising the lipo-polyamino acid conjugate of formula (I) as defined herein, and one or more lipids selected from the group consisting of ionisable lipids, cationic lipids, neutral lipids, and anionic lipids. More particularly, the lipid nanoparticle (LNP) or liposome comprises the lipo-polyamino acid conjugate of formula (I) as defined herein, a ionizable lipid or a cationic lipid, a phospholipid, and a sterol. Even more particularly, the lipid nanoparticle (LNP) or liposome comprises: i) the lipo-polyamino acid conjugate of formula (I) as defined herein in an amount from 0.1 to 10 mol%, more particularly from 1 to 5 mol% or from 1 to 6 mol%, even more particularly from 2 to 5% mol%; ii) a ionizable lipid or a cationic lipid in an amount from 30 to 70 mol%, more particularly from 40 to 60 mol%; iii) a phospholipid in an amount from 1 to 20 mol%, more particularly from 5 to 15 mol%; and iv) a sterol in an amount from 20 to 60 mol%, more particularly from 30 to 50 mol%; wherein the percentages are expressed with respect to the sum of the mol% of the lipids and the lipo-polyamino acid conjugate of formula (I).

In another embodiment, optionally in combination with one or more features of the various embodiments described above or below, the invention relates to a lipid nanoparticle (LNP) or a liposome comprising the lipo-polyamino acid conjugate of formula (I) as defined herein, one or more lipids selected from the group consisting of ionisable lipids, cationic lipids, neutral lipids, and anionic lipids, and one or more nucleic acids.

The lipid nanoparticles and the liposomes containing one or more nucleic acids may be prepared by standard methods. Typically, the process for the preparation of LNPs comprises: i) preparing a first alcoholic mixture comprising one or more lipids and the lipo-polyamino acid conjugate of the invention in a suitable alcohol such as for example ethanol; ii) preparing a second aqueous composition comprising one or more nucleic acids and an aqueous solvent (an acidic buffer); and iii) mixing i) with ii) in a microfluidic mixing device. The microfluidic mixing allows thorough and rapid mixing of the lipid phase and the nucleic acid phase in a microscale device. Depending on the process parameters, and in particular on the total flow rate, the skilled person will be able to modulate the size of the LNPs.

Active agents

As used herein, the term "pharmaceutically active agent" refers to and agent that has pharmacological activity and is used for curing, mitigating, treating or preventing a disease in a mammal, in particular a human. The term "cosmetic active agent" refers to an agent that does not provide any therapy but is used for aesthetic purposes, for example to improve the appearance, preserve, condition, cleanse, color or protect the skin, nails or hair.

As used herein, the term "cell-targeting agent" refers to any molecule, macromolecule, or biomacromolecule, displaying affinity for a molecule present in the human or animal body, which is able to direct the conjugates or the self-assembled particles thereof by directing them towards the target site for therapeutic treatment since e.g., it selectively binds to receptors that are expressed or over-expressed on specific cell types. Cell-targeting groups are well known in the art. The term therefore includes ligands for specific receptors or antigens, such as antibodies for a specific antigen, folic acid for its receptor or sugars such as galactose for its hepatic receptors. The targeting agent may be contained in the self-assembled particles formed from the conjugated.

Examples of cell-targeting groups include, but are not limited to, galactosamine, folate, a Her-2 binding peptide, TLR agonists, β-D-Glucose, Asn-Gly-Arg peptide, angiopep2, folic acid, aptamers (A-9, A10, Anti-gp120, TTA1, sgc8, Anti MUC-1, AS1411), primaquine, zidovudine, superoxide dismutase, prednisolone, platinum, cisplatin, sulphamethoxazole, amoxicillin, etoposide, mesalzine, doxorubicin, paclitaxel, 5-amino salicylic acid, denosumab, docetaxel, calcitonin, proanthocyanidin, methotrexate, camptothecin, galactose, glycyrrhetinic acid, lactose, hyaluornic acid, octeotride, lactobionic acid, β-galactosyl moiety, arabino-galactan, chitosan, azo-based poly-phosphazene, azo group and 4-amino-benzyl-carbamate, succinate, 4,4’-dihydroxyazo benzene-3-carboxilic acid, cyclic RGD penta-peptide, Aspartic acid octapeptide, alendronate, transferrin, bisphosphonate adendronate, mono sialoganglioside GM1, gluthatione, E-selectin thioaptamer, poloxamer-407, a urokinase-type plasminogen activator receptor (uPAR) antagonist, a CXCR4 chemokine receptor antagonist, a GRP78 peptide antagonist, an RGD peptide, an RGD cyclic peptide, a luteinizing hormone-releasing hormone (LHRH) antagonist peptide, an aminopeptidase targeting peptide, a brain homing peptide, a kidney homing peptide, a heart homing peptide, a gut homing peptide, an integrin homing peptide, an angiogenic tumor endothelium homing peptide, an ovary homing peptide, a uterus homing peptide, a sperm homing peptide, a microglia homing peptide, a synovium homing peptide, a urothelium homing peptide, a prostate homing peptide, a lung homing peptide (e.g. RCPLSHSLICY), laminin receptor binding peptide (e.g. YIGSR) a skin homing peptide, a retina homing peptide, a pancreas homing peptide, a liver homing peptide, a lymph node homing peptide, an adrenal gland homing peptide, a thyroid homing peptide, a bladder homing peptide, a breast homing peptide, a neuroblastoma homing peptide, a lymphoma homing peptide, a muscle homing peptide, a wound vasculature homing peptide, an adipose tissue homing

peptide, a virus binding peptide, or a fusogenic peptide.

The term "penetration enhancing agent" as used herein refers to moiety or compound that increases the permeability of an active agent, in particular selected from a pharmaceutically active agent, a cell-targeting agent, a cosmetically active agent, and a diagnostically active agent. The penetration enhancing agent is also known as permeation enhancer.

Examples of penetration enhancing agents include, without limitation, cell penetrating peptides, surfactants, terpenes, sulfoxides, pyrrolidones, fatty acids, fatty alcohols, urea, azones, fatty alcohols, fatty acids, fatty esters; such as for example, lauryl sarcosine, octoxynol, phenylsulfonate, pluronic, sodium laurate, sodium oleate, sorbitan dilaurate, sorbitan dioleate, sorbitan trilaurate, sorbitan trioleate, sodium octyl sulfate, alkyl ammonium halides, decanol, dodecanol, linolenyl alcohol, oleyl alcohol, butyl acetate, cetyl lactate, lauryl lactate, myristyl lactate, diethyl sebacate, diethyl succinate, diisopropyl sebacate, glycerol monolaurate, glycerol monooleate, glycerol monolinoleate, isopropyl isostearate, isopropyl linoleate, isopropyl myristate, isopropyl palmitate, methyl caprate, methyl laurate, methyl valerate, octyl acetate, oleyl oleate, sorbitan dilaurate, dodecyl acetate, sorbitan dioleate, sorbitan monolaurate, sorbitan trilaurate, sorbitan trioleate, capric acid, hexanoic acid, lactic acid, lauric acid, linoleic acid, linolenic acid, neodecanoic acid, oleic acid, palmitic acid, lecithin, phospholipids, sodium deoxycholate, and sodium taurocholate.

For the purposes of the present invention, pharmaceutically active agents include low molecular weight drugs, peptides, antibodies, hormones, enzymes, nucleic acids, proteins, and combinations thereof.

As used herein, the term "nucleic acid" refers to DNA or RNA. In a particular embodiment, optionally in combination with any of the embodiments provided above or below, the nucleic acid is an DNA/RNA hybrid, a short interfering RNA (siRNA), a microRNA (miRNA), a single guide RNA (sgRNA), a donorDNA, a self-amplyfing/replicating RNA, a circularRNA (oRNA), a plasmid DNA (pDNA), a closed-linear DNA (cIDNA), a short hairpin RNA (shRNA), messenger RNA (mRNA), and antisense RNA (aRNA), a messenger RNA (mRNA), a CRISPR guide RNA, an antisense nucleic acid, a decoy nucleic acid, an aptamer, and a ribozyme to name a few, and encompasses both the nucleotide sequence and any structural embodiments thereof, such as double stranded, single stranded, helical, hairpin, etc, and may contain modified or unmodified bases. When distinct nucleic acids are provided, they may be all DNA molecules or all RNA molecules or may be mixtures of DNA and RNA molecules or molecules comprising an association of DNA and RNA strands.

The nucleic acid may be a poly- or oligonucleotide, such as oligo- or poly-double stranded RNA, oligo- or poly-double stranded DNA, oligo- or poly-single stranded RNA, oligo- or poly-single stranded DNA. Each of the nucleotides contained in the nucleic acid may be a naturally occurring nucleotide or a chemically-modified, non-naturally occurring nucleotide. The strand length of the nucleic acid is not particularly limited and the nucleic acid may have a short strand ranging from 10 to 200 bases, preferably from 20 to 180 bases, preferably from 25 to 100 bases, preferably from 30 to 50 bases; or the nucleic acid may have a relatively long strand of from 200 to 20000 bases, more preferably of from 250 to about 15000 bases.

In accordance with a particular embodiment, optionally in combination with any of the embodiments provided above or below, the nucleic acid is closed-linear DNA (clDNA), i.e. molecules wherein the double stranded region is flanked and protected by two single stranded loops thereby generating dumbbell-shaped molecules.

In a more particular embodiment, optionally in combination with any of the embodiments provided above or below, the cIDNA consists of a stem region comprising a double stranded DNA sequence of interest covalently closed at both ends by hairpin loops, the clDNA comprising at least two modified nucleotides.

As used herein, the term "closed linear DNA" or "clDNA" refers to a single stranded covalently closed DNA molecule that forms a "dumbbell" or "doggy-bone" shaped structure under conditions allowing nucleotide hybridization. Therefore, although the cIDNA is formed by a single stranded DNA molecule, the formation of the "dumbbell" structure by the hybridization of two complementary sequences within the same molecule generates a structure consisting on a double-stranded middle segment flanked by two single-stranded loops. The skilled in the art know how to generate clDNA from open or closed double stranded DNA using routine molecular biology techniques. For instance, those skilled in the art knows that a cIDNA can be generated by attaching hairpin DNA adaptors, for instance, by the action of a ligase, to both ends of an open double stranded DNA. "Hairpin DNA adaptor" refers to a single stranded DNA that forms a stem-loop structure by the hybridization of two complementary sequences, wherein the stem region formed is closed at one end by a single stranded loop and is open at the other end.

A "modified nucleotide" is any nucleotide (e.g., adenosine, guanosine, cytidine, uracil, and thymidine) that has been chemically modified -by modification of the base, the sugar or the phosphate group- or that incorporates a non-natural moiety in its structure. Thus, the modified nucleotide may be naturally or non-naturally occurring depending on the modification

As used herein, the term "peptide" refers to molecules that comprise two or more consecutive amino acids linked to one another via peptide bonds. The term peptide includes oligopeptides and polypeptides. The term "protein" refers to large peptides, in particular peptides having at least about 50 amino acids. For the purposes of the invention, the terms peptide and protein are used interchangeably.

Examples of proteins of interest include, without limitation cytokines, interleukins, tumor necrosis factor (TNF), interferons, integrins, chimeric antigen receptors (CARs), antibodies, hormones, growth factors, enzymes), collagen, fibrinogen, elastin, tubulin, thrombin, serum albumin, erythropoietin, granulocyte colony stimulating factor (G-CSF), colony stimulating factor (CSF), and the like.

In the context of the present disclosure the expression, "diagnostically active agent", also referred to as "labeling or imaging agent", refers to any substance that is used as a label, or that enhances specific structures in any imaging technique. An imaging agent, hence, includes optical imaging agent, magnetic resonance imaging agent, radioisotope, and contrast agent. Imaging or labelling agents are well known in the art. Particular examples or imaging or labelling agents are gases such as sterilized air, oxygen, argon, nitrogen, fluor, perfluorocarbons, carbon dioxide, nitrogen dioxide, sulfur hexafluoride, xenon and helium; commercially available agents used in positron emission tomography (PET), computer assisted tomography (CAT), single photon emission computerized tomography, x-ray, fluoroscopy, and magnetic resonance imaging (MRI). Examples of suitable materials for use as contrast agents in MRI include the gadolinium chelates currently available, such as diethylene triamine pentaacetic acid (DTPA) and gadopentotate dimeglumine, as well as iron, magnesium, manganese, copper and chromium. Examples of materials useful for CAT and x-rays include iodine based materials for intravenous administration, such as ionic monomers typified by diatrizoate and iothalamate, non-ionic monomers such as iopamidol, isohexol, and ioversol, non-ionic dimers, such as iotrol and iodixanol, and ionic dimers, for example, ioxagalte. Other useful materials include barium for oral use and non-soluble salts such as zinc acetate. In some molecules, an imaging agent is a dye. In some molecules, an imaging agent is a fluorescent moiety. In some molecules, a fluorescent moiety is selected from: a fluorescent protein, a fluorescent peptide, a fluorescent dye, a fluorescent material or a combination thereof. Examples of fluorescent dyes include, but are not limited to, xanthenes (e.g., rhodamines, rhodols and fluoresceins, and their derivatives); bimanes; coumarins and their derivatives (e.g., umbelliferone and aminomethyl coumarins); aromatic amines (e.g., dansyl; squarate dyes); benzofurans; fluorescent cyanines; indocarbocyanines; carbazoles; dicyanomethylene pyranes; polymethine; oxabenzanthrane; xanthene; pyrylium; carbostyl; perylene; acridone; quinacridone; rubrene; anthracene; coronene; phenanthrecene; pyrene; butadiene; stilbene; porphyrin; pthalocyanine; lanthanide metal chelate complexes; rare-earth metal chelate complexes; and derivatives of such dyes. Examples of fluorescein dyes include, but are not limited to, 5-carboxyfluorescein, fluorescein-5-isothiocyanate, fluorescein-6-isothiocyanate and 6-carboxyfluorescein. Examples of rhodamine dyes include, but are not limited to, tetramethylrhodamine-6-isothiocyanate, 5-carboxytetramethylrhodamine, 5-carboxy rhodol derivatives, tetramethyl and tetraethyl rhodamine, diphenyldimethyl and diphenyldiethyl rhodamine, dinaphthyl rhodamine, rhodamine 101 sulfonyl chloride (sold under the tradename of TEXAS RED(R)). Examples of cyanine dyes include, but are not limited to, Cy3, Cy3B, Cy3.5, Cy5, Cy5.5, Cy7, IRDYE680, Alexa Fluor 750, IRDye800CW, ICG. Examples of fluorescent peptides include GFP (Green Fluorescent Protein) or derivatives of GFP (e.g., EBFP, EBFP2, Azurite, mKalama1, ECFP, Cerulean, CyPet, YFP, Citrine, Venus, YPet). Fluorescent labels are detected by any suitable method. For example, a fluorescent label may be detected by exciting the fluorochrome with the appropriate wavelength of light and detecting the resulting fluorescence, e.g., by microscopy, visual inspection, via photographic film, by the use of electronic detectors such as charge coupled devices (CCDs), photomultipliers, etc. In some molecules, the imaging agent is labeled with a positron-emitting isotope (e.g., 18F) for positron emission tomography (PET), gamma-ray isotope (e.g., 99mTc) for single photon emission computed tomography (SPECT), or a paramagnetic molecule or nanoparticle (e.g.,Gd3+ chelate or coated magnetite nanoparticle) for magnetic resonance imaging (MRI). In some molecules, the imaging agent is labeled with: a gadolinium chelate, an iron oxide particle, a super paramagnetic iron oxide particle, an ultra small paramagnetic particle, a manganese chelate or gallium containing agent. Examples of gadolinium chelates include, but are not limited to diethylene triamine pentaacetic acid (DTPA), 1,4,7,10-tetraazacyclododecane-1,4,7,10-tetraacetic acid (DOTA), and 1,4,7-triazacyclononane-N,N',N"-triacetic acid (NOTA). In some molecules, the imaging agent is a near-infrared fluorophore for near-infra red (near-IR) imaging, a luciferase (firefly, bacterial, or coelenterate) or other luminescent molecule for bioluminescence imaging, or a perfluorocarbon-filled self-assembled particle for ultrasound. In some molecules, the imaging agent is a nuclear probe. In some molecules, the imaging agent is a SPECT or PET radionuclide probe. In some molecules, the radionuclide probe is selected from: a technetium chelate, a copper chelate, a radioactive fluorine, a radioactive iodine, a indiuim chelate. Examples of Tc chelates include, but are not limited to HYNIC, DTPA, and DOTA. In some molecules, the imaging agent contains a radioactive moiety, for example a radioactive isotope such as $^{211}$At, $^{131}$I, $^{125}$I, $^{90}$Y $^{186}$Re, $^{188}$Re, $^{153}$Sm, $^{212}$Bi, $^{32}$P, $^{64}$Cu radioactive isotopes of Lu, and others. The diagnostically active agents agent may be contained in the self-assembled particles formed from the conjugated.

Compositions

The present invention also relates to a composition comprising the self-assembled particle as defined herein, together with one or more appropriate excipients or carriers.

In one embodiment, optionally in combination with one or more features of the various embodiments described above or below, the composition is a pharmaceutical composition comprising a therapeutically effective amount of: (a self-assembled particle which comprises the lipo-polyamino acid conjugate as defined herein and one or more nucleic acids, together with one or more pharmaceutically acceptable excipients or carriers.

The expression "therapeutically effective amount" as used herein, refers to the amount of a polymer that, when administered, is sufficient to prevent development of, or alleviate to some extent, one or more of the symptoms of the disease which is addressed. The specific dose of the polymer of the invention to obtain a therapeutic benefit may vary depending on the particular circumstances of the individual patient including, among others, the size, weight, age and sex of the patient, the nature and stage of the disease, the aggressiveness of the disease, and the route of administration.

For the purpose of the present invention, the term "pharmaceutically acceptable excipients or carriers" refers to components which are appropriate for use in pharmaceutical technology for the preparation of compositions for medical use. Each component should be "acceptable" in the sense of being compatible with the other ingredients of the composition. When used in contact with the tissue or organ of humans and animals should not have excessive toxicity, irritation, allergic response, immunogenicity, or other problems or complications, commensurate with a reasonable benefit/risk ratio.

The lipo-polyamino acid conjugates described in the present disclosure, and the self-assembled particles and pharmaceutical compositions containing them may be used jointly with other, additional drugs, to provide combined therapy. Said additional drugs may be a part of the same pharmaceutical composition or, alternatively, may be provided in the form of a separate composition for simultaneous or non-simultaneous administration.

In another embodiment, optionally in combination with one or more features of the various embodiments described above or below, the composition is a diagnostic composition comprising a diagnostically effective amount of: a self-assembled particle which as defined herein, and one or more diagnostically active agents, together with one or more diagnostically acceptable excipients or carriers.

The term "diagnostic composition" refers to a composition suitable for use in diagnostic, particularly in imaging diagnostic technology. The term "diagnostically effective amount" as used herein, refers to the effective amount of a detection polymer that, when administered, is sufficient for the diagnosis of a disease or disorder; particularly as imaging diagnostic use as contrast imaging agent. The dose of the detection polymer administered will of course be determined by the particular circumstances surrounding the case, including the polymer administered, the route of administration, the particular condition being diagnosticated, and the similar considerations. The diagnostic composition of the present invention comprises one or more diagnostically acceptable excipients or carriers. The term "diagnostically acceptable" refers to that excipients or carriers suitable for use in the diagnosing technology for preparing compositions with diagnostic use; particularly by imaging diagnostic use. The detection of these diagnostic agents in the body of the patient can be carried out by the well-known techniques used such as in imaging diagnostic with magnetic resonance imaging (MRI) and X-ray.

In another embodiment, optionally in combination with one or more features of the various embodiments described above or below, the composition is a cosmetic composition comprising a cosmetically effective amount of: a self-assembled particle as defined herein and one or more cosmetically active agents, together with one or more cosmetically acceptable excipients or carriers.

The term "cosmetically effective amount" as used herein, refers to the effective amount of a cosmetically active agent that, when administered, is intended to improve its appearance or to beautify, preserve, condition, cleanse, color or protect the skin, nails or hair without a medical application. The term "cosmetically acceptable" or "dermatologically acceptable" excipients or carriers is used interchangeably in this document and refer to components which are appropriate for use in human skin contact without toxicity, incompatibility, instability, inappropriate allergic response, among others.

The compositions of the invention may be in solid or liquid form. Non-limiting examples of solid forms include frozen forms, lyophilized forms and spray-dried forms. Depending on the desired purpose, the skilled person will know the most appropriate formulation and excipients to be used. Examples of excipients or carriers include, without limitation, diluents, binders, glidants, disintegrants, lubricants colorants, mixtures thereof, and other components known in the state of the art. Any administration route may be used such as e.g. oral, topical, rectal or parenteral route (including subcutaneous, intraperitoneal, intradermal, intramuscular, intravenous route, etc.).

## Uses

The lipo-polyamino acid conjugates of the invention, self-assembled particles, and compositions thereof may be used in therapeutic applications. In particular, they may be used as non-viral vectors of general use for biomedical applications, such as vaccines or gene therapy, being effective for transfection of hosts eukaryotic cells in culture, *in vivo* or *ex vivo,* monocellular parasites and bacteria, including gene editing using the CRISP/Cas9 methodology. Further, they may be used in protein-based therapy; particularly protein-based vaccine against viral infections or as a therapeutic protein-based vaccine against cancers or infectious diseases.

Thus, the invention relates to a therapeutic product which is or which comprises: a) a self-assembled particle as defined herein; or alternatively, b) a composition containing the self-assembled particle a), for use in medicine.

This aspect may also be formulated as a method for treating or preventing a disease or disorder in a subject, more particularly a mammal, and even more particularly a human, comprising administering the therapeutic product as defined herein, and one or more pharmaceutically acceptable excipients or carriers.

Non-limiting examples of diseases that may be treated and/or prevented by the derivatives of the present invention include neurodegenerative disorders, neurological diseases, cancer, infectious diseases, disorders related to aging, neuro-inflammation, demyelinating disorders, multiple sclerosis, ischemic disorders, ischemia-reperfusion damage, amyloidotic disease, cardiomyopathy, spinal cord injury, immune disorders, inflammatory disorders, rare diseases, wound healing, skin related diseases and lysosomal storage diseases.

Non-limiting examples of neurodegenerative diseases include Alzheimer's disease, Parkinson's disease, amyotrophic lateral sclerosis, cerebral ischaemia, post-encephalitic Parkinsonisms, dystonias, Tourette syndrome, periodic limb movement pathologies, restless legs syndrome, attention deficit hyperactivity disorders, Huntington's disease, progressive supranuclear palsy, Pick's disease, fronto-temporal dementia and neuromuscular diseases.

The term "disorder" as used herein is intended to be generally synonymous, and is used interchangeably with, the terms "disease," "syndrome," and "condition" (as in medical condition), in that all reflect an abnormal condition of the human or animal body or of one of its parts that impairs normal functioning, is typically manifested by distinguishing signs and symptoms.

In the context of the present invention, the terms "treat", "treating" and "treatment", as used herein, refers to ameliorating symptoms associated with a disease or disorder, including preventing or delaying the onset of the disease or disorder symptoms, and/or lessening the severity or frequency of symptoms of the disease or disorder.

According to one embodiment, optionally in combination with any of the embodiments provided above or below, the invention relates to the therapeutic product as defined herein for use (i) as transfection reagent for transfecting at least one active agent into a cell; (ii) for use in the *in vivo* or *ex vivo* production of biologics encoding a recombinant protein, a peptide or an antibody, or in the production of recombinant virus; (iii) for use as a therapeutic or prophylactic vaccine against viral infections or as a therapeutic vaccine against cancers or infectious diseases; or (iv) for use in genome engineering, for cell reprogramming, for differentiating cells or for gene-editing.

In a particular embodiment, optionally in combination with any of the embodiments provided above or below, the present invention relates to the therapeutic product as defined herein as transfection reagent for delivering one or more nucleic acids (regardless of size and structure, circular and linear nucleic acids) to target cells, in *in vivo, in vitro* or *ex vivo.* More particularly, the active agent is selected from the group consisting of low molecular weight drugs, peptides, proteins, antibodies, nucleic acids, aptamers, and combinations thereof.

The present invention also relates to a method for *in vitro, ex vivo* and *in vivo* transferring active agents comprising the therapeutic product as defined herein.

In a particular embodiment, optionally in combination with any of the embodiments provided above or below, the therapeutic product is a self-assembled particle which comprises the lipo-polyamino acid conjugate as defined herein and one or more nucleic acids, or alternatively, a composition containing the self-assembled particle which comprises the lipo-polyamino acid conjugate as defined herein and one or more nucleic acids.

The transfection reagents of the invention are also useful for co-transfection of two or more active agents simultaneously, e.g. two or more nucleic acids, simultaneously. Transfection compositions (such as kits), as well as methods of using the transfection reagents to deliver nucleic acid to target cells are also within the scope of the present invention.

The present invention also provides therapeutic products as defined herein for inducing a regulating effect on the expression of one or more target proteins responsible or involved in genetic hereditary diseases or complex genetic diseases, immune diseases, cancers, viral infections in various tissues/organs or tumors.

The present invention also relates to the *in vitro* or *ex vivo* use of therapeutic products as defined herein in the production of biologics, in particular biologics encoding a recombinant protein, a peptide or an antibody; or in the production of recombinant virus, such as adeno-associated virus (AAV), lentivirus (LV), adenovirus, oncolytic virus, or baculovirus, or viral or virus-like particles, in particular said products comprising the lipo-polyamino acid conjugates of the invention and at least one nucleic acid molecule for transfection. As used herein, the term "biologics" refers to proteins or nucleic acids or combinations thereof, living entities such as cells or viruses, cell compartments, organoids, and tissues.

The present invention also relates to an *in vitro* or *ex vivo* use of the therapeutic products as defined herein, in particular said products comprising the lipo-polyamino acid conjugates of the invention and at least one nucleic acid molecule for transfection, for genome engineering, for cell reprogramming, for differentiating cells or for gene-editing.

The compositions for transfecting cells comprise the therapeutic products as defined herein, in particular said products comprising the lipo-polyamino acid conjugates of the invention and at least one nucleic acid molecule for transfection, and an acceptable excipient, buffering agent, cell culture medium, or transfection medium.

The present invention is also directed to the therapeutic products as defined herein for use as a therapeutic or prophylactic vaccine against viral infections, or a therapeutic vaccine against cancers. Generally, in this aspect, the vaccine is delivered through direct administration such as systemic, intramuscular, intradermal, intraperitoneal, intratumoral, oral, topical, or sub-cutaneous administration, and, in said vaccine, the composition is in association with a pharmaceutically acceptable vehicle. In other words, the vaccine can be injected directly into the body, in particular in a human individual, for inducing a cellular and/or a humoral response.

The cell targeting is achieved through different mechanisms and depends on the nature and properties of the transfection reagent, method or protocol composition or formulation and the route of administration.

The lipo-polyamino acid conjugates of the invention, self-assembled particles, and compositions thereof may be used also in cosmetic and diagnostic applications.

Accordingly, another aspect of the invention relates to a diagnostic product which is or which comprises: a') a self-assembled particle as defined herein and one or more diagnostically active agents; or alternatively, b') a composition containing the self-assembled particle a'), for use in diagnostics.

This aspect of the invention may also be formulated as the use of a') a self-assembled particle as defined herein and one or more diagnostically active agents; or alternatively, b') a composition containing the self-assembled particle a'), in diagnostics. Further, it may also be formulated as a method for the diagnostic of a disease or condition, comprising administering a') a self-assembled particle as defined herein and one or more diagnostically active agents; or alternatively, b') a composition containing the self-assembled particle a'), in a subject in need thereof, more particularly a mammal, and even more particularly a human.

In a particular embodiment, the invention relates to microbubbles which comprise the lipo-polyamino acid conjugate as defined herein and one or more diagnostically active agents, in particular contrast agents, for use in diagnostics.

The detection of these imaging agents can be carried out by well-known techniques such as imaging diagnostic techniques. Examples of imaging diagnostic techniques suitable for the present disclosure include, but not limited to, are magnetic resonance imaging (MRI), X-ray, positron emission tomography (PET), single-photon emission computed tomography (SPECT), fluorescence microscopy, and *in vivo* fluorescence.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

## Examples

*Analytical methods*

*NMR spectroscopy*

NMR spectra were recorded at 27 °C (300 K) on a 300 UltrashieldTM from Bruker (Billerica MA, USA). Data were processed with the software Topspin (Bruker GmbH, Karlsruhe, Germany). Samples were prepared at a concentration of 20 - 10 mg/mL approx. in the required solvent.

*Size Exclusion Chromatography (SEC)*

For SEC measurements in dimethylformamide (DMF) containing 0.1 % (w/w) of lithium bromide as an additive, a GPC max (Malvern Instruments) autosampler was used with a flow rate of 0.7 mL/min at 60 °C with TSKgel Alpha-4000 column from Tosoh Bioscience. Viscotek TDA-302 was used as an integrated detection system. The system was calibrated with polymethyl methacrylate (PMMA) (Mw = 65 kDa; PDI = 1.05) from PSS. For this Mw determination an integrated triple detection system was used [Refractive Index, Light Scattering (two angles: 7 and 90 °) and Ultraviolet-Visible detector].

For SEC measurements in aqueous containing 0.1M of sodium nitrate and 0.005% of sodium azide as an additive, a GPC max (Malvern Instruments) pump and autosampler was used with a flow rate of 0.7 mL/min at 25 °C with TSKGel PWXL G5000 column from Tosoh Bioscience. Viscotek TDA-305 was used as an integrated detection system. The system was calibrated with polyethylene oxide (PEO) (Mw = 24 kDa; PDI = 1.01) from Malvern Panalytical. For this Mw determination an integrated triple detection system was used [Refractive Index, Light Scattering (two angles: 7 and 90 °) and Ultraviolet-Visible detector].

*Size Distribution*

Dynamic Light Scattering (DLS) measurements were performed using a Malvern Zetasizer NanoZS instrument,

equipped with a 532 nm laser at a fixed scattering angle of 173 °. Polymer solutions were prepared under different conditions (MilliQ water or PBS at different concentrations and temperatures), solutions were sonicated for 10 min and allowed to age for the required time, filtered through a 1.20 μm cellulose membrane filter and measured. Size distribution was measured (diameter, nm) for each polymer per triplicate with n > 3 measurements, automatic optimization of beam focusing and attenuation was applied for each sample.

Example 1. Synthesis of lipo-shielding $C_{14}$-PGA(DIOL)

1.1 General procedure for the polymerization of $C_{14}$-PGlu(OBzl)

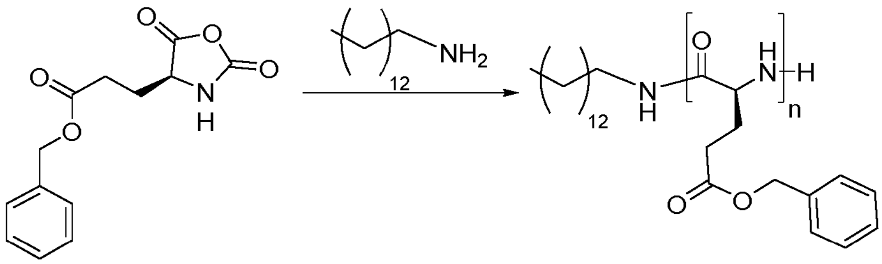

Glutamic acid Benzyl ester NCA (10 g, 40.12 mmol) was added to a Schlenk tube fitted with a stirring bar and a stopper. After 3 cycles of vacuum/$N_2$, the mixture was dissolved in anhydrous DMF (150 mg reagent per mL DMF). Then, the initiator (Tetradecylamine 96%, TCI EUROPE N.V.: DP23: 0.392 g, 1.74 mmol, DP8: 1.13 g, 5.02 mmol) was diluted in DMF (5 mL) and added to the reaction mixture, which was stirred at 10 °C for 16 hours. Once NCA consumption was confirmed by IR, the reaction mixture was poured into diethyl ether to precipitate the product. The precipitate was isolated by centrifugation (3750 rpm, 4 min) and dried under vacuum. Homopolymer ($C_{14}$-PGluOBzl) was isolated as a white solid. Yield: 70-90%. 1H NMR (300 MHz, DMSO) δ 8.38 - 7.82 (m, NH amide), 7.48 - 7.04 (m, CH aryl), 5.42 - 4.81 (m, $CH_2$ benzylic), 4.01 (m, CH), 2.57 - 1.79 (m, $CH_2$), 1.20 (s, $CH_2$), 0.82 (t, J = 6.35 Hz, $CH_3$).

Following the above general procedure, the polymers shown in Table 1 were obtained:

**Table 1.** Polymers based on $C_{14}$-PGlu(OBzl) (Lipo-$PGA_x$)

| Code | Initiator | DP (n)for Glu(OBzl)[a] | Mw by GPC (KDa)[b] | PDI |
|---|---|---|---|---|
| Lipo-PGA1 | Tetradecylamine | 23 | 5.9 | 1.03 |
| Lipo-PGA2 | Tetradecylamine | 8 | 2.7 | 1.10 |
| [a]*Determined by NMR.* [b]*Determined by GPC, wherein the cited DP numbers are subject to a reasonable uncertainty within the range ±20%.* | | | | |

1.2 General procedure for capping the N-terminal:

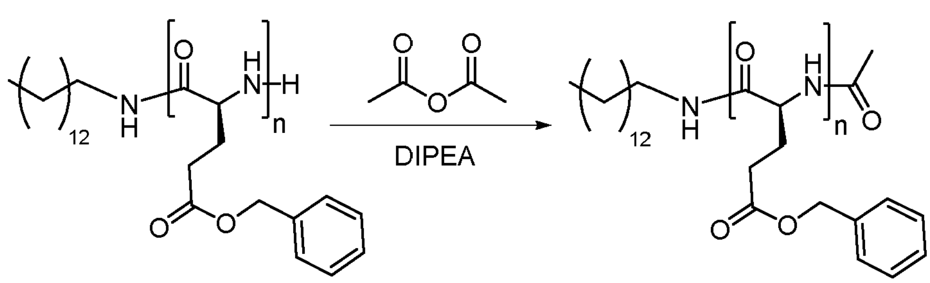


$C_{14}$-PGlu(OBzl) (PGADIOL1: 2.0 g, 0.38 mmol, PGADIOL2: 2.0 g, 1.02 mmol) as obtained in Example 1.1 was dissolved in DMF (200 mg per mL DMF) and acetic anhydride (10 eq) and DIPEA (3 eq) were added to the reaction mixture. Then, the mixture was stirred at RT for 1 hour. The reaction mixture was poured into diethyl ether to precipitate the product.

The precipitate ($C_{14}$-PGlu(OBzl)-$COCH_3$) was isolated by centrifugation (3750 rpm, 4 min) and dried under vacuum.

Yield: 60-70%. $^1H$ NMR (300 MHz, TFA) δ 8.05 - 7.73 (brs, CH aryl), 5.69 (m, $CH_2$ benzylic), 5.25 (m, CH), 3.88 (s, $CH_3$), 3.26 - 2.36 (m, $CH_2$), 1.83 (brs, $CH_2$), 1.40 (t, J = 6.6 Hz, $CH_3$).

Following the above general procedure, the polymers shown in Table 2 were obtained:

**Table 2.** Polymers based on $C_{14}$-PGlu(OBzl)-$COCH_3$ (Lipo-$PGA_x$)

| Code | Precursor | DP (n)for Glu(OBzl)[a] | Mw by GPC (KDa)[b] | PDI |
|---|---|---|---|---|
| Lipo-PGA3 | Lipo-PGA1 | 29 | 6.9 | 1.02 |
| Lipo-PGA4 | Lipo-PGA2 | 9 | 3.3 | 1.08 |
| [a]*Determined by NMR.* [b]*Determined by GPC, wherein the cited DP numbers are subject to a reasonable uncertainty within the range ±20%.* | | | | |

1.3 General procedure for deprotection step:

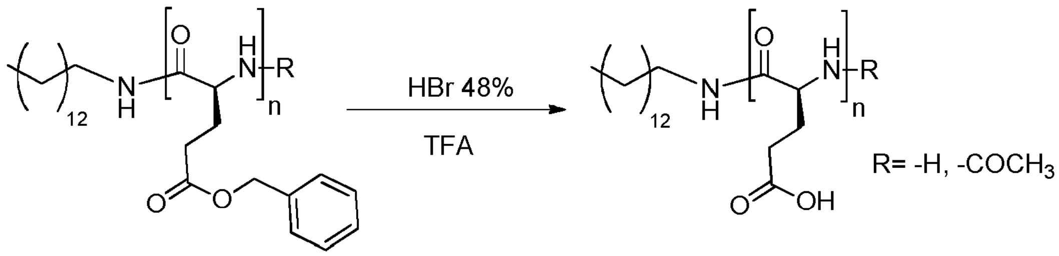


The lipo-polymer $C_{14}$-PGlu(OBzl)-$COCH_3$ (Lipo-PGA1-4: 6.4 mmol) as obtained in Example 1.2 was dissolved in trifluoroacetic acid (100 mg/mL) and HBr 48% (2 eq per Glutamic acid unit) was added. The mixture was stirred at RT for 16 hours. The reaction mixture was poured into diethyl ether to precipitate the product. The precipitate was washed with a mixture of Acetonitrile:$H_2O$ (8:2) until pH 4-5. Then, the product is washed again with diethyl ether, isolated by centrifugation (3750 rpm, 4 min) and dried under vacuum. The polymer ($C_{14}$-PGA) was isolated as a white solid.

Yield: 95%. $^1H$ NMR (300 MHz, $D_2O$) δ 4.35 (m, CH), 3.22 (m, $CH_3$), 2.69 - 1.76 (m, $CH_2$), 1.53 (brs, $CH_2$), 1.30 (s, $CH_2$), 0.89 (t, *J* = 6.4 Hz, $CH_3$).

Following the above general procedure, the polymers shown in Table 3 were obtained:

**Table 3.** Polymers based on $C_{14}$-PGA (Lipo-$PGA_x$)

| Code | Precursor | R | DP (n)for Glu(ONa)[a] |
|---|---|---|---|
| Lipo-PGA5 | Lipo-PGA3 | -$COCH_3$ | 22 |
| Lipo-PGA6 | Lipo-PGA4 | -$COCH_3$ | 9 |
| Lipo-PGA7 | Lipo-PGA1 | -H | 11 |
| Lipo-PGA8 | Lipo-PGA2 | -H | 6 |
| [a] *Determined by NMR, wherein the cited DP numbers are subject to a reasonable uncertainty within the range ±20%.* | | | |

1.4 General procedure for peptide coupling with CDI for the synthesis of $C_{14}$-PGA(DIOL)

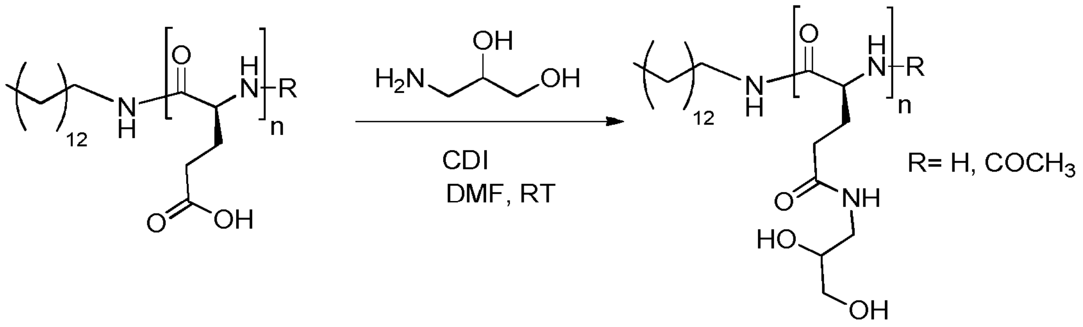


$C_{14}$-PGA (PGADIOL 0.4 g, 3.1 mmol) as obtained in Example 1.3 was dissolved in DMF (50 mg/mL). Then, CDI (1.6 eq per Glutamic acid unit) was added to the reaction mixture and stirred for 30 minutes at room temperature. After this time, the 3-aminopropane-1,2-diol (1.3 eq per Glutamic acid unit) dissolved in 2 mL of DMF was added. The mixture was stirred at room temperature for 16 hours. The reaction mixture was poured into acetone to precipitate the product. Precipitate was isolated by centrifugation (3750 rpm, 4 min) and dried under vacuum. The final product was isolated as a white solid.

Yield: 50-60%. $^1$H NMR (300 MHz, $D_2O$) $\delta$ 4.38 (brs, CH), 3.83 (brs, CH), 3.59 (ddd, $J$ = 18.1, 11.8, 5.2 Hz, $CH_2$), 3.32 (ddd, $J$ = 21.1, 14.0, 5.8 Hz, $CH_2$), 2.66 - 1.82 (m, $CH_2$),1.53 (brs. $CH_2$), 1.29 (s, $CH_2$), 0.88 (t, $J$ = 6.5 Hz, $CH_3$).

Following the above general procedure, the polymers shown in Table 4 were obtained:

**Table 4.** Polymers based on capped $C_{14}$-PGA(Diol) (Lipo-PGA$_x$)

| Code | Precursor | DP (n) for Glu(Diol)[a] | Mw by GPC (KDa)[b] | PDI |
|---|---|---|---|---|
| Lipo-PAA1 | Lipo-PGA5 | 20 | 4.0 | 1.19 |
| Lipo-PAA2 | Lipo-PGA6 | 8 | 1.8 | 1.13 |
| Lipo-PAA3 | Lipo-PGA7 | 11 | 4.1 | 1.10 |
| Lipo-PAA4 | Lipo-PGA8 | 6 | 2.2 | 1.08 |

*[a]Determined by NMR. [b]Determined by GPC, wherein the cited DP numbers are subject to a reasonable uncertainty within the range ±20%.*

Example 2. Synthesis of lipo-shielding $C_{14}$-PLys(Thioqlycerol)$_x$

2.1 General procedure for the polymerization of $C_{14}$-PLys(OBzl)

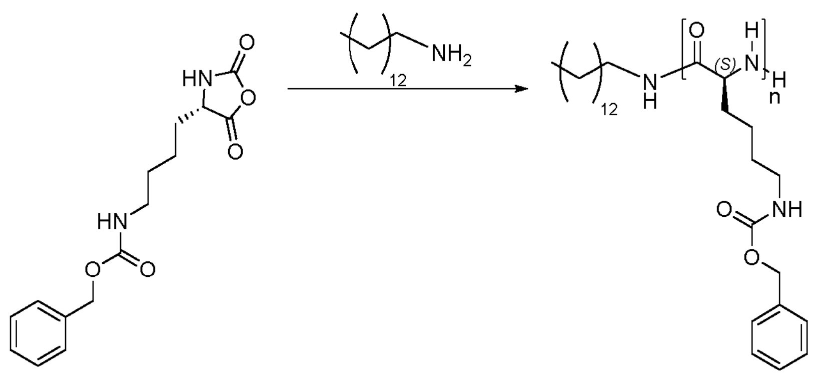

L-Lysine Benzyl ester NCA (10 g, 32.64 mmol) was added to a Schlenk tube fitted with a stirring bar and a stopper. After 3 cycles of vacuum/$N_2$, the mixture was dissolved in anhydrous DMF (150mg reagents per mL DMF). Then, the initiator (Tetradecylamine 96%, TCI EUROPE N.V.: DP23: 0.319 g, 1.42 mmol, DP8: 0.917 g, 1.08 mmol) was diluted in DMF (5 mL) and added to the reaction mixture, which was stirred at 10 °C for 16 hours. Once NCA consumption was confirmed by IR, the reaction mixture was poured into diethyl ether to precipitate the product. The precipitate was isolated by centrifugation (3750 rpm, 4 min) and dried under vacuum. Homopolymer ($C_{14}$-PLys(OBzl)) was isolated as a white

solid.

Yield: 70-90%. $^1$H NMR (300 MHz, DMSO) δ 8.31 - 7.69 (m, NH amide), 7.41 - 6.98 (m, CH aryl), 4.97 (m, $CH_2$ benzylic), 2.92 (m, $CH_2$), 2.07 - 1.09 (m, $CH_2$), 0.83 (t, J= 5.5 Hz, $CH_3$).

Following the above general procedure, the polymers shown in Table 5 were obtained:

**Table 5.** Polymers based on $C_{14}$-PLys(OBzl) (Lipo-$PLys_x$)

| Code | Initiator | DP (n) for Lys(OBzl)[a] | Mw by GPC (KDa)[b] | PDI |
|---|---|---|---|---|
| Lipo-PLys1 | Tetradecylamine | 27 | 7.1 | 1.23 |
| Lipo-PLys2 | Tetradecylamine | 9 | 3.4 | 1.59 |
| *[a]Determined by NMR. [b]Determined by GPC, wherein the cited DP numbers are subject to a reasonable uncertainty within the range ±20%.* | | | | |

2.2 General procedure for capping the N-terminal:

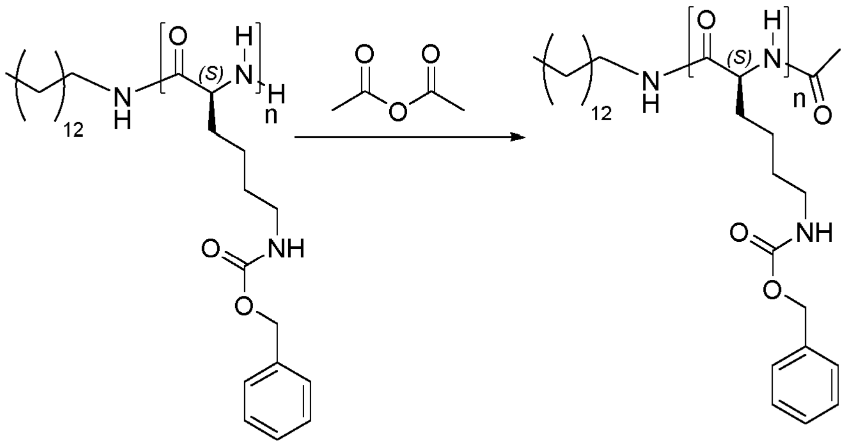

$C_{14}$-PLys(OBzl) (PLysTG1: 1.5g, 0.24 mmol, PLysTG2: 1.5g, 0.65 mmol) as obtained in Example 2.1 was capped following the procedure described above in example 1.2.

Yield: 60-70%. $^1$H NMR (300 MHz, TFA) δ 8.19 - 7.75 (m, CH aryl), 5.70 (brs, CH), 5.11 (brs, CH benzylic), 3.81 (brs, $CH_2$), 2.74 (s, $CH_3$), 1.92 - 2.63 (m, $CH_2$), 1.80 (brs, $CH_2$), 1.37 (t, $J$ = 6.6 Hz, $CH_3$).

Following this procedure, the polymers shown in Table 6 were obtained:

**Table 6.** Polymers based on $C_{14}$-PLys(OBzl)-$COCH_3$ ($PLys_x$)

| Code | Precursor | DP (n) for Lys(OBzl)[a] | Mw by GPC (KDa)[b] | PDI |
|---|---|---|---|---|
| Lipo-PLys3 | Lipo-PLys1 | 22 | 7.2 | 1.21 |
| Lipo-PLys4 | Lipo-PLys2 | 9 | - | - |
| *[a]Determined by NMR. [b]Determined by GPC, wherein the cited DP numbers are subject to a reasonable uncertainty within the range ±20%.* | | | | |

2.3 General procedure for deprotection step:

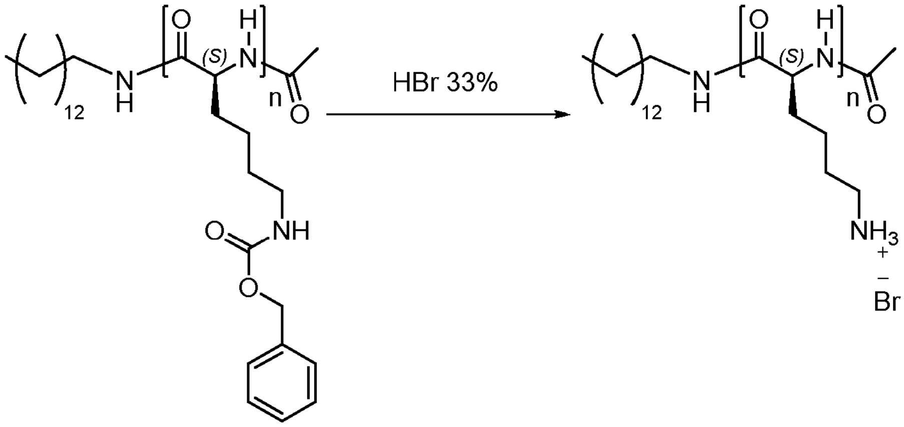


The lipo-polymer $C_{14}$-PLys(OBzl)-$COCH_3$ (PLys: 1.25 g, 4.7 mmol); as obtained in Example 2.2 was dissolved in chloroform (50 mg/mL) and HBr 33% (4 eq per Lysine unit) was added. The mixture was stirred at RT for 16 hours. An orange solid appears. The solid is recovered by filtration and washed with diethyl ether. The precipitate was washed with a mixture of Acetonitrile:$H_2O$ (8:2) until pH 4-5. Then, the product is washed again with diethyl ether, isolated by centrifugation (3750 rpm, 4 min) and dried under vacuum. The polymer was isolated as a yellowish solid. Final purification by dialysis was performed (Mw cutt off 2000 Da) to obtain a white solid ($C_{14}$-PLys).

Yield: 70%. $^1$H NMR (300 MHz, $D_2O$) $\delta$ 4.29 (m, CH), 3.04 (m, $CH_2$), 2.07 (s, $CH_3$), 1.93 - 1.37 (m, $CH_2$), 1.31 (brs, $CH_2$), 0.89 (t, $J$ = 6.7 Hz, $CH_3$).

Following this procedure, the polymers shown in Table 7 were obtained:

**Table 7.** Polymers based on $C_{14}$-PLys (Lipo-$PLys_x$)

| Code | Precursor | DP (n)for Lys[a] |
|---|---|---|
| Lipo-PLys5 | Lipo-PLys3 | 28 |
| Lipo-PLys6 | Lipo-PLys4 | 8 |
| *[a]Determined by NMR, wherein the cited DP numbers are subject to a reasonable uncertainty within the range ±20%.* | | |

2.4 General procedure for peptide coupling with chloroacetic anhydride for the synthesis of $C_{14}$-PLys(ClAc)

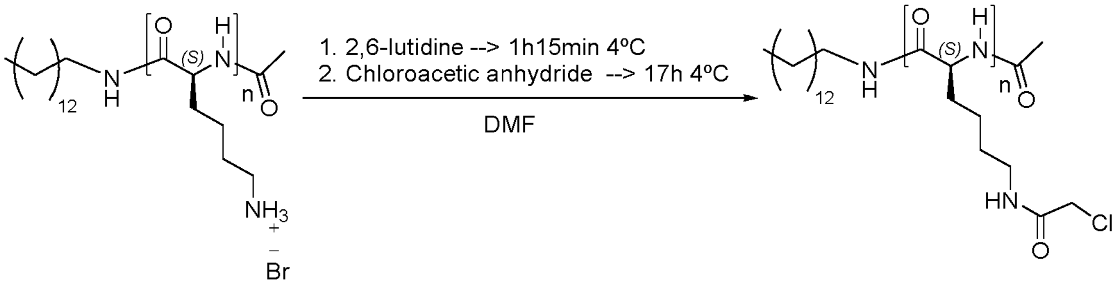


$C_{14}$-PLys (PLys 0.5 g, 0.1 mmol) as obtained in Example 2.3 was dissolved in cold DMF (50 mg/mL). Then, 2,6-lutidine (0.25 mL per mL DMF) was added to the reaction mixture and stirred for 75 minutes at 4 °C. After this time, cold Chloroacetic anhydride (4 eq per Lysine unit) dissolved in DMF (514 mg/mL) was added dropwise. The mixture was stirred at 4 °C for 16 hours. The reaction mixture was poured into hot ethanol. The mixture was left at 4 °C during 45 minutes to precipitate the product. Precipitate was isolated by filtration, washed with ethanol and dried under vacuum. The final product ($C_{14}$-PLys(ClAc)) was isolated as a white solid.

Yield: 80-90%. $^1$H NMR (300 MHz, TFA) $\delta$ 5.19 (brs, CH), 4.79 (s, $CH_2$), 3.99 (brs, $CH_2$), 2.90 (s, $CH_3$), 2.60 - 1.98 (m, $CH_2$), 1.87 (brs, $CH_2$), 1.41 (t, $J$ = 6.6 Hz, $CH_3$).

Following this procedure, the polymers shown in Table 8 were obtained:

**Table 8.** Polymers based on $C_{14}$-PLys(ClAc) (Lipo-$PLys_x$)

| Code | Precursor | DP (n) for Lys(ClAc)[a] |
|---|---|---|
| Lipo-PLys7 | Lipo-PLys5 | 30 |
| Lipo-PLys8 | Lipo-PLys6 | 9 |
| [a]*Determined by NMR, wherein the cited DP numbers are subject to a reasonable uncertainty within the range ±20%.* | | |

2.5 General procedure for thioglycerol coupling for the synthesis of $C_{14}$-PLys(Thioglycerol)

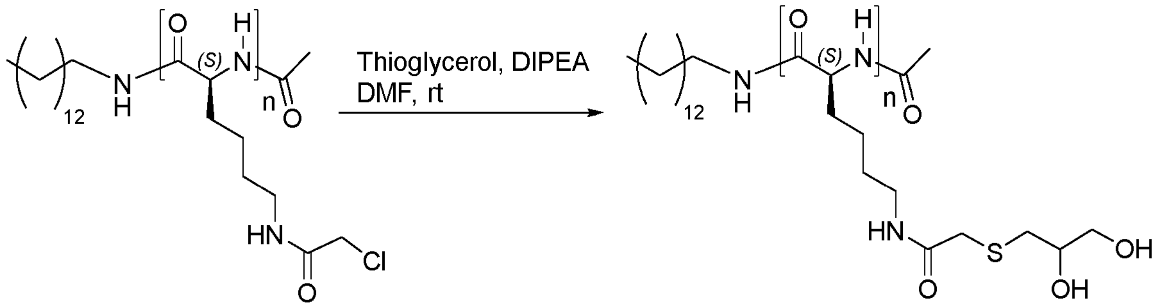


$C_{14}$-PLys(ClAc) (PLysTG: 502 mg, 0.1 mmol) as obtained in Example 2.4 was dissolved in DMF (22 mg/mL). Then, Thioglycerol (8 eq per Lysine unit) and DIPEA (8 eq per Lysine unit) were added to the reaction mixture. The reaction mixture was stirred at room temperature for 16h. After this time, the reaction mixture was poured into diethyl ether Precipitate was isolated by filtration, washed with diethyl ether and dried under vacuum. The product was dissolved in $H_2O$, purified by sephadex column (PD-10 Desalting Columns) and lyophilized. The final product was isolated as a white solid.

Yield: 60-70%. $^1H$ NMR (300 MHz, $D_2O$) δ 4.10 (brs, CH), 3.88 (brs, CH), 3.64 (ddd, *J* = 22.9, 12.1, 4.6 Hz, $CH_2$), 3.34 (brs, $CH_2$), 3.25 (brs, $CH_2$), 2.75 (ddd, *J* = 21.4, 13.8, 6.1 Hz, $CH_2$), 2.20 - 1.14 (m, $CH_2$), 0.88 (m, $CH_3$).

Following this procedure, the polymers shown in Table 9 were obtained:

Table 9. Polymers based on capped $C_{14}$-PLys(Thioglycerol) (Lipo-$PLys_x$)

| Code | Precursor | DP (n) for Lys(TG)[a] |
|---|---|---|
| Lipo-PAA5 | Lipo-PLys7 | 33 |
| Lipo-PAA6 | Lipo-PLys8 | 10 |
| [a]*Determined by NMR.* [b]*Determined by GPC, wherein the cited DP numbers are subject to a reasonable uncertainty within the range ±20%.* | | |

Example 3. Synthesis of lipo-shielding $C_{14}$-PLys(Betaine)

3.1 General procedure for the polymerization of $C_{14}$-PLys(TFA)

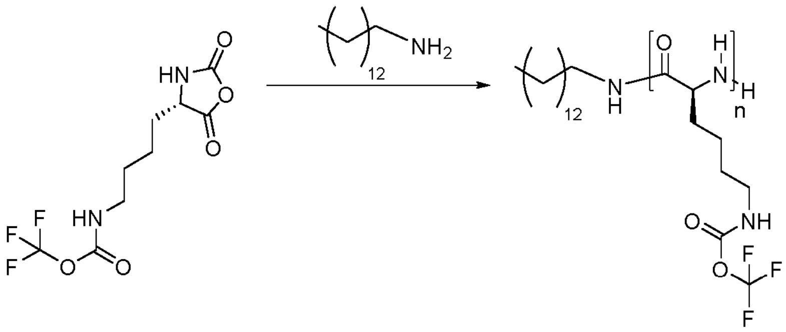


L-Lysine TFA NCA (10 g, 37.29 mmol, DP 5) was added to a Schlenk tube fitted with a stirring bar and a stopper. After 3 cycles of vacuum/$N_2$, the mixture was dissolved in anhydrous DMF (150mg reagents per mL DMF). Then, the initiator (1.67g, 7.45 mmol, Tetradecyl amine 96%, TCI EUROPE N.V.) was diluted in DMF (5 mL) and added to the reaction mixture, which was stirred at 10 °C for 16 hours. Once NCA consumption was confirmed by IR, the reaction mixture was poured into diethyl ether to precipitate the product. The precipitate was isolated by centrifugation (3750 rpm, 4 min) and dried under vacuum. Homopolymer ($C_{14}$-PLys(TFA)) was isolated as a white solid.

Yield: 70-90%. 1H NMR (300 MHz, TFA) δ 5.35 - 4.88 (m, CH), 4.4 (m, $CH_2$), 2.77 - 2.00 (m, $CH_2$), 1.91 (brs, $CH_2$), 1.45 (t, *J* = 6.7 Hz, $CH_3$).

Following the above general procedure, the polymer shown in Table 10 was obtained:

**Table 10.** Polymers based on $C_{14}$-PLys(TFA) (Lipo-PBet$_x$)

| Code | Initiator | DP (n) for Lys(TFA)[a] | Mw by GPC (KDa)[b] | PDI |
|---|---|---|---|---|
| Lipo-PBet1 | Tetradecylamine | 5 | 2.06 | 1.06 |
| Lipo-PBet2 | Tetradecylamine | 23 | 7.0 | 1.32 |

[a]*Determined by NMR.* [b]*Determined by GPC, wherein the cited DP numbers are subject to a reasonable uncertainty within the range ±20%.*

<u>3.2 General procedure for deprotection step:</u>

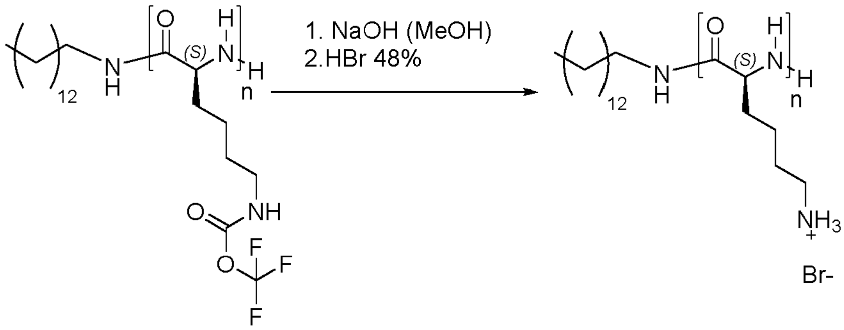


The lipo-polymers $C_{14}$-PLys(TFA)as obtained in Example 3.1 was dissolved in cold methanol (300 mg/mL) and cold NaOH $_{aq}$ (1.5 eq per Lysine unit) was added. The mixture was stirred at RT for 16 hours. The reaction mixture was quenched with HBr 48% until pH 2. The precipitate was washed with a mixture of Acetone, isolated by centrifugation (3750 rpm, 4 min) and dried under vacuum. The product was dissolved in $H_2O$, the pH adjusted to 4 with NaOH 1M and lyophilized. The polymer ($C_{14}$-PLys) was isolated as a white solid.

Yield: 70-80%. $^1$H NMR (300 MHz, TFA) δ 5.31 (m, CH), 3.94 (m, $CH_2$), 2.92 - 2.12 (m, $CH_2$), 1.95 (brs, $CH_2$), 1.49 (t, *J* = 6.7 Hz, $CH_3$).

Following the above general procedure, the polymer shown in Table 11 was obtained:

**Table 11.** Polymers based on $C_{14}$-PLys (Lipo-PBet$_x$)

| Code | Precursor | DP (n) for Lys(TFA)[a] |
|---|---|---|
| Lipo-PBet3 | Lipo-PBet1 | 5 |
| Lipo-PBet4 | Lipo-PBet2 | 36 |
| *[a]Determined by NMR, wherein the cited DP numbers are subject to a reasonable uncertainty within the range ±20%.* | | |

3.3 General procedure for di-methylation of the amine group of $C_{14}$-PLys$_n$.

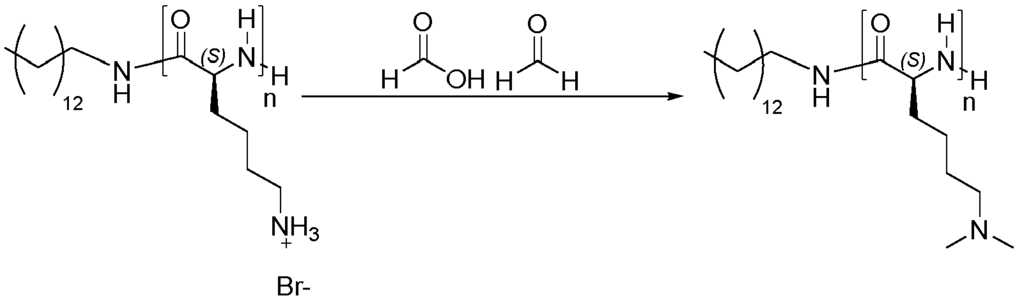

The lipo-polymer $C_{14}$-PLys as obtained in Example 3.2 was dissolved in formaldehyde (1 eq per Lysine unit) and formic acid (2.3 eq per Lysine unit). The mixture was heated to 85 °C and stirred for 16 hours. The reaction mixture was concentrated by rotavapor and redissolved in methanol. The mixture was precipitated with diethyl ether, isolated by centrifugation (3750 rpm, 4 min) and dried under vacuum. The product was dissolved in $H_2O$, purified by sephadex column (PD-10 Desalting Columns) and lyophilized. The polymer ($C_{14}$-PLys(Dimet)) was isolated as a white solid.

Yield: 50%. $^1H$ NMR (300 MHz, $D_2O$) δ 4.49 - 4.01 (m, CH), 3.17 (brs, $CH_2$), 2.86 (s, $CH_3$), 1.97 - 1.35 (m, $CH_2$), 1.26 (brs, $CH_2$), 0.84 (t, *J* = 6.7 Hz, $CH_3$).

Following the above general procedure, the polymer shown in Table 12 was obtained:

Table 12. Polymers based on $C_{14}$-PLys(Dimet) (Lipo-PBet$_x$)

| Code | Precursor | DP (n) for Lys(TFA)[a] |
|---|---|---|
| Lipo-PBet5 | Lipo-PBet3 | 5 |
| Lipo-PBet6 | Lipo-PBet4 | 36 |
| *[a]Determined by NMR, wherein the cited DP numbers are subject to a reasonable uncertainty within the range ±20%.* | | |

3.4 General procedure for incorporating tert-butyl bromoacetate to $C_{14}$-PLys(Dimet)

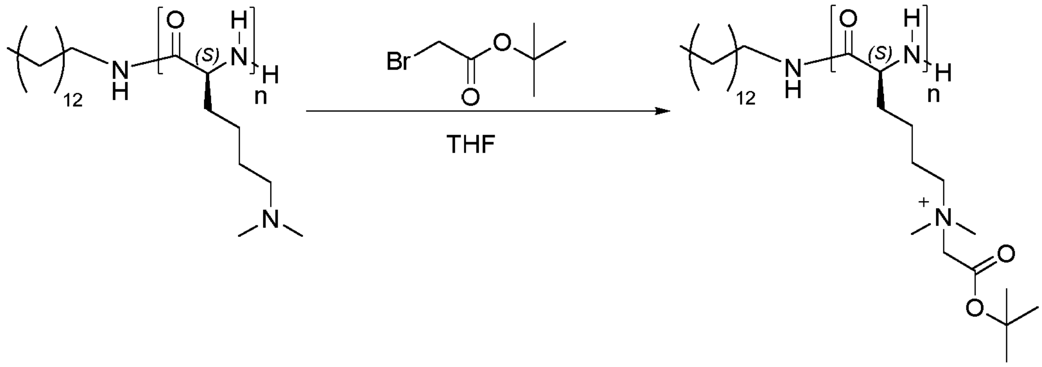


$C_{14}$-PLys(Dimet) as obtained in Example 3.3 was dissolved in DMF (50 mg/mL). Then, tert-butyl bromoacetate (6 eq per Lysine unit) was added to the reaction mixture and stirred for 5 minutes at RT. After this time, TBAI (0.5 eq per Lysine unit) and DIPEA (3 eq per Lysine unit) were added. The mixture was stirred at RT for 16 hours. The reaction mixture was concentrated by rotavapor and redissolved in DCM. The mixture was precipitated with diethyl ether, isolated by centrifugation (3750 rpm, 4 min) and dried under vacuum. The product was used without further purification or characterization. Yield: 75%.

3.5 General procedure for tert-butyl deprotection step:

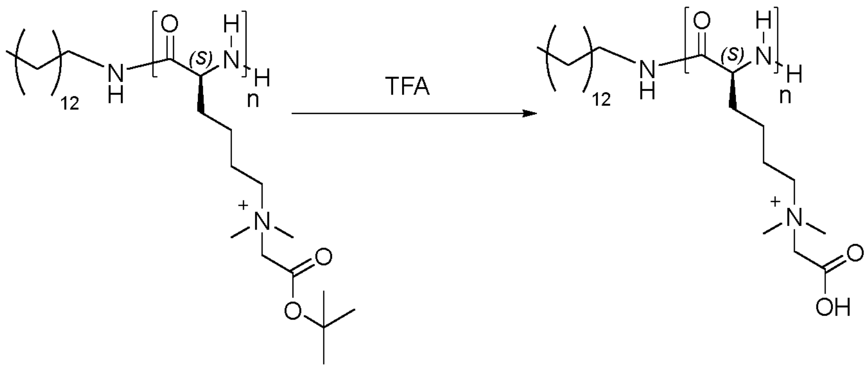


The protected lipo-polymer $C_{14}$-PBet as obtained in Example 3.4 was dissolved in cold trifluoroacetic acid (50 mg/mL) and stirred at RT for 24 hours. The reaction mixture was poured into diethyl ether to precipitate the product. The precipitate was isolated by centrifugation (3750 rpm, 4 min) and dried under vacuum. The solid was redissolved in $H_2O$, dialyzed against miliQ water (Mw cutt of 3000 Da) during 24h and lyophilized. The polymer was isolated as a white solid.

Yield:70%. 1H NMR (300 MHz, $D_2O$) δ 4.31 (m, CH), 4.05 (m, $CH_2$), 3.70 (brs, $CH_2$), 3.27 (s, $CH_3$), 2.09 - 1.41 (m, $CH_2$), 1.31 (m, $CH_2$), 0.92 (t, $J$ = 6.2 Hz, $CH_3$).

Following the above general procedure, the polymer shown in Table 13 was obtained:

**Table 13.** Polymers based on $C_{14}$-PBet (Lipo-PBet$_x$)

| Code | Precursor | DP (n) for Lys(TFA)[a] | Mw by GPC (KDa)[b] | PDI |
|---|---|---|---|---|
| Lipo-PAA7 | Lipo-PBet5 | 6 | 1.9* | 1.06* |
| Lipo-PAA8 | Lipo-PBet6 | 36 | 6.6 | 1.33 |

[a]*Determined by NMR, wherein the cited DP numbers are subject to a reasonable uncertainty within the range ±20%.*
[b]*Determined by GPC, wherein the cited DP numbers are subject to a reasonable uncertainty within the range ±20%.*based in the precursor.*

Example 4. Synthesis of lipo-shielding DMPE(14:0)-PGA(DIOL)

4.1 General procedure for the polymerization of DMPE(14:0)-PGluOBzl

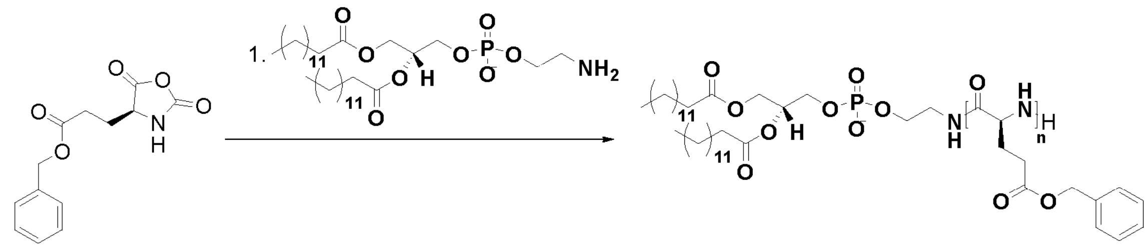


N-Carboxy-*L*-glutamic acid-benzyl- anhydride (NCA) was added to a Schlenk tube fitted with a stirring bar and a stopper. After 3 cycles of vacuum/$N_2$, monomer was dissolved in anhydrous DMF. Then, the initiator (1,2-dimyristoyl-sn-glycero-3-phosphoethanolamine, DMPE(14:0), Avanti Polar Lipids) was diluted in chloroform (5 mL) and added to the reaction mixture, which was stirred at room temperature for 16 hours. Once NCA consumption was confirmed by IR the reaction mixture was poured into diethyl ether to precipitate the product. The precipitate (DMPE(14:0)-PGlu(OBzl)) was isolated by centrifugation (3750 rpm, 4 min) and dried under vacuum. Yield: 70-90%. 1H NMR (300 MHz, TFA) δ8.08 (m, CH aryl), 5.93 (m, $CH_2$ benzylic), 5.49 (brs, CH), 3.56 - 2.71 (m, $CH_2$), 2.49 (m, $CH_2$), 2.08 (m, $CH_2$), 1.64 (t, $J$ = 6.6 Hz, $CH_3$).

Following the above general procedure, the polymer shown in Table 14 was obtained:

**Table 14.** Polymers based on DMPE(14:0)-PGlu(OtBu) (Lipo-$PGA_x$)

| Code | Initiator | DP (n)for Glu(OBzl)[a] | Mw by GPC (KDa)[b] | PDI |
|---|---|---|---|---|
| Lipo-PGA9 | DMPE(14:0) | 48 | 11.07 | 1.05 |
| Lipo-PGA10 | DMPE(14:0) | 36 | 8.6 | 1.11 |

*[a]Determined by NMR. [b]Determined by GPC, wherein the cited* DP *numbers are subject to a reasonable uncertainty within the range ±20%.*

4.2 General procedure for deprotection step:

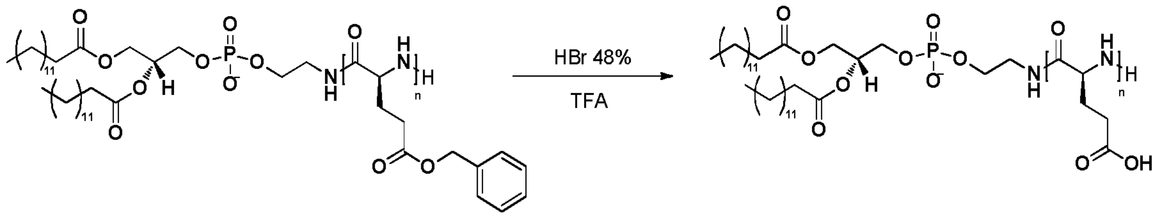


The lipo-polymer DMPE(14:0)-PGlu(OBzl) as obtained in Example 4.1 was dissolved in trifluoroacetic acid (100 mg/mL) and HBr 48% (2 eq per Glutamic acid unit) was added. The mixture was stirred at RT for 16 hours. The reaction mixture was poured into diethyl ether to precipitate the product. The precipitate was washed with a mixture of Acetonitrile:$H_2O$ (8:2) until pH 4-5. Then, the product is washed again with diethyl ether, isolated by centrifugation (3750 rpm, 4 min) and dried under vacuum.

The polymer (DMPE(14:0)-PGA) was isolated as a white solid. Yield: 80%.

$^1H$ NMR (300 MHz, D2O) δ 4.47 - 3.80 (m, CH), 2.45 - 1.76 (m, $CH_2$), 1.57 (brs, $CH_2$), 1.24 (m, $CH_2$), 0.83 (t, $J$ = 6.55 Hz, $CH_3$).

Following the above general procedure, the polymer shown in Table 15 was obtained:

**Table 15.** Polymers based on DMPE(14:0)-PGA (Lipo-$PGA_x$)

| Code | Precursor | DP (n)for Glu(ONa)[a] |
|---|---|---|
| Lipo-PGA11 | Lipo-PGA9 | 30 |

(continued)

| Code | Precursor | DP (n)for Glu(ONa)[a] |
|---|---|---|
| Lipo-PGA12 | Lipo-PGA10 | 21 |
| [a]*Determined by NMR.* | | |

4.3 General procedure for peptide coupling with CDI for the synthesis of DMPE(14:0)-PGA(DIOL)

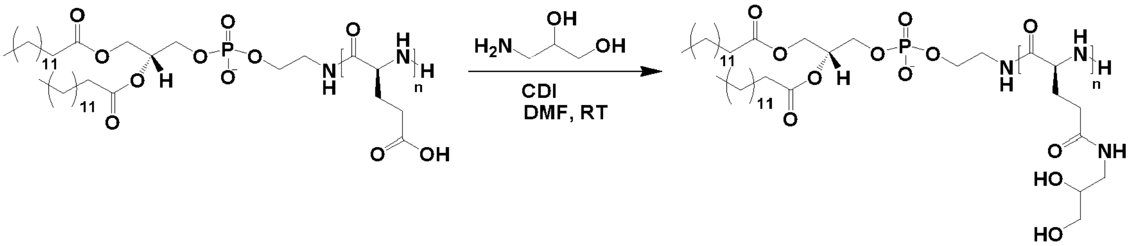


Introduction of 3-aminopropane-1,2-diol to the lateral chain of DMPE(14:0)-PGA as obtained in Example 4.2 was carried out following the procedure described above in example 1.4, generating the corresponding DMPE(14:0)-PGA(DIOL).

Yield: 60-70. $^{1}H$ NMR (300 MHz, D2O) $\delta$ 4.38 (brs, CH), 3.87 (brs, CH), 3.63 (ddd, $J$ = 18.2, 12.0, 5.5 Hz, $CH_2$), 3.35 (ddd, $J$ = 21.1, 14.1, 5.7 Hz, $CH_2$), 2.68- 1.92 (m, $CH_2$), 1.34 (brs, $CH_2$), 0.94 (s, $CH_3$).

Following the above general procedure, the polymer shown in Table 16 was obtained:

**Table 16.** Polymers based on DMPE(14:0)-PGA(DIOL) (Lipo-PGA$_x$)

| Code | Precursor | DP (n) for Glu(DIOL)[a] | Mw by GPC (KDa)[b] | PDI |
|---|---|---|---|---|
| Lipo-PAA9 | Lipo-PGA13 | 44 | 9.6 | 1.51 |
| Lipo-PAA10 | Lipo-PGA14 | 32 | 7.0 | 1.18 |
| [a]*Determined by NMR.* [b]*Determined by GPC, wherein the cited DP numbers are subject to a reasonable uncertainty within the range ±20%.* | | | | |

Example 5. Synthesis of lipo-shielding Dimyrist-GABA-PGA(DIOL)$_n$ (reference example)

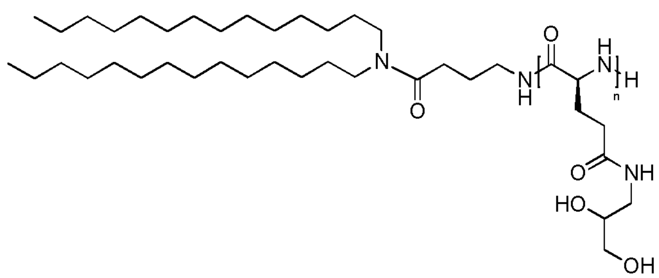

5.1 General procedure for the synthesis of initiator (y)

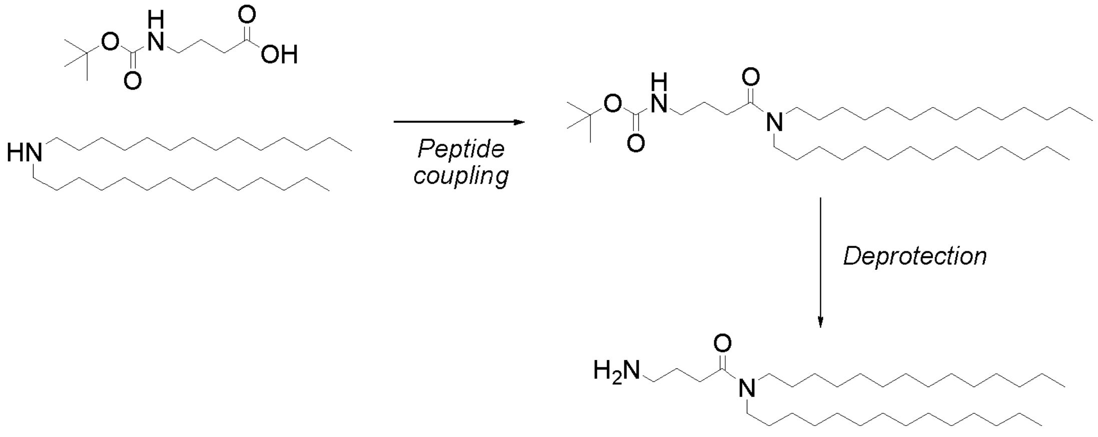


(y)

5.1a. General procedure for synthesis of tert-butyl (4-(ditetradecylamino)-4-oxobutyl)carbamate.

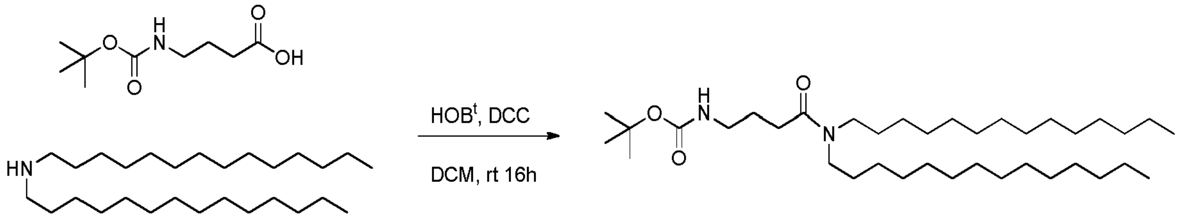


Boc-GABA-OH (4-(Boc-amino)butyric acid: 400 mg, 1.968 mmol) was dissolved in DCM (9 mL). HOBt (1.1 eq, 2.16 mmol, 292 mg) and dicyclohexyl carbodiimide (DCC) (1.1 eq, 2.16 mmol, 447 mg) were added to the reaction mixture and the solution stirred at rt for 30 minutes. Then, Dimyrist (ditetradecylamine: 1.1 eq, 2.16 mmol, 885 mg) was added and the reaction mixture stirred for 16 hours at room temperature. The completion of the reaction was monitored by TLC. The reaction was filtered, and the filtrate was concentrated to dryness using rotary evaporation. Then, the crude of the reaction was purified by Flash Column chromatography using Hexane: Ethyl acetate (8:2) as eluent. The product obtained was concentrated to dryness and isolated as a yellowish oil (m obtained: 1.12g). Yield: 95%.

$^1$H NMR (300 MHz, $CHCl_3$) δ 4.84 (brs, CH), 3.27 (m, $CH_2$), 3.17 (dd, J = 13.6, 6.4 Hz, $CH_2$), 2.32 (t, J= 7 Hz, $CH_2$), 1.82 (p, J = 6.6 Hz, CH), 1.57-1.46 (m, $CH_2$), 1.42 (s, $CH_3$), 1.26 (brs, $CH_2$), 0.87 (t, J = 6.7 Hz, $CH_3$).

5.1b. General procedure for the deprotection step.

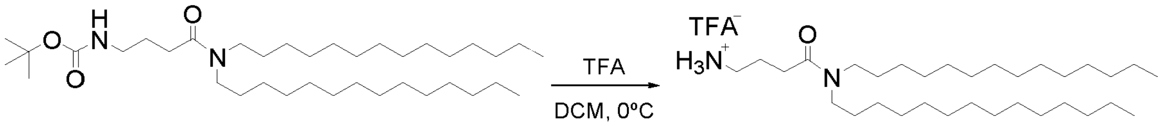


The tert-butyl (4-(ditetradecylamino)-4-oxobutyl)carbamate was dissolved in DCM (9 mL) and TFA was added dropwise to the reaction mixture at 0°C. The reaction mixture was stirred at room temperature for 16h. Then, the reaction crude was dryness using rotary evaporation and used without prior purification in the next reaction. Yield: 100%.

$^1$H NMR (300 MHz, $CHCl_3$) δ 7.80 (brs, $NH_2$), 3.26-2.93 (m, $CH_2$), 2.55 (brs, $CH_2$), 1.93 (brs, CH), 1.46 (m, $CH_2$), 1.19 (brs, $CH_2$),0.81 (t, J = 6.7 Hz, $CH_3$).

5.2 General procedure for the polymerization of Dimyrist-GABA-PGlu(OBzl)

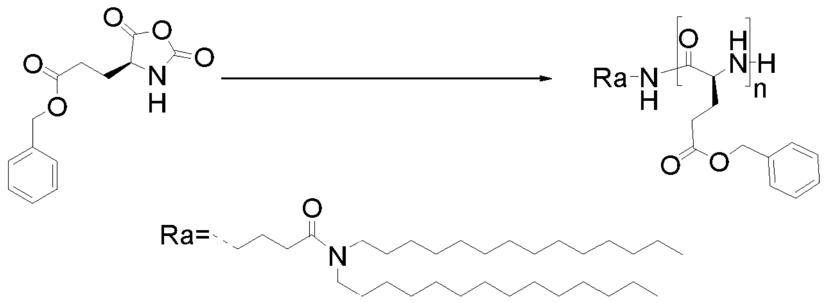

Glutamic acid Benzyl ester NCA (1.33 g, 10.29 mmol) was added to a Schlenk tube fitted with a stirring bar and a stopper. After 3 cycles of vacuum/$N_2$, the mixture was dissolved in anhydrous DMF (150 mg reagent per mL DMF). Then, the initiator (Dimyrist-GABA-$NH_3$TFA.: DP20: 254 mg, 0.51 mmol) was diluted in DMF (5 mL) and DIPEA (1 eq, 0.51 mmol, 896 μL) was added to the initiator solution. Then, the initiator solution was added to the reaction mixture, which was stirred at 10 °C for 16 hours. Once NCA consumption was confirmed by IR, the reaction mixture was poured into diethyl ether to precipitate the product. The precipitate was isolated by centrifugation (3750 rpm, 4 min) and dried under vacuum. Homopolymer (Dimyrist-GABA-PGluOBzl) was isolated as a white solid. Yield: 70-90%.

$^1$H NMR (300 MHz, TFA) δ 7.36 (m, CH aryl), 5.35 (m, $CH_2$ benzylic), 4.78 (m, CH), 3.66 (m, $CH_2$), 2.92 - 1.98 (m, $CH_2$), 1.84 (d, J = 7.7 Hz, $CH_2$), 1.53-1.30 (m, $CH_2$), 0.82 (t, J = 5.9 Hz, $CH_3$).

**Table 17.** Polymers based on DimyristGABA$NH_2$-PGlu (Lipo-PGA$_x$)

| Code | Initiator | DP (n) for Glu(OBzl)[a] | Mw by GPC (KDa)[b] | PDI |
|---|---|---|---|---|
| Lipo-PGA15 | DimyristGABA$NH_2$ | 15 | 4.73 | 1.01 |
| Lipo-PGA16 | DimyristGABA$NH_2$ | 93 | 20.98 | 1.17 |
| [a]*Determined by NMR.* [b]*Determined by GPC, wherein the cited DP numbers are subject to a reasonable uncertainty within the range* ±20%. | | | | |

5.3 General procedure for deprotection step:

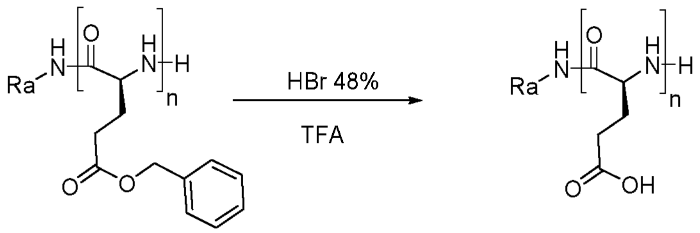


The lipo-polymer Dimyrist-GABA-PGlu(OBzl)-$COCH_3$ (1. g, 4.8 mmol) as obtained in Example x.2 was dissolved in trifluoroacetic acid (100 mg/mL) and HBr 48% (2 eq per Glutamic acid unit) was added. The mixture was stirred at RT for 16 hours. The reaction mixture was poured into diethyl ether to precipitate the product. The precipitate was washed with a mixture of Acetonitrile:$H_2O$ (8:2) until pH 4-5. Then, the product is washed again with diethyl ether, isolated by centrifugation (3750 rpm, 4 min) and dried under vacuum. The polymer (Dimyrist-GABA-PGA) was isolated as a white solid.

Yield: 95%. $^1$H NMR (300 MHz, $D_2O$) δ 4.92 (m, CH), 3.70 (m, $CH_3$), 3.13-2.08 (m, $CH_2$), 1.87 (brs, $CH_2$), 1.44 (m, $CH_2$), 0.94 (brs, $CH_3$).

**Table 18.** Polymers based on DimyristGABA-PGA (Lipo-PGAn)

| Code | Precursor | DP (n) for Glu(ONa)[a] |
|---|---|---|
| Lipo-PGA17 | Lipo-PGA15 | 15 |

(continued)

| Code | Precursor | DP (n) for Glu(ONa)[a] |
|---|---|---|
| Lipo-PGA18 | Lipo-PGA16 | 67 |
| [a] *Determined by NMR, wherein the cited DP numbers are subject to a reasonable uncertainty within the range* ±20%. | | |

5.4 General procedure for peptide coupling with CDI for the synthesis of $C_{14}$-PGA(DIOL)$_n$.

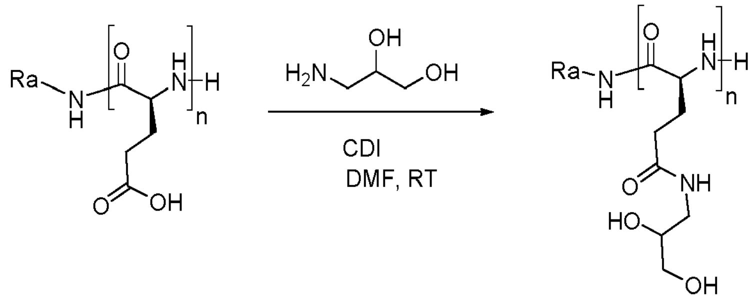


Dimyrist-GABA-PGA (PGADIOL 0.4 g, 3.3 mmol) as obtained in Example 5.3 was dissolved in DMF (50 mg/mL). Then, CDI (1.6 eq per Glutamic acid unit) was added to the reaction mixture and stirred for 30 minutes at room temperature. After this time, the 3-aminopropane-1,2-diol (1.3 eq per Glutamic acid unit) dissolved in 2 mL of DMF was added. The mixture was stirred at room temperature for 16 hours. The reaction mixture was poured into acetone to precipitate the product. Precipitate was isolated by centrifugation (3750 rpm, 4 min) and dried under vacuum. The final product was isolated as a white solid. Yield: 50-60%.

$^1$H NMR (300 MHz, $D_2O$) δ 4.33 (brs, CH), 3.88 (brs, CH), 3.71-3.49 (m, $CH_2$), 3.43-3.20 (m, $CH_2$), 2.98 (dd, *J* = 13.2, 3.5 Hz, $CH_2$), 2.83 (dd, *J* = 13.2, 8.5 Hz, $CH_2$), 2.56 - 1.91 (m, $CH_2$),1.30 (brs. $CH_2$), 0.90 (brs, $CH_3$).

**Table 19.** Polymers based on capped Dimyrist-GABA-PGA(Diol) (Lipo-PGAn)

| Code | Precursor | DP (n) for Glu(Diol)[a] | Mw by GPC (KDa)[b] | PDI |
|---|---|---|---|---|
| Lipo-PAA11 | Lipo-PGA17 | 16 | 3.91 | 1.07 |
| Lipo-PAA12 | Lipo-PGA18 | 94 | 19.46 | 1.29 |
| [a]*Determined by NMR.* [b]*Determined by GPC, wherein the cited DP numbers are subject to* a *reasonable uncertainty within the range* ±*20%.* | | | | |

Example 6. Lipid nanoparticles formulation

In the following examples the oligonucleotides used were: a pDNA purchased from Plasmid Factory, with reference PF461 (pCMV-luc)), containing 6233 bp expressing luciferase and a mRNA purchased from Trilink, with reference L-1201-1000 CleanCap Fluc mRNA (5moU) expressing luciferase as reporter gene. Any other oligonucleotide with the characteristics above indicated could be used to carry out the experiments below. The ionizable lipid DLin-MC3-DMA, or (6Z,9Z,28Z,31Z)-heptatriacont-6,9,28,31-tetraene-19-yl 4-(dimethylamino)butanoate was purchased from Nanosoft Polymers. Shielding lipid 1,2-Dimyristoyl-rac-glycero-3-methoxypolyethylene glycol-2000 (DMG-PEG2000) was purchased from Avanti Polar Lipids. Both polymers were used to form benchmark LNPs. Structural lipids used to form LNPs were: 1,2-distearoyl-sn-glycero-3-phosphocholine (DSPC, from Avanti Polar Lipids, 850365P) and Cholesterol (Sigma-Aldrich, C8667).

A microfluidic HerringboneMixer from Darwing Microfluidics was used for the formulation of LNPs 1-12. The reactor presents 2 inlet-channels (one for the DNA in aqueous phase and the other for the lipid mixture in ethanol) and 1 outlet-channel. The specifications of the mixer are the following:

- Slide format: 25 x 75 m
- Channel depth: 0.08 mm

- Channel width: 0.1 to 0.5 mm
- Volume: 3.3 μL
- Volume Mixer: 0.47 μL
- Length Mixer: 28.7 mm
- Material: Glass
- Connectors: 1/4"-28 Fittings

In addition, two programmable pumps control the fluid flow rates of the syringes (NE-1000 Programmable Single Syringe Pump, Syringe Pump, USA). The system accepts infusion rates from 0.73 μL/h (1 mL syringe) to 2100 mL/h (60 mL syringe). This methodology provides reproducibility to the formation of LNPs as well as the possibility of scaling up the process. Benchmark LNPs were formulated as follows:

- Lipids were disolved in ethanol at 50/10/38.5/1.5 molar ratio (MC3/DSPC/Cholesterol/DMG-PEG)
- pDNA was diluted in acetat buffer 25 mM at pH 4
- Amine to phosphate ratio (N/P) was 4
- Flow ratio was 3:1 (ethanol:$H_2O$)
- Total flow was 2 mL/min
- Tubing internal diameter was 0.5 mm
- After formulation, LNPs were diluted in 10 mM Phosphate buffer saline (PBS) pH 7.4 and concentrated with centrifugal concentrators (Vivaspin™ 500, Sigma) to remove ethanol

The formulations containing the lipo-polyamino acid conjugates of formula (I) were prepared analogously with the difference that lipo-polyamino acid conjugates of formula (I) were used instead of DMG-PEG and the lipid ratio was different (shown below).

Benchmark LNPs 13-17 were formulated with NanoScaler (KNAUER) as follows:

- Lipids were dissolved in ethanol at 46.3/9.4/42.7/1.6 molar ratio (ALC-0315/DSPC/Cholesterol/ DMG-PEG).
- mRNA was diluted in acetate buffer 25 mM at pH 4
- Amine to phosphate ratio (N/P) was 6.2
- Flow ratio was 1:3 (ethanol:H2O)
- Total flow rate for LNPs was 4.5 mL/min (3mL/min undiluted LNPs + 1.5mL/min in -line dilution with PBS)

After formulation, LNPs were further diluted in 10 mM Phosphate buffer saline (PBS) pH 7.4 to decrease ethanol concentration and purified with centrifugal concentrators (Vivaspin™ 500, Sigma) to remove ethanol. The formulation containing the polymer of the invention was prepared analogously with the difference that different LipoPAA was used instead of shielding lipid DMG-PEG and the lipid ratio was different (shown below Table 27 and 28).

## Example 7. LNPs Characterization

### 7.1 Size and polydispersity

Size and polydispersity (PDI) of the formulated LNPs containing mRNA or pDNA at different N/P ratios (positively-chargeable polymer amine (N = nitrogen) groups to negatively-charged nucleic acid phosphate (P)), different molar ratios and shielding polymers were performed using a Malvern ZetasizerNanoZS instrument, equipped with a 532 nm laser at a fixed scattering angle of 173°. 50 μl of the samples were measured using a quartz glass high performance cuvette (Hellma Analytics). Size distribution was measured (diameter, nm) with n ≥ 3 measurements.

Benchmark LNPs containing MC3 as ionizable lipid, DMG-PEG as shielding lipid and mRNA as cargo, were formulated at 50/10/38.5/1.5 molar ratios for MC3/DSPC/Cholesterol/DMG-PEG obtaining the following results:

**Table 20.** Size and PDI of comparative benchmark LNPs containing mRNA

| Comp. LNP | Q total (mL/min) | % MC3 | % DSPC | % Chol | % DMG-PEG | NIP | PDI | Z-average (nm) |
|---|---|---|---|---|---|---|---|---|
| Comp. LNP1 | 2 | 50 | 10 | 38.5 | 1.5 | 4 | 0.125 | 114.7±1.8 |
| Comp. LNP2 | 2 | 50 | 10 | 38.5 | 1.5 | 4 | 0.227 | 105.9±0.6 |
| Comp. LNP3 | 2 | 50 | 10 | 38.5 | 1.5 | 4 | 0.166 | 103.8±1.5 |

(continued)

| Comp. LNP | Q total (mL/min) | % MC3 | % DSPC | % Chol | % DMG-PEG | NIP | PDI | Z-average (nm) |
|---|---|---|---|---|---|---|---|---|
| Comp. LNP4 | 2 | 50 | 10 | 38.5 | 1.5 | 4 | 0.228 | 96.6±0.4 |

The presence of DMG-PEG at 1.5% is enough to stabilize the LNPs obtaining a good size (expressed as Z-average) and PDI and reproducible formulations.

Table 21 shows the results obtained of Lipo-PAA1 formulated at molar ratio of 2% and 5% containing pDNA as cargo.

**Table 21.** Size and PDI of Lipo-PAA1 LNPs containing pDNA.

| LNP | Q total (mL/min) | % MC3 | % DSPC | % Chol | % Lipo-PAA1 | NIP | PDI | Z-average (nm) |
|---|---|---|---|---|---|---|---|---|
| LNP1 | 2 | 50 | 10 | 38 | 2 | 4 | 0.184 | 167.1 ±1.9 |
| LNP2 | 2 | 50 | 10 | 38 | 2 | 4 | 0.191 | 154.2 ±1.0 |
| LNP3 | 2 | 50 | 10 | 38 | 2 | 4 | 0.179 | 133.5±0.7 |

Results suggest that the best results were achieved at 2% of Lipo-PAA1 being consistent in all replicates, showing low PDI values and appropriate sizes. This composition was also used to formulate LNPs with mRNA obtaining low PDI values and appropriate sizes (Table 22).

**Table 22.** Size and PDI of Lipo-PAA1 LNPs containing mRNA.

| LNP | Q total (mL/min) | % MC3 | % DSPC | % Chol | % Lipo-PAA1 | NIP | PDI | Z-average (nm) |
|---|---|---|---|---|---|---|---|---|
| LNP4 | 2 | 50 | 10 | 38 | 2 | 4 | 0.194 | 213.3±2.0 |
| LNP5 | 2 | 50 | 10 | 38 | 2 | 4 | 0.116 | 188.6±1.1 |

Table 23 shows the results obtained of Lipo-PAA2 formulated at molar ratio of 5% containing pDNA as cargo.

**Table 23.** Size and PDI of Lipo-PAA2 LNPs containing pDNA.

| LNP | Q total (mL/min) | % MC3 | % DSPC | % Chol | % Lipo-PAA2 | NIP | PDI | Z-average (nm) |
|---|---|---|---|---|---|---|---|---|
| LNP6 | 2 | 50 | 10 | 35 | 5 | 4 | 0.187 | 128.4±0.75 |
| LNP7 | 2 | 50 | 10 | 35 | 5 | 4 | 0.120 | 124.7 ±1.8 |
| LNP8 | 2 | 50 | 10 | 35 | 5 | 4 | 0.158 | 126.0 ± 1.1 |

In this case, results suggest that the best results were achieved at 5% of Lipo-PAA2. In addition, when using 5% of polymer the results were consistent in all replicates, showing low PDI values and appropriate sizes. This composition was also used to formulate LNPs with mRNA obtaining low PDI values and appropriate sizes (Table 24).

**Table 24.** Size and PDI of Lipo-PAA2 LNPs containing mRNA.

| LNP | Q total (mL/min) | % MC3 | % DSPC | % Chol | % Lipo-PAA2 | NIP | PDI | Z-average (nm) |
|---|---|---|---|---|---|---|---|---|
| LNP9 | 2 | 50 | 10 | 35 | 5 | 4 | 0.396 | 238.8±2.0 |
| LNP10 | 2 | 50 | 10 | 35 | 5 | 4 | 0.223 | 172.4±0.7 |

Table 25 shows the results obtained of Lipo-PAA7 formulated at molar ratio of 5% directly containing mRNA as cargo.

**Table 25.** Size and PDI of Lipo-PAA7 LNPs containing mRNA.

| LNP | Q total (mL/min) | % MC3 | % DSPC | % Chol | % Lipo-PAA7 | NIP | PDI | Z-average (nm) |
|---|---|---|---|---|---|---|---|---|
| LNP11 | 2 | 50 | 10 | 35 | 5 | 4 | 0.213 | 155.9±1.7 |

As Lipo-PAA9 is similar to Lipo-PAA1, Table 26 shows the results obtained at molar ratio of 2% directly containing mRNA as cargo.

**Table 26.** Size and PDI of Lipo-PAA9 LNPs containing mRNA.

| LNP | Q total (mL/min) | % MC3 | % DSPC | % Chol | % Lipo-PAA9 | NIP | PDI | Z-ave (nm) |
|---|---|---|---|---|---|---|---|---|
| LNP12 | 2 | 50 | 10 | 38 | 2 | 4 | 0.178 | 108.2±0.5 |

Benchmark LNPs containing ALC-0315 as ionizable lipid, DMG-PEG as shielding lipid and mRNA as cargo, were formulated at 46.3/9.4/44.3-x/x molar ratios for ALC-0315/DSPC/Cholesterol/DMG-PEG or $C_{14}$-PSar obtaining the following results:

**Table 27.** Size and PDI of comparative benchmark DMG-PEG and $C_{14}$-PSar LNPs containing mRNA formulated at molar ratio of 1.6 and 5% respectively:

| Comp. LNP | LipoPAAs | Q total (mL/min) | % Chol | % Lipo-Shield (x) | NIP | PDI | Z-average (nm) |
|---|---|---|---|---|---|---|---|
| Comp. LNP13 | DMG-PEG | 4.5 | 42.7 | 1.6 | 6.17 | 0.206 | 84.8 ± 2.6 |
| Comp. LNP14 | $C_{14}$-PSar | 4.5 | 39.3 | 5 | 6.17 | 0.117 | 75.2 ± 2.8 |

LNPs were obtained with a good size (expressed as Z-average) and PDI and reproducible formulations.

Table 28 shows the results obtained of Lipo-PAA1, Lipo-PAA8 and Lipo-PAA10 formulated at molar ratio of 2% containing mRNA as cargo:

**Table 28.** Size and PDI of Lipo-PAAs LNPs containing mRNA.

| LNP | LipoPAAs | Q total (mL/min) | % ALC-0315 | % DSPC | % Chol | % Lipo-PAA (x) | NIP | PDI | Z-average (nm) |
|---|---|---|---|---|---|---|---|---|---|
| LNP15 | LipoPAA1 | 4.5 | 46.3 | 9.4 | 42.3 | 2 | 6.2 | 0.144 | 97.0 ± 4.7 |
| LNP16 | LipoPAA8 | 4.5 | 46.3 | 9.4 | 42.3 | 2 | 6.2 | 0.166 | 150.4 ± 22.5 |
| LNP17 | LipoPAA10 | 4.5 | 46.3 | 9.4 | 42.3 | 2 | 6.2 | 0.142 | 120.7 |

Results suggest that at 2% of Lipo-PAA1, Lipo-PAA8 and Lipo-PAA10 are consistent in all replicates, showing low PDI values and appropriate sizes.

<u>7.2 Free oligonucleotide detection by gel electrophoresis</u>

The possible presence of free pDNA or mRNA after LNPs formulation were assessed using an electrophoresis gel as first screening method. To perform the electrophoresis, E-gel Power Snap Electrophoresis Device and E-Gel Power Snap Camera (Invitrogen) was used. 2% agarose gels prepared that include the SYBR Gold DNA marker (E-Gel™ EX Agarose Gels, 2%, Invitrogen) were used following manufacturer's instructions. The LNPs (20 μl) composed by the different lipo-polypeptide shieldings indicated in Figure 1 were evaluated, and also the disassembly of the LNPs in the presence of 10% triton X-100 (Scharlab,S.L) and 2 mg/mL heparin (PanReacAppliChem, Spain). Once the gel was loaded (20μl/well), the equipment protocol was selected according to the type of gel used (in our case, protocol approximately 10 min, although the time can be modified according to the samples).

In all cases, no free mRNA was observed when different lipo-polypeptide shieldings were tested. However, when 10 %Triton X-100 was used, free mRNA signals were observed due to that capacity of Triton X-100, as a surfactant, to disassemble the different components of the LNPs, and thus, release the LNPs cargo (Representative image of the gels can be observed in Figure 1).

7.3 pKa of the formulated LNPs

The positively charged amino groups in nanoparticles depends on the pH of the medium and the apparent pKa can predict these changes. The influence of lipo-polypeptides on the LNPs surface can alter this apparent pKa, and therefore, affect on the tranfection efficiency. LNPs apparent pKa values were determined by measuring the fluorescence of 2-(p-toluidino)-6-napthalene sulfonic acid (TNS) during titration with different buffer solutions: from pH 4 to 5.5 (0.5 pH unit increments) with 20 mM Acetate buffer; from pH 6 to 7.5 (0.5 pH unit increments) with 20 mM Phosphate buffer; from pH 8 to 9 (0.5 pH unit increments) with 20 mM Borate buffer. Formulations were diluted to 20 μM lipid concentration with different buffer solutions and added to a black 96-well plate. TNS solution (0.3 mM in DMSO) was added to a final concentration of 6 μM. Fluorescence was quantified at $\lambda_{em}$ = 445 nm, $\lambda_{ex}$ = 321 nm and the pH at half-maximal fluorescence intensity was defined as pKa.

**Table 29.** Different parameters of LNPs formulated with lipo-polypeptides. Total flow used was 2mL/min. N/P ratio was 4.

| LNP | Lipo-shielding Compound | % MC3 | % DSPC | % Chol | % Lipo-shield | PDI | Z-average (nm) | pKa |
|---|---|---|---|---|---|---|---|---|
| Comp. LNP4 | DMG-PEG | 50 | 10 | 38.5 | 1.5 | 0.228 | 96.6±0.4 | 6.3-6.6 |
| LNP4 | Lipo-PAA1 | 50 | 10 | 38 | 2 | 0.194 | 213.3±2 | 6.2-6.3 |
| LNP9 | Lipo-PAA2 | 50 | 10 | 35 | 5 | 0.396 | 238.8±2 | 6.6-6.7 |

The pKa of the LNPs formulated with Lipo-PAA1 and Lipo-PAA2 are similar to the benchmark LNPs, which is comprised between 6.2 to 6.7. It is reported that this range of pKA is ideal to obtain the best performance of the LNPs.

Example 8. *In vitro* biological studies in HeLa cells

8.1 Cell Culture

HeLa cells were cultured in DMEM high glucose with Glutamax (Gibco- Thermo Fisher # 61965-059) supplemented with 10% of Fetal Bovine Serum (Hyclone # SV30160.03HI, provided by GE Healthcare Europe GmbH) and 1 % penicillin/streptomycin. Transfections were carried out on 96-well plates containing 10000cells/well in a final volume of 100μl, and cells were incubated 24 hours at 37 °C and 5% $CO_2$. After 24 h from cell seeding, the medium was removed and refreshed with 90μl of complete medium. 10μl of each LNPs formulation were added to the cells. In the case of positive control (Lipofectamine™ 2000 Transfection Reagent, Invitrogen™), manufacturer guidelines were followed. After 24 hours cells were recovered and processed.

8.2 ATP Evaluation for Cell Toxicity evaluation

After 24h post-incubation with the LNPs formulation, the medium was aspirated and 50 μl/well of ATPLite reagent (ATPLite PerkinElmer #6016731) were added. The plate was incubated 10 minutes at room temperature in the dark. Luminiscence was read spectrophotometrically using VictorNivo (PerkinElmer) following manufacturer's instructions and data was represented as the percentage of cell viability, taken untreated control cells as 100%.

8.3 Luciferase Assay for transfection efficiency evaluation

After 24h post-incubation with the LNPs formulation, 100μl of BrightGlo reagent (Promega # E2620) was added in each well following manufacturer instructions. After 5 minutes of incubation at room temperature luciferase activity was measured using VictorNivo (PerkinElmer). Data was represented as luminescence relative to the percentage of transfection relative to the positive control of transfection.

8.4 Biological activity of LNPs in HeLa cells

The transfection efficiency and the cell viability of the LNPs formulations in HeLa cells is reported in the following table. The transfection data is represented as % of the positive control Lipofectamine™ 2000 being the positive control 100% after 24h of treatment and cell viability is compared to non-treated (NT) cells, being the ATP content readout of NT (non-treated) cells equal to 100%.

**Table 30.** Different parameters of LNPs formulated with lipo-polypeptides. Molar ratio was 50/10/40-x/x, where x is the Total flow used was 2mL/min. N/P ratio was 4 and nucleic acid content was 1μg mRNA/well. lipo-polypeotide. Total flow used was 2mL/min. N/P ratio was 4 and nucleic acid content was 1 μg mRNA/well.

| LNP | Lipo-shielding Compound | % Chol | % Lipo-shield (x) | PDI | Z-average (nm) | % Transf vs Lipofect | % Viability |
|---|---|---|---|---|---|---|---|
| Comp. LNP4 | DMG-PEG | 38.5 | 1.5 | 0.228 | 96.6±0.4 | 17.9±11.0 | 95.1±5.3 |
| LNP4 | Lipo-PAA1 | 38 | 2 | 0.194 | 213.3±2 | 257.2±56.8 | 91.9±9.6 |
| LNP9 | Lipo-PAA2 | 35 | 5 | 0.396 | 238.8±2 | 61.3±13.3 | 92.7±19.8 |

According to the results, the transfection obtained from both LNPs containing PGADIOL derivates was higher than those LNPs containing DMG-PEG. Additionally, the compound Lipo-PAA1 transfected the cells 2.5-fold more efficiently than the gold standard Lipofectamine. Therefore, it can be concluded that by adding lipo-PAA into LNPs, the following is obtained: i) stable nanoparticles with acceptable size and polydispersity; ii) non-cytotoxic formulation, and iii) high transfection efficiency. These results demonstrate that the lipo-PAA presented in this application can be an alternative of the actual DMG-PEG compound.

Example 9. *In vitro* biological studies in HEK293 cells

9.1 Cell Culture

HEK293 cells were cultured in DMEM high glucose with Glutamax (Gibco- Thermo Fisher # 61965-059) supplemented with 10% of Fetal Bovine Serum (Hyclone # SV30160.03HI, provided by GE Healthcare Europe GmbH) and 1 % penicillin/streptomycin. Transfections were carried out on 96-well plates containing 30000 cells/well in a final volume of 100μl, and cells were incubated 24 hours at 37 °C and 5% $CO_2$. After 24 h from cell seeding, the medium was removed and refreshed with 90μl of complete medium. 10μl of each LNPs formulation were added to the cells. After 24 hours cells were recovered and processed.

9.2 ATP Evaluation for Cell Toxicity evaluation

After 24h post-incubation with the LNPs formulation, the medium was aspirated and 50 μl/well of ATPLite reagent (ATPLite PerkinElmer #6016731) were added. The plate was incubated 10 minutes at room temperature in the dark. Luminiscence was read spectrophotometrically using VictorNivo (PerkinElmer) following manufacturer's instructions and data was represented as the percentage of cell viability, taken untreated control cells as 100%.

9.3 Luciferase Assay for transfection efficiency evaluation

After 24h post-incubation with the LNPs formulation, 100 μl of BrightGlo reagent (Promega # E2620) was added in each well following manufacturer instructions. After 5 minutes of incubation at room temperature luciferase activity was measured using VictorNivo (PerkinElmer). Data was represented as luminescence relative to the percentage of transfection relative to the positive control of transfection.

9.4. Biological activity of LNPs in HEK293 cells

The transfection efficiency and the cell viability of the LNPs formulations in HEK293 cells is reported in the following table. The transfection data is represented as % of the positive control DMG-PEG with the formulation reported 46.3/9.4/44.3-x/x molar ratios for ALC-0315/DSPC/Cholesterol/DMG-PEG or LipoPAA being x the different molar ratios for each LipoPAA employed, and being the positive control 100% after 24h of treatment and cell viability is compared to non-treated (NT) cells, being the ATP content readout of NT (non-treated) cells equal to 100%.

**Table 31.** Different parameters of LNPs formulated with lipo-polypeptides. Molar ratio was 46.3/9.4/44.3-x/x, where x is the lipo-polypeptide. Total flow used was 3+1.5mL/min. N/P ratio was 6.17 and nucleic acid content was 50, 150 and 300 ng mRNA/well. %Transfection is calculated considering the formulation ALC-0315/DSPC/Cholesterol/DMG-PEG 46.3/9.4/42.7/1.6:

| Comp. LNP | Lipo-shielding Compound | % Chol | % Lipo-Shield (x) | PDI | Z-average (nm) | % Transf vs DMG-PEG (50 ng mRNA/well) | % Transf vs DMG-PEG (150 ng mRNA/well) | % Transf vs DMG-PEG (300 ng mRNA/well) | % Viability |
|---|---|---|---|---|---|---|---|---|---|
| LNP15 | Lipo-PAA1 | 42.3 | 2 | 0.144 | 97.0 ± 4.7 | 327 ± 64 | 195 ± 95 | 208 ± 64 | >80 % |
| LNP16 | Lipo-PAA8 | 42.3 | 2 | 0.166 | 150.4 ± 22.5 | 311 ± 160 | 323 ± 150 | 335 | >80 % |
| LNP17 | Lipo-PAA10 | 42.3 | 2 | 0.142 | 120.7 | 512 | 364 | 332 | >80 % |
| Comp. LNP13 | DMG-PEG | 42.7 | 1.6 | 0.206 | 84.8 ± 2.6 | 100 | 100 | 100 | >80 % |
| Comp. LNP14 | $C_{14}$-PSar | 39.3 | 5 | 0.117 | 75.2 ± 2.8 | 105 ± 45 | 103 ± 49 | 131± 67 | >80 % |

According to the results, the transfection obtained from LNPs containing Lipo-PAA1 and Lipo-PAA8 and Lipo-PAA10 was higher than those LNPs containing DMG-PEG as well as the PEG alternative C14-PSar. Additionally, It is remarkable that, structurally different conjugates transfected the cells from 1.9 to 3.3-fold (Lipo-PAA1), from 3.1 to 3.3-fold (Lipo-PAA8), and from 3.3 to 5.1-fold (Lipo-PAA10) more efficiently than the commercial standard DMG-PEG at different concentrations, while C14PSar showed only comparable transfection to DMG-PEG.

Therefore, it can be concluded that by adding lipo-PAA into LNPs, the following is obtained: i) stable nanoparticles with acceptable size and polydispersity; ii) non-cytotoxic formulation, and iii) high transfection efficiency. These results demonstrate that the lipo-PAA presented in this application can be an alternative of the actual DMG-PEG compound as well as to the $C_{14}$PSar.

Example 10. *In vivo* transfection capacity of LNPs

10.1 Experimental details

All animal experiments were conducted in accordance with the approved animal protocols. For in vivo mRNA transfection, male Balb/c mice aged 6 weeks were intravenously (iv) administered with Comp.LNP13 and LNP15 (n=3-5). At 4 and 24h postinjection of Comp.LNP13 (n=4), and LNP15 (n=5), mice were injected subcutaneously Luciferin (15 mg/kg), and after 10 minutes mice were euthanized via $CO_2$ inhalation and organs dissected (blood, liver, spleen, lungs, kidney and ALN (Axillary Lymph Nodes). Image acquisition was obtained by IVIS Spectrum (settings: Binning factor 8 F/Stop 1 Exp AUTO FOV 22,5 (D)). The weight of the different organs was obtained and then freezed with $N_2$ and -80°C. Image Analysis was performed by Living Image 4.5.4 (settings: ROI (Draw auto, threshold 2%) and data represented in total counts / tissue g.

*10.2 In vivo* transfection results

Mice after administration of the different formulations were weighted obtaining the following results in Table 32:

**Table 32.** Body weight (g) of mice administered iv with the formulations at time 4 and 24h.

| Formulation | Body weight (g) |
|---|---|
| PBS | 22.7 ± 0.9 |
| Zymogen | 22.3 ± 0.9 |
| Comp.LNP13 24h | 21.4 ± 2.9 |
| Comp.LNP13 4h | 21.2 ± 0.5 |
| LNP15 24h | 20.2 ± 0.7 |
| LNP15 4h | 21.7 ± 0.8 |

Mice body weights remained in the range of 20-22 g, showing no significant differences. The Image analysis by Living Image 4.5.4 of the different organs dissected showed the total counts per g of tissue represented in FIG.2. Statistical analysis by Kruskal-Wallis test determined no significant differences between Comp.LNP13 (containing DMG-PEG) at 4 h with Comp.LNP13 24h and LNP15 4h in liver (A). Organs B-D (ALNs, spleen, and lungs respectively) show no significant differences between Comp.LNP13 and LNP15. Therefore, transfection capacity between positive control (Comp.LNP13) and LNP15 at 4h have comparable results.

**Citation List**

- Nogueira S. et al., "Polysarcosine-Functionalized Lipid Nanoparticles for Therapeutic mRNA Delivery", ACS Appl. Nano Mater., 2020, 3, 10634-10645
- T. W. Green and P.G. M. Wuts, Protective Groups in Organic Chemistry, Wiley, 3rd ed. 1999, Chapter 5 (pp. 369-451)
- T. W. Green and P.G. M. Wuts, Protective Groups in Organic Chemistry, Wiley, 3rd ed. 1999, Chapter 7 (pp. 495-653)
- WO 2020/069718 A1

## Claims

**1.** A lipo-polyamino acid conjugate of formula (I), a salt thereof, or any stereoisomer or mixtures of stereoisomers, either of the compound of formula (I), or of any of its salts,

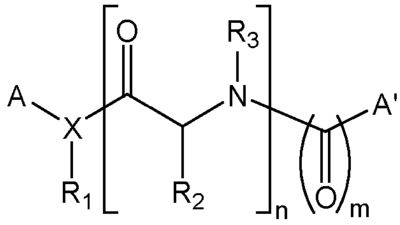

(I)

wherein

n is an integer from 5 to 150;
m is 0 or 1;
X is selected from the group consisting of N, S, and O;
$R_1$ is selected from the group consisting of H, -($C_1$-$C_{18}$)alkyl, and -($C_2$-$C_{18}$)alkenyl, with the condition that $R_1$ is absent when X is O or S;
$R_2$ is a radical selected from the group consisting of (II), (III), (IV), and (V)

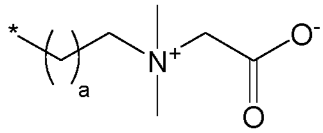

(II)

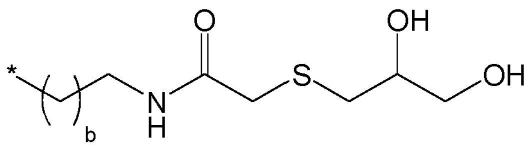

(III)

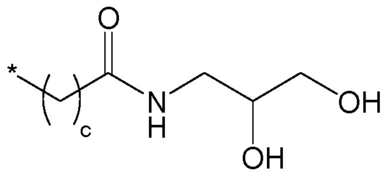

(IV)

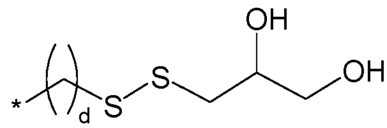

(V)

wherein:
a, b, c, and d are independently an integer from 1 to 4;
$R_3$ is H;
A is a lipid-like moiety $R_5$,
A' is selected from the group consisting of H, and -($C_1$-$C_6$)alkyl;
wherein

(i) the lipid-like moiety $R_5$ has the formula (XIX):

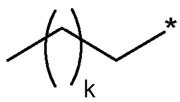

(XIX)

wherein k is 10, 11, 12, 13, 14, 15, or 16; or alternatively
(ii) the lipid-like moiety $R_5$ is ($C_6$-$C_{18}$)alkenyl or alternatively has the formula (XX):

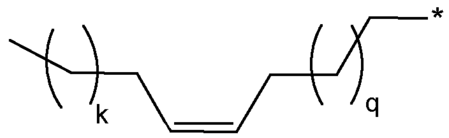

(XX)

wherein k is 10, 11, 12, 13, 14, 15, or 16, and q is an integer from 0 to 18; or alternatively
(iii) the lipid-like moiety $R_5$ has the formula (IX):

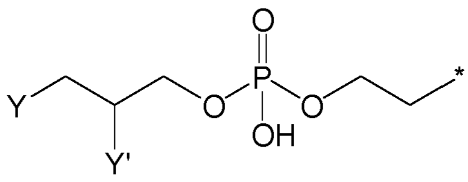

(IX)

wherein Y and Y' are independently selected from -OH, -COORx, and -OCOR$_X$; and each Rx is a radical independently selected from formula (ixa) and (ixb):

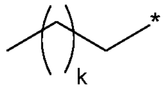 ; 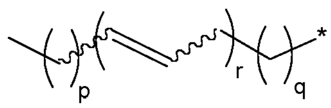

(ixa) (ixb)

wherein the wavy bonds mean that the stereochemistry of the double bonds is undefined, k is 10, 11, 12, 13, 14, 15, or 16; q is an integer from 0 to 18; p is 10, 11, 12, 13, 14, 15, 16, 17, or 18; and r is an integer from 0 to 6; or alternatively
(iv) the lipid-like moiety $R_5$ has the formula (X):

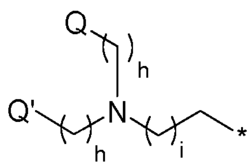

(X)

wherein Q and Q' are independently selected from -OCORy and -COORy; each h is independently an integer from 1 to 18; i is an integer from 0 to 12; and each Ry is a radical independently selected from formula (xa), (xb), and (xc):

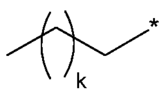 ; 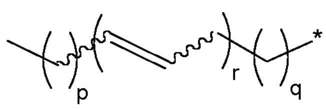 ; 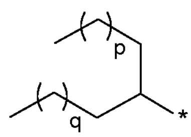

(xa) (xb) (xc)

wherein the wavy bonds mean that the stereochemistry of the double bonds is undefined, k is 10, 11, 12, 13, 14, 15, or 16; q is an integer from 0 to 18, p is 10, 11, 12, 13, 14, 15, 16, 17, or 18; and r is an integer from 0 to 6; or alternatively

(v) the lipid-like moiety $R_5$ has the formula (XII):

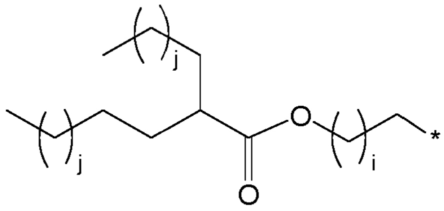

(XII)

wherein i is an integer from 0 to 18; and each j is independently 3, 4, 5, 6, 7, 8, 9, 10, 11, 12; or alternatively

(vi) the lipid-like moiety $R_5$ has the formula (XIII):

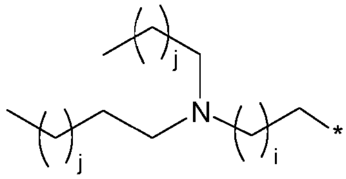

(XIII)

wherein i is an integer from 0 to 18; and each j is independently 10, 11, 12; or alternatively

(vii) the lipid-like moiety $R_5$ has the formula (XIV):

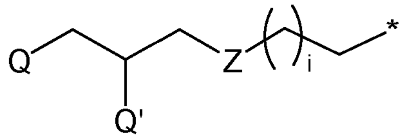

(XIV)

wherein Q and Q' are independently selected from -OCORy and -COORy; Z is selected from -OCO-, -COO-, -NRz'CO-, and -CONRz'-; each Rz' is H or Rz; each Rz is $(C_1-C_{18})$alkyl or $-(C_2-C_{18})$alkenyl; i is an integer from 0 to 18; and each Ry is a radical independently selected from formula (xiva) and (xivb):

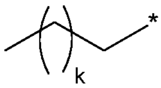

;

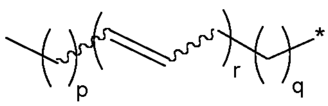

(xiva) (xivb)

wherein the wavy bonds mean that the stereochemistry of the double bonds is undefined, k is 10, 11, 12, 13, 14, 15, or 16; q is an integer from 0 to 18; p is 10, 11, 12, 13, 14, 15, 16, 17, or 18; and r is an integer from 0 to 6.

**2.** The lipo-polyamino acid conjugate according to claim 1, wherein n is an integer from 5 to 100 .

**3.** The lipo-polyamino acid conjugate according to claim 2, wherein n is an integer from 5 to 50.

**4.** The lipo-polyamino acid conjugate according to claim 3, wherein n is an integer from 5 to 30.

**5.** The lipo-polyamino acid conjugate according to claim 4, wherein n is an integer from 5 to 25.

6. A self-assembled particle comprising the lipo-polyamino acid conjugate of formula (I) as defined in any of claims 1-5, and one or more nucleic acids.

7. The self-assembled particle according to claim 6 which is a lipid nanoparticle comprising

(i) the lipo-polyamino acid conjugate of formula (I) as defined in any of claims 1-5 in an amount from 0.1 to 10 mol%;
ii) a ionizable or a cationic lipid in an amount from 30 to 70 mol%;
iii) a phospholipid in an amount from 1 to 20 mol%;
iv) a sterol in an amount from 20 to 60 mol%; and

further comprising a nucleic acid;
wherein the percentages are expressed with respect to the sum of the mol% of the lipids and the lipo-polyamino acid conjugate of formula (I).

8. A composition comprising the self-assembled particle as defined in any of the claims 6-7, together with one or more appropriate excipients or carriers.

9. A therapeutic product which comprises:

a) a self-assembled particle as defined in any of the claims 6-7, or alternatively,
b) a composition as defined in claim 8,

for use in medicine.

10. The therapeutic product for use according to claim 9, for use (i) as transfection reagent for transfecting at least one active agent into a cell; (ii) for use in the *in vivo* or *ex vivo* production of biologics encoding a recombinant protein, a peptide or an antibody, or in the production of recombinant virus; (iii) for use as a therapeutic or prophylactic vaccine against viral infections or as a therapeutic vaccine against cancers or infectious diseases; or (v) for use in genome engineering, for cell reprogramming, for differentiating cells or for gene-editing.

11. A diagnostic product which comprises:

a') a self-assembled particle as defined in any of the claims 6-7, and one or more diagnostically active agents, or alternatively,
b') a composition containing the self-assembled particle a'),
for use in diagnostics.

## Patentansprüche

1. Ein Lipo-Polyaminosäure-Konjugat der Formel (I), ein Salz davon oder ein Stereoisomer oder Gemische von Stereoisomeren, entweder der Verbindung der Formel (I) oder eines ihrer Salze,

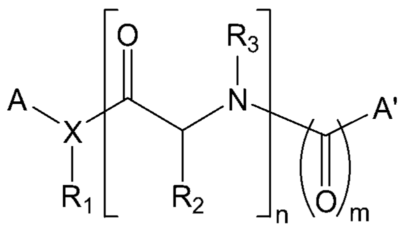

(I)

wobei

n eine ganze Zahl von 5 bis 150 ist;

m = 0 oder 1 ist;
X ausgewählt ist aus der Gruppe bestehend aus N, S und O;
$R_1$ ausgewählt ist aus der Gruppe bestehend aus H, -($C_1$-$C_{18}$)Alkyl und -($C_2$-$C_{18}$)Alkenyl, mit der Maßgabe, dass $R_1$ fehlt, wenn X = O oder S ist;
$R_2$ ein Rest ist ausgewählt aus der Gruppe bestehend aus (II), (III), (IV) und (V)

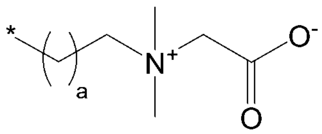

(II)

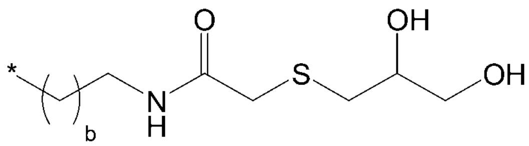

(III)

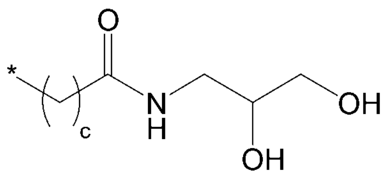

(IV)

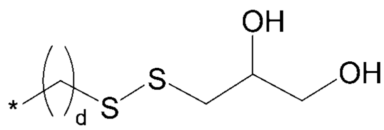

(V)

wobei:
a, b, c und d unabhängig voneinander eine ganze Zahl von 1 bis 4 sind;
$R_3$ = H ist;
A ein lipidähnlicher Teil $R_5$ ist,
A' ausgewählt ist aus der Gruppe bestehend aus H und -($C_1$-$C_6$)-Alkyl;
wobei

(i) der lipidähnliche Teil $R_5$ die Formel (XIX) hat:

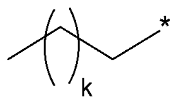

(XIX)

wobei k = 10, 11, 12, 13, 14, 15 oder 16 ist; oder ersatzweise
(ii) der lipidähnliche Teil $R_5$ = ($C_6$-$C_{18}$)-Alkenyl ist oder ersatzweise der Formel (XX) entspricht:

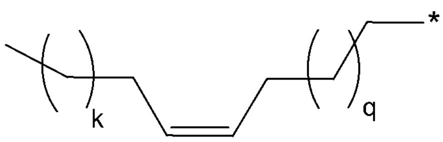

(XX)

wobei k = 10, 11, 12, 13, 14, 15 oder 16 ist und q eine ganze Zahl von 0 bis 18 ist; oder ersatzweise
(iii) der lipidähnliche Teil $R_5$ der Formel (IX) entspricht:

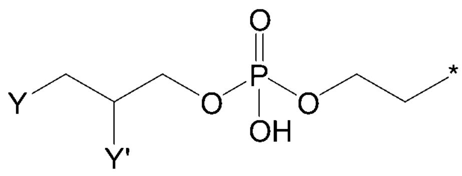

(IX)

wobei Y und Y' unabhängig voneinander ausgewählt sind aus -OH, -COORx und -OCORx; und jedes Rx ein Rest ist, der unabhängig ausgewählt ist aus den Formeln (ixa) und (ixb):

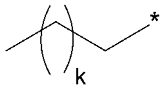

;

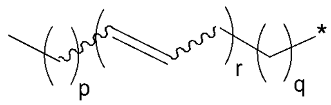

(ixa) (ixb)

wobei die Wellenbindungen bedeuten, dass die Stereochemie der Doppelbindungen undefiniert ist, k = 10, 11, 12, 13, 14, 15 oder 16 ist; q eine ganze Zahl von 0 bis 18 ist; p = 10, 11, 12, 13, 14, 15, 16, 17 oder 18 ist; und r eine ganze Zahl von 0 bis 6 ist; oder ersatzweise

(iv) der lipidähnliche Teil $R_5$ der Formel (X) entspricht:

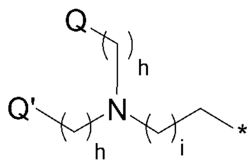

(X)

wobei Q und Q' unabhängig voneinander aus -OCORy und COORy ausgewählt sind-; jedes h unabhängig voneinander eine ganze Zahl von 1 bis 18 ist; i eine ganze Zahl von 0 bis 12 ist; und jedes Ry ein Rest ist, das unabhängig voneinander aus den Formeln (xa), (xb) und (xc) ausgewählt ist:

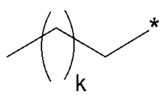

;

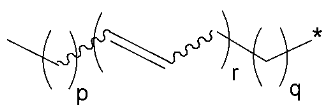

;

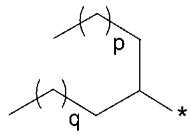

(xa) (xb) (xc)

wobei die Wellenbindungen bedeuten, dass die Stereochemie der Doppelbindungen undefiniert ist, k = 10, 11, 12, 13, 14, 15 oder 16 ist; q eine ganze Zahl von 0 bis 18 ist, p = 10, 11, 12, 13, 14, 15, 16, 17 oder 18 ist; und r eine ganze Zahl von 0 bis 6 ist; oder ersatzweise

(v) der lipidähnliche Teil $R_5$ der Formel (XII) entspricht:

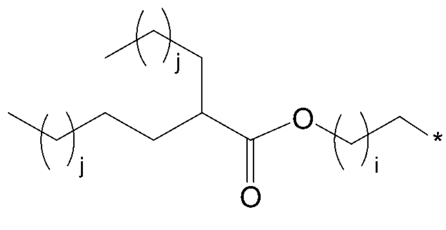

(XII)

wobei i eine ganze Zahl von 0 bis 18 ist; und jedes j unabhängig voneinander 3, 4, 5, 6, 7, 8, 9, 10, 11, 12 ist;

oder ersatzweise
(vi) der lipidähnliche Teil $R_5$ der Formel (XIII) entspricht:

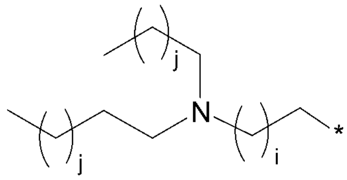

(XIII)

wobei i eine ganze Zahl von 0 bis 18 ist; und jedes j unabhängig voneinander 10, 11, 12 ist; oder ersatzweise
(vii) der lipidähnliche Teil $R_5$ der Formel (XIV) entspricht:

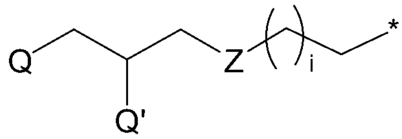

(XIV)

wobei Q und Q' unabhängig voneinander ausgewählt sind aus -OCORy und -COORy; Z ausgewählt ist aus -OCO-, -COO-, -NRz' CO- und CONRz -'-; jedes Rz' H oder Rz ist; jedes Rz ($C_1$-$C_{18}$)-Alkyl oder -($C_2$-$C_{18}$)-Alkenyl ist; i eine ganze Zahl von 0 bis 18 ist; und jedes Ry ein Rest ist, der unabhängig voneinander ausgewählt ist aus den Formeln (xiva) und (xivb):

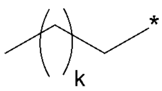

;

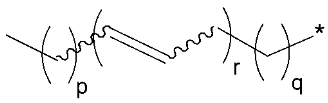

(xiva) (xivb)

wobei die Wellenbindungen bedeuten, dass die Stereochemie der Doppelbindungen undefiniert ist, k = 10, 11, 12, 13, 14, 15 oder 16 ist; q eine ganze Zahl von 0 bis 18 ist; p = 10, 11, 12, 13, 14, 15, 16, 17 oder 18 ist; und r eine ganze Zahl von 0 bis 6 ist.

**2.** Das Lipo-Polyaminosäure-Konjugat nach Anspruch 1, wobei n eine ganze Zahl von 5 bis 100 ist.

**3.** Das Lipo-Polyaminosäure-Konjugat nach Anspruch 2, wobei n eine ganze Zahl von 5 bis 50 ist.

**4.** Das Lipo-Polyaminosäure-Konjugat nach Anspruch 3, wobei n eine ganze Zahl von 5 bis 30 ist.

**5.** Das Lipo-Polyaminosäure-Konjugat nach Anspruch 4, wobei n eine ganze Zahl von 5 bis 25 ist.

**6.** Ein selbstorganisiertes Partikel umfassend das Lipo-Polyaminosäure-Konjugat der Formel (I), wie in einem der Ansprüche 1 bis 5 definiert, und eine oder mehrere Nukleinsäuren.

**7.** Das selbstorganisierte Partikel nach Anspruch 6, das ein Lipid-Nanopartikel ist, das Folgendes umfasst

(i) das Lipo-Polyaminosäure-Konjugat der Formel (I) wie in einem der Ansprüche 1 bis 5 definiert in einer Menge von 0,1 bis 10 Mol-%;
ii) ein ionisierbares oder ein kationisches Lipid in einer Menge von 30 bis 70 Mol-%;
iii) ein Phospholipid in einer Menge von 1 bis 20 Mol-%;
iv) ein Sterin in einer Menge von 20 bis 60 Mol-%; und

ferner umfassend eine Nukleinsäure;
wobei die Prozentsätze in Bezug auf die Summe der Mol-% der Lipide und des Lipo-Polyaminosäure-Konjugats der Formel (I) ausgedrückt sind.

**8.** Eine Zusammensetzung umfassend das selbstorganisierte Partikel wie in einem der Ansprüche 6 bis 7 definiert, zusammen mit einem oder mehreren geeigneten Hilfsstoffen oder Trägersubstanzen.

**9.** Ein therapeutisches Produkt, das Folgendes umfasst:

a) ein selbstorganisiertes Partikel wie in einem der Ansprüche 6 bis 7 definiert, oder ersatzweise
b) eine Zusammensetzung wie in Anspruch 8 definiert,

zur Verwendung in der Medizin.

**10.** Das therapeutische Produkt zur Verwendung nach Anspruch 9, zur Verwendung (i) als Transfektionsreagenz zur Transfektion von mindestens einem Wirkstoff in eine Zelle; (ii) zur Verwendung bei der *in vivo*- oder ex vivo-Produktion von Biologika, die ein rekombinantes Protein, ein Peptid oder einen Antikörper kodieren, oder bei der Produktion von rekombinanten Viren; (iii) zur Verwendung als therapeutischer oder prophylaktischer Impfstoff gegen Virusinfektionen oder als therapeutischer Impfstoff gegen Krebs oder Infektionskrankheiten; oder (v) zur Verwendung in der Genomtechnik, zur Zellreprogrammierung, zur Differenzierung von Zellen oder zum Gen-Editing.

**11.** Ein diagnostisches Produkt, das Folgendes umfasst:

a') ein selbstorganisiertes Partikel wie in einem der Ansprüche 6 bis 7 definiert, und ein oder mehrere diagnostisch aktive Mittel, oder ersatzweise,
b') eine Zusammensetzung, die das selbstorganisierte Partikel a') enthält,
zur Verwendung in der Diagnostik.

## Revendications

**1.** Un conjugué lipo-poly(acide aminé) de formule (I), un sel de celui-ci, ou tout stéréoisomère ou mélanges de stéréoisomères, soit du composé de formule (I), soit de l'un de ses sels,

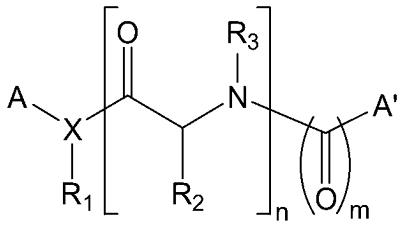

(I)

où

n est un nombre entier de 5 à 150 ;
m est 0 ou 1 ;
X est choisi dans le groupe constitué par N, S et O ;
$R_1$ est choisi dans le groupe constitué par H, -alkyle en ($C_1$-$C_{18}$) et -alcényle en ($C_2$-$C_{18}$), à la condition que $R_1$ soit absent lorsque X est O ou S ;
$R_2$ est un radical choisi dans le groupe constitué par (II), (III), (IV) et (V)

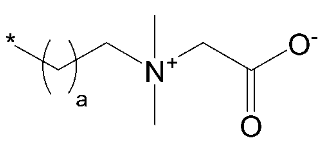
(II)

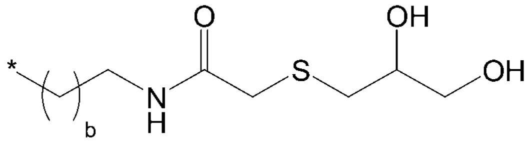
(III)

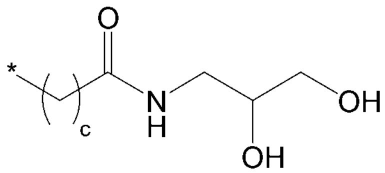
(IV)

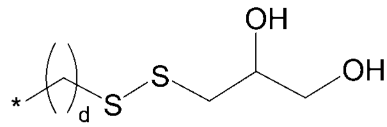
(V)

où :
a, b, c et d sont indépendamment un nombre entier de 1 à 4 ;
$R_3$ est H ;
A est un fragment de type lipide $R_5$,
A' est choisi dans le groupe constitué par H et alkyle en -($C_1$-$C_6$) ;
où

(i) le fragment de type lipide $R_5$ a la formule (XIX) :

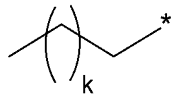
(XIX)

où k est 10, 11, 12, 13, 14, 15 ou 16 ; ou, en variante,
(ii) le fragment de type lipide $R_5$ est alcényle en ($C_6$-$C_{18}$) ou a la formule (XX) :

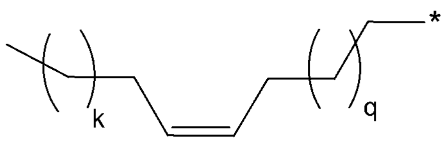
(XX)

dans laquelle k est 10, 11, 12, 13, 14, 15 ou 16, et q est un nombre entier de 0 à 18 ; ou, en variante,
(iii) le fragment de type lipide $R_5$ a la formule (IX) :

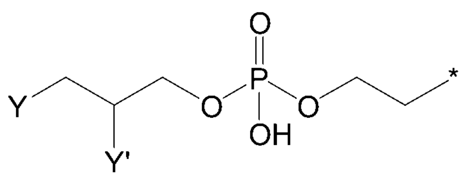
(IX)

dans laquelle Y et Y' sont choisis indépendamment parmi -OH, -COORx et -OCORx ; et chaque Rx est un

radical choisi indépendamment parmi la formule (ixa) et (ixb) :

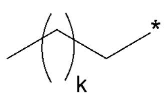 ; 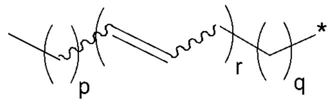

(ixa) (ixb)

où les liaisons ondulées signifient que la stéréochimie des doubles liaisons n'est pas définie, k est 10, 11, 12, 13, 14, 15 ou 16 ; q est un nombre entier de 0 à 18 ; p est 10, 11, 12, 13, 14, 15, 16, 17 ou 18 ; et r est un nombre entier de 0 à 6 ; ou, en variante,

(iv) le fragment de type lipide $R_5$ a la formule (X) :

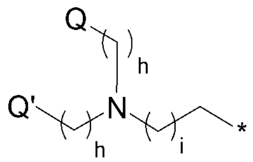

(X)

dans laquelle Q et Q' sont indépendamment choisis parmi -OCORy -et COORy ; chaque h est indépendamment un nombre entier de 1 à 18 ; i est un nombre entier de 0 à 12 ; et chaque Ry est un radical indépendamment choisi parmi les formules (xa), (xb) et (xc) :

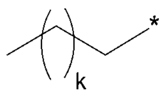 ; 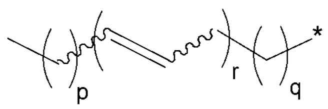 ; 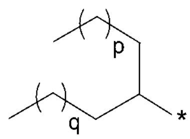

(xa) (xb) (xc)

où les liaisons ondulées signifient que la stéréochimie des doubles liaisons n'est pas définie, k est 10, 11, 12, 13, 14, 15 ou 16 ; q est un nombre entier de 0 à 18, p est 10, 11, 12, 13, 14, 15, 16, 17 ou 18 ; et r est un nombre entier de 0 à 6 ; ou, en variante,

(v) le fragment de type lipide $R_5$ a la formule (XII) :

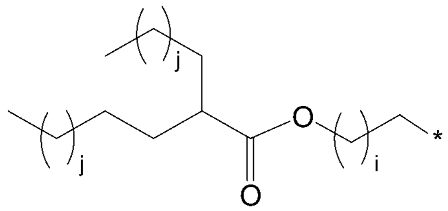

(XII)

dans laquelle i est un nombre entier de 0 à 18 ; et chaque j est indépendamment 3, 4, 5, 6, 7, 8, 9, 10, 11, 12 ; ou, en variante,

(vi) le fragment de type lipide $R_5$ ala formule (XIII) :

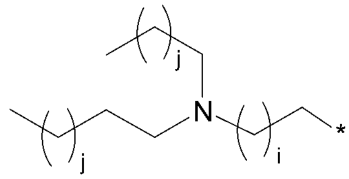

(XIII)

où i est un nombre entier de 0 à 18 ; et chaque j est indépendamment 10, 11, 12 ; ou, en variante, (vii) le fragment de type lipide $R_5$ a la formule (XIV) :

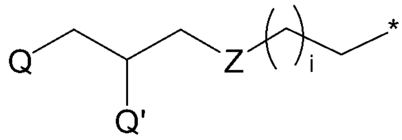

(XIV)

dans laquelle Q et Q' sont choisis indépendamment parmi -OCORy -et COORy ; Z est choisi parmi - OCO-, -COO-, -NRz' CO- et CONRz-'- ; chaque Rz' est H ou Rz ; chaque Rz est alkyle en ($C_1$-$C_{18}$) ou alcényle en ($C_2$-$C_{18}$) ; i est un nombre entier de 0 à 18 ; et chaque Ry est un radical choisi indépendamment parmi les formules (xiva) et (xivb) :

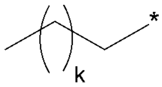

;

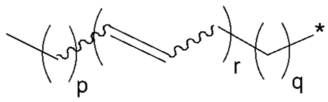

(xiva) (xivb)

où les liaisons ondulées signifient que la stéréochimie des doubles liaisons n'est pas définie, k est 10, 11, 12, 13, 14, 15 ou 16 ; q est un nombre entier de 0 à 18 ; p est 10, 11, 12, 13, 14, 15, 16, 17 ou 18 ; et r est un nombre entier de 0 à 6.

2. Le conjugué lipo-poly(acide aminé) selon la revendication 1, dans lequel n est un nombre entier de 5 à 100.

3. Le conjugué lipo-poly(acide aminé) selon la revendication 2, dans lequel n est un nombre entier de 5 à 50.

4. Le conjugué lipo-poly(acide aminé) selon la revendication 3, dans lequel n est un nombre entier de 5 à 30.

5. Le conjugué lipo-poly(acide aminé) selon la revendication 4, dans lequel n est un nombre entier de 5 à 25.

6. Une particule auto-assemblée comprenant le conjugué lipo-poly(acide aminé) de formule (I) tel que défini dans l'une quelconque des revendications 1 à 5, et un ou plusieurs acides nucléiques.

7. La particule auto-assemblée selon la revendication 6 qui est une nanoparticule lipidique comprenant

(i) le conjugué lipo-poly(acide aminé) de formule (I) tel que défini dans l'une quelconque des revendications 1 à 5 en une quantité allant de 0,1 à 10 % en moles ;
ii) un lipide ionisable ou cationique en une quantité allant de 30 à 70 % en moles ;
iii) un phospholipide en une quantité allant de 1 à 20 % en moles ;
iv) un stérol en une quantité allant de 20 à 60 % en moles ; et

comprenant en outre un acide nucléique ;
dans lequel les pourcentages sont exprimés par rapport à la somme du pourcentage molaire des lipides et du conjugué lipo-poly(acide aminé) de formule (I).

8. Une composition comprenant la particule auto-assemblée telle que définie dans l'une quelconque des revendications

6 à 7, avec un ou plusieurs excipients ou véhicules appropriés.

**9.** Un produit thérapeutique qui comprend :

a) une particule auto-assemblée telle que définie dans l'une quelconque des revendications 6 et 7, ou, en variante,
b) une composition telle que définie dans la revendication 8,

pour son utilisation en médecine.

**10.** Le produit thérapeutique pour l'utilisation selon la revendication 9, pour l'utilisation (i) comme réactif de transfection pour transfecter au moins un agent actif dans une cellule ; (ii) pour l'utilisation dans la production *in vivo* ou *ex vivo* de médicaments biologiques codant pour une protéine recombinante, un peptide ou un anticorps, ou dans la production de virus recombinants ; (iii) pour l'utilisation comme vaccin thérapeutique ou prophylactique contre les infections virales ou comme vaccin thérapeutique contre des cancers ou des maladies infectieuses ; ou (v) pour son utilisation dans l'ingénierie du génome, pour la reprogrammation cellulaire, pour la différenciation des cellules ou pour la modification localisée de séquence génomique.

**11.** Un produit de diagnostic qui comprend :

a') une particule auto-assemblée telle que définie dans l'une quelconque des revendications 6 à 7, et un ou plusieurs agents actifs diagnostiques, ou, en variante,
b') une composition contenant la particule auto-assemblée a'),
pour son utilisation dans les diagnostics.

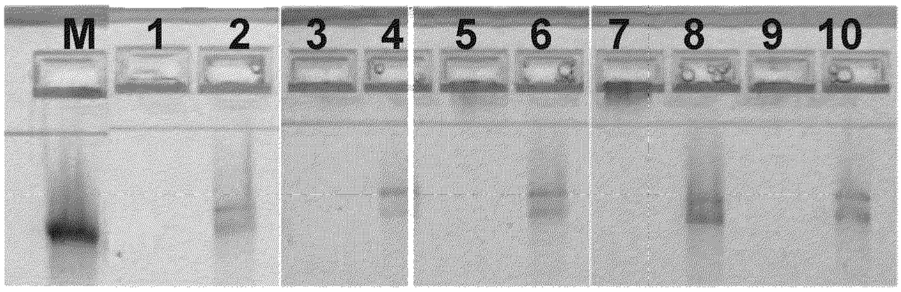
M
1
2
3
4
5
6
7
8
9
10

FIG. 1

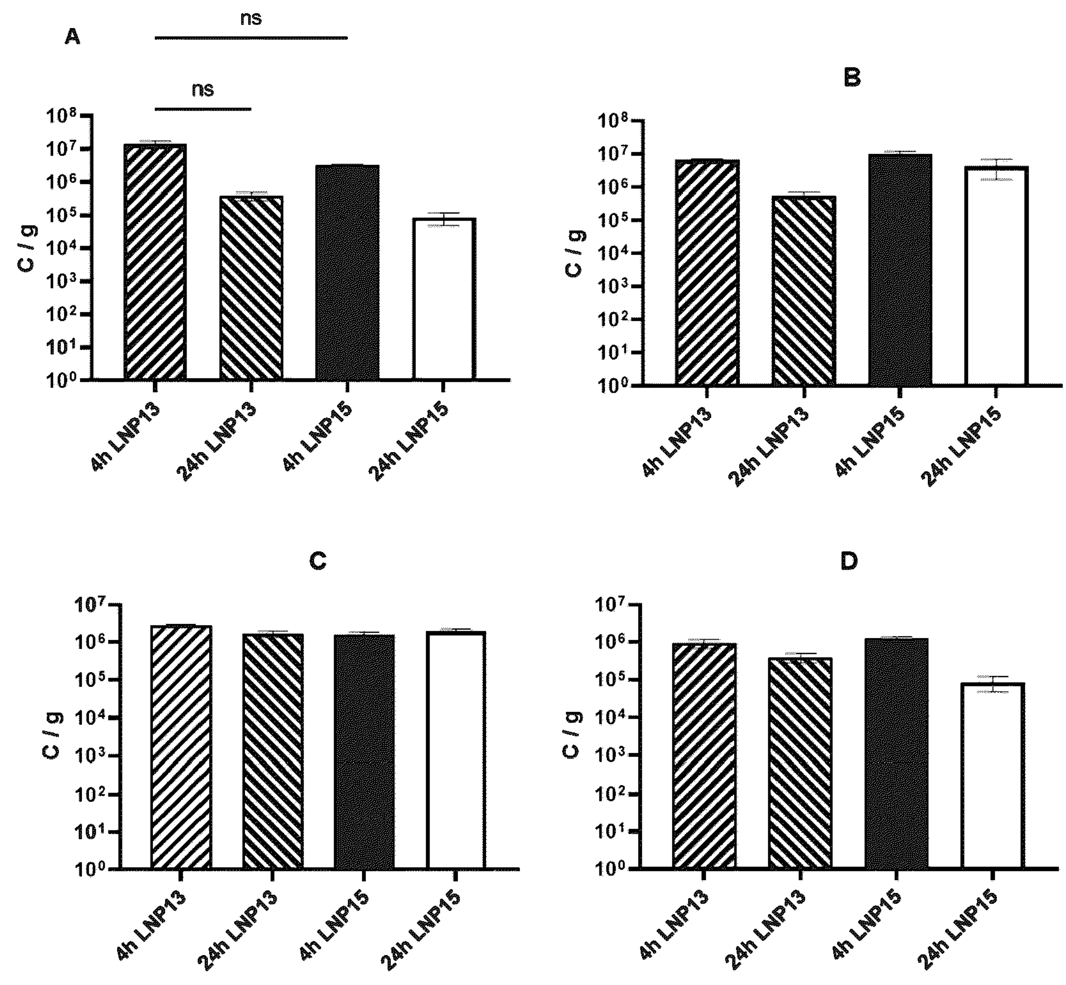
A
ns
ns
C / g
4h LNP13
24h LNP13
4h LNP15
24h LNP15
B
C
D

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020069718 A1


### Non-patent literature cited in the description


- *ACS Appl. Nano Mater.*, 2020, vol. 3, 10634-10645
- **T. W. GREEN** ; **P.G. M. WUTS**. Protective Groups in Organic Chemistry. Wiley, 1999, 369-451
- **T. W. GREEN ,P.G. M. WUTS**. Protective Groups in Organic Chemistry. Wiley, 1999, 495-653
- **NOGUEIRA S. et al.** Polysarcosine-Functionalized Lipid Nanoparticles for Therapeutic mRNA Delivery. *ACS Appl. Nano Mater.*, 2020, vol. 3, 10634-10645
- **T. W. GREEN** ; **P.G. M. WUTS**. Protective Groups in Organic Chemistry. Wiley, 1999, 495-653